# EUROPEAN PATENT APPLICATION

(11) **EP 1 882 742 A1**
(43) Date of publication of application: **30.01.2008**
(21) Application number: 06118112.9
(22) Date of filing: 28.07.2006
(51) Int. Cl.: C12N 15/82

(54) **Plants defective for soluble starch synthase IV (SSIV) activity, methods for obtaining the same, and uses thereof**

(71) Applicant: INSTITUT NATIONAL DE LA RECHERCHE AGRONOMIQUE (INRA), 75341 Paris Cédéx 07 (FR); Consejo Superior de Investigaciones Cientificas (CSIC), 28006 Madrid (ES)
(72) Inventor: Planchot, Véronique, 44300 Nantes (FR); Merida, Angel, 41009 Sevilla (FR); Roldan, Isaac, 11687 El Gastor (ES); D`Hulst, Christophe, 59150 Wattrelos (FR); Lucas, Mercédes, 28053 Madrid (ES); Wattebled, Fabrice, 49630 Maze (FR); Delvalle, David, 59230 Saint Amand Les Eaux (FR)
(74) Representative: Michelet, Alain

(57) **Abstract**

This invention relates to a plant which is modified so as to be rendered defective for Soluble Starch Synthase IV (SSIV), having plant cells which contain starch granules having an increased granule size, as compared to the same plant that is not defective for Soluble Starch Synthase IV (SSIV).

SEQUENCE LISTING

<110>  INSTITUT NATIONAL DE LA RECHERCHE AGRONOMIQUE - INRA
       CSIC (Consejo Superior de Investigaciones Cientificas

<120>  Plants defective for Soluble Starch Synthase IV (SSIV), methods for
obtaining the same, and uses thereof

<130>  U221-EU - INSTITUT NATIONAL DE LA RECHERCHE AGRONOMIQUE - INRA
       CSIC (Consejo Superior de Investigaciones Cientificas

<160>  27

<170>  PatentIn version 3.1

<210>  1
<211>  3123
<212>  DNA
<213>  Arabidopsis thaliana

<400>  1
atgacgacga agctatcgag cttctgtttc ttaacccatg gattagcagg aatctcctgc      60

gagagagaac atggaagttc tcgtcgattt ttctatctcc cttctcgtcg attagtttct     120

acttcgtgta agatgcgaca acaacgtggt ttcgactcta gtaaaagaca agaggtcaag     180

aaaggctccc ctaaaccgat cctatctata aattcaggtc ttcagagcaa caatgatgag     240

gaatctgatc tggagaatgg ttctgcggac agtgtcccga gtcttaagtc agatgctgaa     300

aagggtagta gtattcatgg cagcatagat atgaatcatg ctgatgagaa tcttgaaaag     360

aaagatgata tacaaacaac tgaagttact cggcgcaaga gtaaaactgc aaagaagaaa     420

ggggagagta ttcatgctac gattgatatt ggacatgatg atgggaagaa tttagataat     480

attactgtgc ctgaggttgc aaaagctttg tcccttaaca aaagtgaagg cgagcagatt     540

tcagatggac agtttgggga actaatgaca atgataagaa gtgcagaaaa aaatattctt     600

cggcttgatg aagcacgggc cactgctctt gacgacctta acaagattct tagtgataag     660

gaagcgttgc agggagaaat caatgtcttg gaaatgaaat tgtctgagac tgatgaaagg     720

attaaaactg ctgctcaaga gaaagcacat gtagagcttc tggaagaaca gttagagaag     780

cttcgtcatg aaatgatctc ccctatagaa agtgatggtt atgttctagc tcttagcaaa     840

gagcttgaaa cattgaagct ggagaatcta tctttgagaa acgatataga aatgcttaag     900

tcagaacttg acagtgttaa agataccggt gaacgtgtgg ttgtgttgga aaaggagtgc     960

tccggtttgg aatcttctgt aaaggatttg gaatctaagt tatcggtttc tcaggaagat    1020

gtctcgcagc tttctactct taaaatcgaa tgcactgatc tatgggcgaa ggtagagact    1080

ctacagctgt tgttagatag agctaccaaa caagcagagc aagcagttat agtgttacaa    1140

cagaaccaag atctaagaaa taaggtcgac aaaattgagg aatcacttaa agaagccaac    1200

gtttataaag aatcttcaga gaaaattcaa cagtacaatg agctaatgca acataaggtg 1260

acattacttg aggagcggct cgaaaagtct gatgcagaga tattctcata tgttcagtta 1320

tatcaagaat cgataaaaga attccaggaa acacttgaaa gtttgaagga agaaagcaag 1380

aaaaaatcaa gagatgaacc ggttgatgat atgccttggg attattggag tcggttactt 1440

ttaactgttg atggatggct gcttgaaaag aaaatagcaa gcaacgatgc tgacttactg 1500

agagacatgg tatggaagaa agatcgaaga attcatgata cctatattga cgtcaaagat 1560

aagaatgaac gtgatgccat ctctgcattt ctcaagcttg tctcatctcc aacaagttca 1620

ggattgtatg tcgttcacat tgcagctgag atggcacctg tagctaaggt gggaggtttg 1680

ggagatgttg tggcaggtct tggtaaggca ttgcaaagaa aaggtcatct ggtggagatt 1740

attcttccca aatatgactg tatgcagtat gatcgcgtgc gtgacttaag ggctttggat 1800

actgttgtgg agtcatattt tgatgggaag ttatataaaa acaaaatctg gattggcact 1860

gttgaaggtt tacctgtaca tttcattgaa cctcaacatc caagcaaatt cttctggaga 1920

ggacagtttt atggagagca agatgatttc agacgcttct cgtattttag ccgagctgca 1980

ctagagttgc ttcttcagtc cggcaaaaaa cccgacatca tacattgtca tgactggcaa 2040

acagcttttg ttgcgccgct gtattgggat ctgtatgctc caaagggatt agattctgca 2100

agaatatgct ttacatgtca taattttgag tatcaaggta ccgcgtctgc ttcagaattg 2160

ggatcttgcg ggcttgatgt taaccagtta aatagaccag acagaatgca ggatcactcg 2220

tctggagata gagtcaatcc tgttaagggc gctataattt tctcaaacat tgtaacaacc 2280

gtgtccccta cttatgcaca agaagttcga acagctgagg gaggaaaagg actccattca 2340

acactcaatt ttcactccaa gaaattcatt ggaatcctca acggcattga cacagattca 2400

tggaatcctg ccaccgaccc cttcctcaag gctcagttca acgctaaaga tctacaaggg 2460

aaagaagaaa acaaacacgc ccttagaaag cagcttggac tttcttcagc ggagtcaaga 2520

cggcctttgg ttggttgcat aacaagatta gtaccacaga aaggagttca tctaatcaga 2580

catgccatat acagaacatt agagttaggt ggacaatttg tacttcttgg ttctagcccg 2640

gttccacata ttcagaggga atttgaaggt attgaacaac agtttaaaag ccatgatcat 2700

gtccggttgt tactgaagta cgatgaagct ctgtctcata cgatttacgc agcctccgat 2760

ttgttcatca ttccgtcaat ttttgagcct tgtggactta cacagatgat cgctatgaga 2820

tatggctcca ttccaattgc gcgaaaaact ggaggcttaa atgacagtgt cttcgacatt 2880

gatgatgata caataccaac acagtttcaa aatggattta catttcaaac cgcggacgaa 2940

cagggtttca attatgcgtt ggagcgagca tttaatcact acaagaaaga tgaagagaaa 3000

tggatgagac ttgtagagaa agtgatgagt atagatttca gctggggatc atcagctaca 3060

caatacgaag aactctacac gagatcagtg tccagagcaa gagctgttcc taatcgcacg 3120

tga 3123

<210> 2
<211> 2919
<212> DNA
<213> Oryza sativa

<400> 2
atggcggctt gcggcgccgc ggcggcggag tattcagctc tcctgtccct ctcatgtggg 60

cctatcactc gccgccgttt cgccgtctcc tgccgcgctc gaccgcccgg caacctcagt 120

gcacagcaga agaagaagcg agggaaaaac attgctccaa aacagcgctc ttcaaatgca 180

aaactcttgc ttactactga ggaaaatgga cagcttccaa gtaccagttt gaggacttct 240

atggaacgtc ctcagaagag tacatccagt gaggacgata ccaatggggc tattagccag 300

atagatgaaa aaattgcagc cattggtaat gaacagcaag agaggtcaaa ggataaacat 360

ttcgaatcag actttcaact tgaagatttt ggggaaatga tacaaaacat ggaaaagaat 420

attctgctcc taaatcaagc tcgtcttcaa gcaattgaag atgttgacaa aattcttact 480

gaaaaggaag ctttgcagaa aaaggttgac actctagaga tgaatttgtc aaaagcactt 540

gcaaccaaag ggaacataaa tacagatatc cctggtgatc atttagagaa atttacaaaa 600

gaaattttga tagaaagtgc tctaagtgga ggcaatccag cacatttgtg tgagtctcca 660

cttttcatgg agctgactgt tctaaaagaa gagaatatgc tacttaaggc tgacgcacaa 720

tttcttaaag caaagattgt tgagtttgct gaaacagagg aattcctatt caaattggag 780

aaagaaaggt cactgttaga cgctacagtt agggagctgg aagctaggtt tctagttgct 840

caaactgata tctggaaagt tgtccctctg caatatgatg tttggatgga gaaagtagaa 900

aatctgcaac acatgcttgg atgtttgaaa aatcatgtag agaagtatgc tgcattgttg 960

gatcagcatg atgatctgca tgacaagatt gatgagctag aagcatcact aaaagaaggg 1020

aaaacatctg agttttctcc ttatgttgtt gaacttctgc aacagaagtt gaaagcagca 1080

aaaagccatc accaagcagg ccatcaagaa acaaacactc acattcaagt atatcagcag 1140

ttaactgagg aatttcaaga taacctaggt aaactaatag aagaaagtgg cagactggag 1200

cattctgcaa atagtatgcc atcagaattc tggagtcaca tcctgctcat gattgatggt 1260

tggttccttg agcggaaaat accaaatact gatgcacgta tgctgagaga aatggcatgg 1320

aagagagatg accgtatctg tgaagcctac tttgcttgca agggtgcaaa agaaagtgat 1380

gttatggaaa ctttcctaaa gctatcgtta tcaggaaaca gttctggctt gcacattgtc 1440

catatagcag cagagatggc tccagtggca aaggttggcg gtctggcaga tgtagttgct 1500

ggtcttggca aggcacttca gactaaggga catttagtag agattgttct tcccaaatac 1560

gactgtatgc aacttgacca gattactaat cttaaggttt tagatgttgt catacaatcc 1620

tattttgatg gaaatctatt cagcaacaac gtgtggacag gaactgttga aggtcttcca 1680

gtctatttta ttgagccaca gcatccttca aaattcttct ggagggccca atactatgga 1740

gagcatgacg attttaaacg ttattcatac tttagccgtg cagcactgga attactttac 1800

caatctggga agaaaattga tataatccac tgccatgact ggcaaactgc atttgttgca 1860

cctctatatt gggatatata tgcaactcga ggcttcagct cagctagaat ttgtttcact 1920

tgccacaact tcgaatacca aggtacagca cctgctcctg atttatcata ttgcggcctt 1980

gatgttgagc agttggatag accagacagg atgcaagaca atgcacatgg aagaataaat 2040

gttgccaagg gtggaattgt ttattccaat attgtgacaa ctgtatcacc aacatatgct 2100

ttagaagtgc gctcagaggg tggtcgtggt ctccaagata cacttaaaat gcattccagg 2160

aaatttgtgg ggatacttaa tggaatcgac acaggaacat ggaacccttc cacagacaga 2220

tttcttgctg tgcaatatag tgctactgat cttcagggga aagcagcaaa caaagcattt 2280

ctcaggaagc agctagggct gtattctgag gatgcctctc aaccactggt agcctgcatt 2340

acgaggctag ttcctcaaaa gggtctacat cttatcaggc atgcaatata caaaacagct 2400

gagttaggag gacagtttgt actcctgggt tcaagtccag ttccacatat tcagagggaa 2460

tttgagggtg ttgcagacca attccagaag aataacaata ttaggttaat tctgaaatat 2520

gatgaagctt tgtctcattg tatttatgcg gcatctgaca tgtttatcat tccatcaatg 2580

tttgagcctt gtggccttac tcagatgata gctatgagat atggttctgt gccaattgtt 2640

cgacaaactg gtggattgtg tgatagtgtt tttgattttg atgatgaaac tatacctgtg 2700

gagctgcgga atggctttac atttgctagg actgatgagc aggatctaag cagttgcttg 2760

gaaagggcat tcagctacta cagcagaaaa cctatggttt ggaaacagtt agtgcagaag 2820

gatatgcaga ttgatttcag ctgggattct ccagcttcac agtacgaaaa cctttatcag 2880

agtgctgttg ctcaggcaag gggagcggct caaacataa 2919

<210> 3
<211> 2760
<212> DNA
<213> Oryza sativa

<400> 3
atggcgtgct tggcggcggg cgccgaggcg gcccctctcc tcttccggcg gcggctagcg 60

ccgtccccag tcgccgcgcg ccgccgcctt ctcgtgtcgt gccgagctcg ccgccgcggt 120

ctcaggttgg tagcccagag cgcaggatcg cgtggttgcg gcgtggtcgg tgcacccggt 180

tgtgattact gggtgaatat gcagagagat gaagcatctg tttccagtga taaagaacgg 240

caagagaaat atggagatga aaatggtata tcaaaccttc aattggaaga tttgatacaa 300

atgatacaaa acactgagaa gaatataatg cttctgaatc aagctcgtct tcaagcattg 360

gaacatgttg aaacagttct taaagaaaaa gaagacttac agaggaagtt gaaaattttg 420

gagacgagat tgtcagaaac agatgcacgg cttaagcttt cagctgaagg gcagttcggt 480

actgagatta atgactctct accagtactg gaattagatg atataaagga agagaatatg 540

ctactgaagg atgacataca atttctgaaa acaatgctta ttgaggttgc tgagacagag 600

aacagtatat tcacattaga gaaggagcgt gctcttttag atgcttcgct tagggagctg 660

gagtctagat ttatagatgc ccaagcagat atgttgaagt ctgatcctag gcagtatgat 720

gcatggtggg agaaagtaga aaatttgggg gacttgcttg agactgcaac aaacaaagta 780

gagaatgctg ctatggttct gggacgcaat catgatttgg aagataaggt cgacaaacta 840

gaggcatcgt tggctgaagc aaatatatca aagttctctt gttattttgt tgatcttttg 900

caggaaaaga taaaatcagt agaagagcgc ttccaagtat gtaatcatga aatgcattct 960

caaattgaac tctatgagaa ttcaatagcg gaatttcacg atattcttag caagctagtg 1020

gaggaaactg agaaacgatc actagagcat tcagcaagta gcatgccttc agaattgtgg 1080

agtaggatat ctcttctgat tgatggttgg ttgctcgaga agagaatatc ctacaatgat 1140

gcaaatacat tacgggaaat ggtcaggaaa agggatagtt gtcttcggga agcatacttg 1200

tcatgcagag gtatgaaaga tagggaaatt gtggacaatt ttctaaagat cacattgcca 1260

gggactagtt ctggcttgca catcatccac atagcagcag agatggctcc tgttgcaaag 1320

gttggtggtt tggcggatgt gatatctggt cttggcaagg cacttcagaa aaagggccac 1380

ctagtagaga ttattcttcc aaaatatgac tgcatgcaga atgaccaagt taataacctt 1440

aaggttttag atgttgttgt acaatcctac tttgaaggaa atttgttcaa caacaaaata 1500

tggactggga ctgttgaagg ccttccagtc tattttatcg agccacaaca tccagcaaaa 1560

ttcttttgga gggcacaata ctatggagaa cacgatgact ttaaacgttt tgcatacttc 1620

agccgtgcag cactggaatt actttaccaa tcgcagaaga aaattgacat catccattgc 1680

catgactggc agactgcatt tgtggcacct ctttattggg aagcatatgc aaatctgggc 1740

ttcaactcag ctagaatttg cttcacctgc cataattttg aatatcaagg agctgctcct 1800

gctcaagatt tagcatgctg tggccttgat gttcagcaac ttgataggga agacaggatg 1860

cgggacaatt cacatggcag aataaatgtt gtcaaggtgg caattgtgta ttccaacatt 1920

gtgacaactg tatcaccaac atatgcacta gaggtgcgat cagagggtgg acgtggacta 1980

caagattcac tcaaattaca ttccaggaaa tttgttggga tacttaatgg aatcgacaca 2040

gacacatgga atccttcaac agatagacat ctcaaggttc aatataatgc taatgatctc 2100

cagggtaagg cagcaaacaa agcagctctc agaaagcagc taaacttatc ttctacaaat 2160

gcttctcaac cactggttgg gtgtattaca aggctagttc ctcaaaaggg tgtacatctc 2220

atcaggcatg caatatacaa aacagctgag ctaggaggac agtttgttct tctgggctca 2280

agtccagtac cacacattca aagagagttt gagggtattg cagaccattt tcagaacaat 2340

aacaatatcc gactgctttt gaagtatgat gattctttat cccattggat ttatgcagca 2400

tctgacatgt tcattgttcc atccatgttt gagccatgtg gcctcacaca gatgattgcc 2460

atgagatatg gttctgtgcc gattgttcgg aaaaccggtg gattgaatga cagtgtcttc 2520

gatttcgacg acgaaacgat acctaaggag ctgcggaatg gctttacgtt tgtgcatcct 2580

gatgaaaagg ctctaagtgg tgcaatggag agagcgttca actactacaa tagaaagcct 2640

gaggtctgga aacagctggt gcagaaggac atgaggatag atttcagctg ggcctcttca 2700

gcttcccagt acgaagatat ctatcaaaga gcagtggctc gagcgagggc agcagcatga 2760

<210> 4
<211> 2745
<212> DNA
<213> Triticum aestivum

<400> 4
atggcgtgct ccgcggcggc gggcgtcgag gcgaccgccc tcctgtcccc gcgctgcccc 60

gccccttccc cgcccgacgg ccgctcccgc cgccgcctcg ccctcgcttc ccgcacgcgc 120

caccgcagcc tcagggcggc cgcgcagcgc cctcacaaga gcgcaaccgg cgccgacccc 180

ctttataaca acagggccaa tgtgcggagc gacgaggcgt cggtttccgc tgaaaaagaa 240

cggcaaagga aatacaacga tggagatggc atatcaaacc ttaagctgga agatttggta 300

ggaatgatac agaacaccga gaagaatata cttcttttga atcaagcccg tcttcaggca 360

atggaacacg ctgataaagt tcttaaagaa aaggaagcct tgcagagaaa gataaacatt 420

ttagagacga ggttgtcaga aacagatgaa caacataagc tttcaagtga agggaatttc 480

agtgactctc cactagcatt ggagcttggt attctaaagg aagagaacat tctactgaag 540

gaggacatag aatttttcaa aacaaagctt atagaggttg ccgagataga ggagggtata 600

ttcaaattgg agaaggagca tgctctttta gatgcttccc ttagggagct ggagtctagg 660

tttatagccg cccaagcaga tacgatgaaa cttggtccta gggatgcctg gtgggagaaa 720

gtagaaaaat tggaagactt gcttgagacc acagcaaacc aagtagagca tgctgctgtg 780

atattggacc acaatcatga tctgcaggat aggcttgaca atttagaggc atcactgcaa 840

gcagcaaata tttcaaagtt ctcttgttct cttgttgatc ttttgcagca aaaggtcaaa 900
ttggtagaag accgcttcca agcatgtaac agcgaaatgc attctcagat tgaactgtac 960
gagcattcaa tagtggaatt tcatgatact cttagcaaac taatagagga aagtgagaaa 1020
agatcactgg agaattttac aggaaacatg ccttcggaac tatggagcaa aatttccctt 1080
ttaattgatg gatggttact ggagaagaaa atatcttaca atgacgcaag tatgttgcga 1140
gaaatggttc agaaaaggga cagtcgtctt cgggaagcat acttgtcata cagaggtacc 1200
gaaaacaggg aagttatgga caacttactt aagatggcat taccaggaac cagttctggt 1260
ttgcacatcg ctcacatagc agcagagatg gctcctgtcg cgaaggttgg tggcctggca 1320
gatgtgatat ctggtcttgg gaaggcactt cagaaaaaag gccatctagt agagatcatt 1380
cttcccaaat acgactgcat gcaggttgac caagttagca atctaaaggt tttagatgtt 1440
cttgtgcagt cctactttga aggaaatatg ttcaacaaca aaatttggac cgggactgtt 1500
gaaggcctac ccgtgtactt tattgagcca cagcatccag cgatgttctt ttcgagggct 1560
cagtactatg gagagcatga tgacttcaaa cgtttttcat acttcagccg tgcggcacta 1620
gaattacttt atcaatctgg gaagaaagtt gatataatcc actgccatga ctggcaaact 1680
gcatttgttg cacctcttta ttgggatgta tatgcaaatc taggcttcaa ctcagctaga 1740
atttgcttca cctgtcataa ttttgaatac caaggaactg ctccagctcg tgatttagca 1800
tggtgtggtc ttgatgttga gcacctagac agaccagaca ggatgcggga caattcgcat 1860
ggcagaataa atgctgttaa gggagcagtt gtgtattcaa acatcgtgac aactgtctcg 1920
ccaacatatg cactagaggt tcgctcagag ggtgggcgtg gactccaaga tacacttaaa 1980
gtacattcca ggaaatttct tgggatactt aatggaatcg acacagatac atggaaccct 2040
tgcacagata ggtatctcaa ggtccagtat aatgctaagg atctccaggg aaaggcagcc 2100
aacaaagcag ccctcagaga gcaactaaac ctggcttctg catatccttc acaaccactg 2160
gttggttgca ttaccaggct ggttgctcag aagggtgtac atcttatcag gcatgcaata 2220
tacaaaacag ctgaattagg aggacagttt gtccttctgg gttcaagtcc agtaccagaa 2280
attcagaggg agtttgaagg tattgcagac cattttcaga acaacaacaa tatccggctg 2340
attttgaagt atgatgatgc gctgtctcat tgcatatatg ctgcgtctga catgttcatt 2400
gttccctcta tatttgagcc atgtggcctc actcagatga tagccatgag atatggttct 2460
gtgccaatcg ttcggaaaac tggtgggctg aatgacagtg tctttgactt cgatgacgaa 2520
acaataccca tggaggtgcg gaacggcttt acatttgtca aggccgacga gcagggccta 2580
agcagcgcga tggagagggc gttcaactgc tacacgagga agcccgaggt gtggaaacag 2640
cttgtgcaga aagacatgac gatcgatttc agctgggaca cctcggcttc gcagtacgag 2700

gacatctacc agaaggcggt ggctcgagcg agggcagtgg cgtga 2745

<210> 5
<211> 2625
<212> DNA
<213> Vigna unguiculata

<400> 5
atgataaaaa atgcagagaa aaatatcctt cttctgaatc aagctagagt tcatgcactt 60

gaagaccttg aaaagattct tgccgaaaag gaagccttac aaggagaaat taatgtctta 120

gcgatgagat tggccgagtc tgatgtgcgg attgaagttg cggcccagga aaagacacgt 180

gttgaactct tggagggtga gttggagaaa ctgcgtagtg aattggcgca aaagggcagt 240

attgaaggaa gagatgctga attgcatgaa cttcaaaatg gagtatttag tgatgcaatt 300

acaaacaatc taagtcataa tgataagatc cattccctta ccgaggagct aaattcaatt 360

agagaagaaa atgccacttt gaagaatgcc atagaatcat ttaaagcaca gctcaatgat 420

gttgcaaaca atgatgaacg tttagcggta ttggaaaaag aacgcttatc tttgcgatca 480

gccctaaaag acatggaatc aaaactgtca atatttccag aagatgtatc tgaactgtct 540

acacttagag ttgaatgcaa ggatttgtct gataaggttg aaaatttgca attgctgcta 600

gacaaagcaa ccaaacaaga cagccaagct gttacagtgc tgcaacaaaa ccaggatctt 660

caaaggaagg ttgataaatt ggaagcatcc ctcgaagaag ctaacatcta taagctgtca 720

tctgataaat tgcaaaaatc taatgaacta atgcaacaaa agataaaact attggagagc 780

cagctacaaa aatcagatga agatataaac tcttacgttc agttgtatca gcagtcagtg 840

aaggaatttc aagatacact cgatcttctg aaaaaagaaa gcaagagaag ggcgccggat 900

gaaccagtgg aagatatgcc gtgggaattc tggagtcggt tgttgcttct aattgatggt 960

tgggcacttg aaaagaagat atcagtagat gatgcaaagc ttttgagaga aaaggtctgg 1020

aagagagaca agagtgttag tgatgtatat atggcttaca aagaaaagac cgaacatgag 1080

gctatatctg catttcttgg gcttacatca tctgcaacaa gtccaggatt gtacgtgatt 1140

catattgcag cagagatggc accagtagct aaggttggtg gcttggggga tgttatcagt 1200

ggtctaagta aagcattgca aaagaaagga caccttgttg aaattatact tcccaaatat 1260

gactgtatgc aatatgaccg cattggtgac ttaagagctt tggatgtggt gatagagtca 1320

tactttgatg gtcaattatt caaaaataaa atctgggttg gcactgttga aggccttcct 1380

gtttatttca ttgagcctca tcatcctggt aagttctttt ggagaggaga ttattatggc 1440

gcgcatgacg acttcagaag attttcatat tttagccgtg cagctcttga gtttcttctt 1500

caagctggca agaagccaga tatcatccat tgccatgatt ggcagacagc atttattgct 1560

cctctatatt gggatgtata tgccccaaaa ggattaaatt cagctaggat atgttttaca 1620

tgccacaact ttgagtatca agggactgca ggagcttcag agttggaagc atgtggtctt 1680

gatagccacc agctaaacag accagataga atgcaggaca actcagcaca taacagagtc 1740

aattctgtca agggtgcagt tgtttactca aacattgtaa caacagtgtc ccccacttat 1800

gctcaagaag tgagaactgc agagggaggt aaaggcctcc attcaactct ctctacccac 1860

tccaaaaagt tcatcggtat tcttaatggt attgacactg atatatggaa tcctgccact 1920

gatccatttc ttcaagtcca gtacaatgca aatgatcttc aagggaaatc agagaataag 1980

gaagccttga gaaggaatct aggtctttct tctgcagacg ttaggaggcc attggttggc 2040

tgcattacaa gattggttcc acaaaaaggt gttcatctta tccgacatgc aatatatcta 2100

acattggaac tgggaggaca atttgttcta cttggttcaa gtccagtccc tcatattcag 2160

agagaatttg agggcattgc caaccacttt cagaatcacg accacataag gttgatattg 2220

aagtatgatg agtctctctc ccatgctatt tatgctgcat ctgacatgtt catcattcca 2280

tcaatctttg aaccttgtgg cctaacacag atgatatcaa tgagatatgg tgccatacct 2340

attgccagaa aaaccggtgg cctaaatgac agtgtttttg acgttgatga tgatacaata 2400

ccttcacagt ttcgaaatgg atttacattc ttgaatgccg atgagaaggg aataaacgat 2460

gccttagtac gtgcaattaa tctcttcaca aacgatccta agagttggaa gcaacttgtt 2520

cagaaggaca tgaacataga tttcagctgg gattcttcag cagcacaata tgaggagctc 2580

tactcaaagt ctgtgactag aggaagggca acaaaacgtg cttaa 2625

<210> 6
<211> 11465
<212> DNA
<213> Arabidopsis thaliana

<400> 6
gagatttttt cacaaaacct ccattgaagc aacttttgta gctttcttcg tttaggtatc 60

taattaaaca tgacgacgaa gctatcgagc ttctgtttct taacccatgg attagcagga 120

atctcctgcg agagagaaca tggaagttct cgtcgatttt tctatctccc ttctcgtcga 180

ttagtttcta cttcgtgtaa gatgcgacaa caacgtggtt tcgagtgagt tttttttttt 240

tgtcgatttc tgtgttgggg tttgagaatt tatgtaccaa ttcgattgat ttatgttggc 300

aggatatatg ccaacgtaaa aatgagggca atcgattgta ctgaatcgga ttttcaaggg 360

tctggccaaa actattccgt gggcacctgg cacacgccct ggagtccggc ccgtttccag 420

ttgagggttg tctacgctta gatgagaagg aaagttgtcc aagacgaatc ccagtgtcct 480

attaccaata gccgacggta tcgataagct tgatgtacat ggtcgataag aaaaggcaat 540

ttgtagatgt taattcccat cttgaaagaa atatagttta aatatttatt gataaaataa 600

caagtcaggt attatagtcc aagcaaaaac ataaatttat tgatgcaagt ttaaattcag 660

aaatatttca ataactgatt atatcagctg gtacattgcc gtagatgaaa gactgagtgc 720

gatattatgt gtaatacata aattgatgat atagctagct tagctcatcg ggggatccat 780

cggatcctag acgcgtgaga tcagatctcg gtgacgggca ggaccggacg gggcggtacc 840

ggcaggctga agtccagctg ccagaaaccc acgtcatgcc agttcccgtg cttgaagccg 900

gccgcccgca gcatgccgcg gggggcatat ccgagcgcct cgtgcatgcg cacgctcggg 960

tcgttgggca gcccgatgac agcgaccacg ctcttgaagc cctgtgcctc cagggacttc 1020

agcaggtggg tgtagagcgt ggagcccagt cccgtccgct ggtggcgggg ggagacgtac 1080

acggtcgact cggccgtcca gtcgtaggcg ttgcgtgcct tccaggggcc cgcgtaggcg 1140

atgccggcga cctcgccgtc cacctcggcg acgagccagg gatagcgctc ccgcagacgg 1200

acgaggtcgt ccgtccactc ctgcggttcc tgcggctcgg tacggaagtt gaccgtgctt 1260

gtctcgatgt agtggttgac gatggtgcag accgccggca tgtccgcctc ggtggcacgg 1320

cggatgtcgg ccgggcgtcg ttctgggctc atggatccga tttgtagaga gagactggtg 1380

atttcagcgt gtcctctcca aatgaaatga acttccttat atagaggaag ggtcttgcga 1440

aggatagtgg gattgtgcgt catcccttac gtcagtggag atatcacatc aatccacttg 1500

ctttgaagac gtggttggaa cgtcttcttt ttccacgatg ctcctcgtgg gtgggggtcc 1560

atctttggga ccactgtcgg cagaggcatc ttgaacgata gcctttcctt tatcgcaatg 1620

atggcatttg taggtgccac cttccttttc tactgtcctt ttgatgaagt gacagatagc 1680

tgggcaatgg aatccgagga ggtttcccga tattaccctt tgttgaaaag tctcaatagc 1740

cctttggtct tctgagactg tatctttgat attcttggag tagacgagag tgtcgtgctc 1800

caccatgttg acgaagattt tcttcttgtc attgagtcgt aaaagactct gtatgaactg 1860

ttcgccagtc ttcacggcga gttctgttag atcctcgatc tgaatttttg actccatggc 1920

ctttgattca gtaggaacta ctttcttaga gactccaatc tctattactt gccttggttt 1980

atgaagcaag ccttgaatcg tccatactgg aatagtactt ctgatcttga gaaatatatc 2040

tttctctgtg ttcttgatgc agttagtcct gaatcttttg actgcatctt taaccttctt 2100

gggaaggtat ttgatctcct ggagattatt actcgggtag atcgtcttga tgagacctgc 2160

cgcgtaggcc tctctaacca tctgtgggtc agcattcttt ctgaaattga agaggctaat 2220

cttctcatta tcggtggtga acatggtatc gtcaccttct ccgtcgaact ttcttcctag 2280

atcgtagaga tagagaaagt cgtccatggt gatctccggg gcaaaggaga tccgtcaatt 2340

ccgattcatt aatgcagctg gcacgacagg tttcccgact ggaaagcggg cagtgagcgc 2400

aacgcaatta atgtgagtta gctcactcat taggcacccc aggctttaca ctttatgctt 2460

ccggctcgta taatgtgtgg aattgtgagc ggataacaat ttcacacagg aaacaggatc 2520

atgagcggag aattaaggga gtcacgttat gacccccgcc gatgacgcgg gacaagccgt 2580

tttacgtttg gaactgacag aaccgcaacg attgaaggag ccactcagcc gcgggtttct 2640

ggagtttaat gagctaagca catacgtcag aaaccattat tgcgcgttca aaagtcgcct 2700

aaggtcacta tcagctagca aatatttctt gtcaaaaatg ctccactgac gttccataaa 2760

ttcccctcgg tatccaatta gagtctcata ttcactctca atcaaagatc cggcccatga 2820

tcatgtggat tgaacaagat ggattgcacg caggttctcc ggccgcttgg gtggagaggc 2880

tattcggcta tgactgggca caacagacaa tcggctgctc tgatgccgcc gtgttccggc 2940

tgtcagcgca ggggcgcccg gttctttttg tcaagaccga cctgtccggt gccctgaatg 3000

aactgcagga cgaggcagcg cggctatcgt ggctggccac gacgggcgtt ccttgcgcag 3060

ctgtgctcga cgttgtcact gaagcgggaa gggactggct gctattgggc gaagtgccgg 3120

ggcaggatct cctgtcatct caccttgctc ctgccgagaa agtatccatc atggctgatg 3180

caatgcggcg gctgcatacg cttgatccgg ctacctgccc attcgaccac caagcgaaac 3240

atcgcatcga gcgagcacgt actcggatgg aagccggtct tgtcgatcag gatgatctgg 3300

acgaagagca tcaggggctc gcgccagccg aactgttcgc caggctcaag gcgcgcatgc 3360

ccgacggcga ggatctcgtc gtgacccatg gcgatgcctg cttgccgaat atcatggtgg 3420

aaaatggccg cttttctgga ttcatcgact gtggccggct gggtgtggcg gaccgctatc 3480

aggacatagc gttggctacc cgtgatattg ctgaagagct tggcggcgaa tgggctgacc 3540

gcttcctcgt gctttacggt atcgccgctc ccgattcgca gcgcatcgcc ttctatcgcc 3600

ttcttgacga gttcttctga gcgggactct ggggttcgaa atgaccgacc aagcgacgcc 3660

caacctgcca tcacgagatt tcgattccac cgccgccttc tatgaaaggt tgggcttcgg 3720

aatcgttttc cgggacgccg gctggatgat cctccagcgc ggggatctca tgctggagtt 3780

cttcgcccac cccctgcttt aatgagatat gcgagacgcc tatgatcgca tgatatttgc 3840

tttcaattct gttgtgcacg ttgtaaaaaa cctgagcatg tgtagctcag atccttaccg 3900

ccggtttcgg ttcattctaa tgaatatatc acccgttact atcgtatttt tatgaataat 3960

attctccgtt caatttactg attgtaccct actacttata tgtacaatat taaaatgaaa 4020

acaatatatt gtgctgaata ggtttatagc gacatctatg atagagcgcc acaataacaa 4080

acaattgcgt tttattatta caaatccaat tttaaaaaaa gcggcagaac cggtcaaacc 4140

taaaagactg attacataaa tcttattcaa atttcaaaag gccccagggg ctagtatcta 4200

cgacacaccg agcggcgaac taataacgtt cactgaaggg aactccggtt ccccgccggc 4260

gcgcatgggt gagattcctt gaagttgagt attggccgtc cgctctaccg aaagttacgg 4320

gcaccattca acccggtcca gcacggcggc cgggtaaccg acttgctgcc ccgagaatta 4380

tgcagcattt ttttggtgta tgtgggcccc aaatgaagtg caggtcaaac cttgacagtg 4440

acgacaaatc gttgggcggg tccagggcga attttgcgac aacatgtcga ggctcagcag 4500

gggctcgatc ccctcgatcg aattcgatct agtaacatag atgacaccgc gcgcgataat 4560

ttatcctagt ttgcgcgcta tattttgttt tctatcgcgt attaaatgta taattgcggg 4620

actctaatca taaaaaccca tctcataaat aacgtcatgc attacatgtt aattattaca 4680

tgcttaacgt aattcaacag aaattatatg ataatcatcg caagaccggc aacaggattc 4740

aatcttaaga aactttattg ccaaatgttt gaacgatcga gctcaattcc ccaccgaggc 4800

tgtagccgac gatggtgcgc caggagagtt gttgattcat tgtttgcctc cctgctgcgg 4860

tttttcaccg aagttcatgc cagtccagcg tttttgcagc agaaaagccg ccgacttcgg 4920

tttgcggtcg cgagtgaaga tccctttctt gttaccgcca acgcgcaata tgccttgcga 4980

ggtcgcaaaa tcggcgaaat tccatacctg ttcaccgacg acggcgctga cgcgatcaaa 5040

gacgcggtga tacatatcca gccatgcaca ctgatactct tcactccaca tgtcggtgta 5100

cattgagtgc agcccggcta acgtatccac gccgtattcg gtgatgataa tcggctgatg 5160

cagtttctcc tgccaggcca gaagttcttt ttccagtacc ttctctgccg tttccaaatc 5220

gccgctttgg acataccatc cgtaataacg gttcaggcac agcacatcaa agagatcgct 5280

gatggtatcg gtgtgagcgt cgcagaacat tacattgacg caggtgatcg gacgcgtcgg 5340

gtcgagttta cgcgttgctt ccgccagtgg cgaaatattc ccgtgcactt gcggacgggt 5400

atccggttcg ttggcaatac tccacatcac cacgcttggg tggtttttgt cacgcgctat 5460

cagctcttta atcgcctgta agtgcgcttg ctgagtttcc ccgttgactg cctcttcgct 5520

gtacagttct ttcggcttgt tgcccgcttc gaaaccaatg cctaaagaga ggttaaagcc 5580

gacagcagca gtttcatcaa tcaccacgat gccatgttca tctgcccagt cgagcatctc 5640

ttcagcgtaa gggtaatgcg aggtacggta ggagttggcc ccaatccagt ccattaatgc 5700

gtggtcgtgc accatcagca cgttatcgaa tcctttgcca cgtaagtccg catcttcatg 5760

acgaccaaag ccagtaaagt agaacggttt gtggttaatc aggaactgtt ggcccttcac 5820

tgccactgac cggatgccga cgcgaagcgg gtagatatca cactctgtct ggcttttggc 5880

tgtgacgcac agttcataga gataaccttc acccggttgc cagaggtgcg gattcaccac 5940

ttgcaaagtc ccgctagtgc cttgtccagt tgcaaccacc tgttgatccg catcacgcag 6000

ttcaacgctg acatcaccat tggccaccac ctgccagtca acagacgcgt ggttacagtc 6060
ttgcgcgaca tgcgtcacca cggtgatatc gtccacccag gtgttcggcg tggtgtagag 6120
cattacgctg cgatggattc cggcatagtt aaagaaatca tggaagtaag actgcttttt 6180
cttgccgttt tcgtcggtaa tcaccattcc cggcgggata gtctgccagt tcagttcgtt 6240
gttcacacaa acggtgatac gtacactttt cccggcaata acatacggcg tgacatcggc 6300
ttcaaatggc gtatagccgc cctgatgctc catcacttcc tgattattga cccacacttt 6360
gccgtaatga gtgaccgcat cgaaacgcag cacgatacgc tggcctgccc aacctttcgg 6420
tataaagact tcgcgctgat accagacgtt gcccgcataa ttacgaatat ctgcatcggc 6480
gaactgatcg ttaaaactgc ctggcacagc aattgcccgg ctttcttgta acgcgctttc 6540
ccaccaacgc tgatcaattc cacagttttc gcgatccaga ctgaatgccc acaggccgtc 6600
gagttttttg atttcacggg ttggggtttc tacaggacgt aacataaggg actgaccacc 6660
ccaagtgcag cgtttaataa atcgtgtgac aggatatatc ttgtggtcaa acttggcatt 6720
tttgaatttt gcattataaa gttttgtttt tctcagcttt agagttatgg tgaatgctca 6780
tagatgtgaa cgtgtttata attatcttga tttagattca tttgtatgag tttggagagg 6840
actatgagaa ttaaaactct gattttactt agtgatgaga tcaaaatgtt gcatttgatt 6900
tttgatatga atccaatttt tgttaaagct agcctctttg tagcttaagt tcctttcatt 6960
tcccatttga tttgcattac accaagtcaa aaaaaatctg atatcttgac taatttgcag 7020
ctctagtaaa agacaagagg tcaagaaagg ctcccctaaa ccgatcctat ctataaattc 7080
aggtcttcag agcaacaatg atgaggaatc tgatctggag aatggttctg cggacagtgt 7140
cccgagtctt aagtcagatg ctgaaaaggg tagtagtatt catggcagca tagatatgaa 7200
tcatgctgat gagaatcttg aaaagaaaga tgatatacaa acaactgaag ttactcggcg 7260
caagagtaaa actgcaaaga agaaagggga gagtattcat gctacgattg atattggaca 7320
tgatgatggg aagaatttag ataatattac tgtgcctgag gttgcaaaag ctttggtatg 7380
gatttctttt tagtgttaat ggtatatcgt aactctcttc ttctgaaaaa ggccttcttt 7440
tgatcactta ttgtaaatca ttttttcagt cccttaacaa aagtgaaggc gagcagattt 7500
cagatggaca gtttggggaa ctaatgacaa tgataagaag tgcagaaaaa agtagaaatt 7560
ttcttatctg tattagttat gtttgtttcc tgatcttgtg aaagcctttg ttaattcctg 7620
atgtcttatt gtgtcagata ttcttcggct tgatgaagca cgggccactg ctcttgacga 7680
ccttaacaag attcttagtg ataaggaagc gttgcaggga gaaatcaatg tcttggaaat 7740
gaaattgtct gagactgatg aaaggattaa aactgctgct caagagaaag cacatgtaga 7800
gcttctggaa gaacagttag agaagcttcg tcatgaaatg atctccccta tagaaagtga 7860

tggttatgtt ctagctctta gcaaagagct tgaaacattg aagctggaga atctatcttt 7920

gagaaacgat atagaaatgc ttaagtcaga acttgacagt gttaaagata ccggtgaacg 7980

tgtggttgtg ttggaaaagg agtgctccgg tttggaatct tctgtaaagg atttggaatc 8040

taagttatcg gtttctcagg aagatgtctc gcagctttct actcttaaaa tcgaatgcac 8100

tgatctatgg gcgaaggtag agactctaca gctgttgtta gatagagcta ccaaacaagc 8160

agagcaagca gttatagtgt tacaacagaa ccaagatcta agaaataagg tcgacaaaat 8220

tgaggaatca cttaaagaag ccaacgttta taaagaatct tcagagaaaa ttcaacagta 8280

caatgagcta atgcaacata aggtgacatt acttgaggag cggctcgaaa agtctgatgc 8340

agagatattc tcatatgttc agttatatca agaatcgata aaagaattcc aggaaacact 8400

tgaaagtttg aaggaagaaa gcaagaaaaa atcaagagat gaaccggttg atgatatgcc 8460

ttgggattat tggagtcggt tacttttaac tgttgatgga tggctgcttg aaaagaaaat 8520

agcaagcaac gatgctgact tactgagaga catggtatgg aagaaagatc gaagaattca 8580

tgatacctat attgacgtca aagataagaa tgaacgtgat gccatctctg catttctcaa 8640

gcttgtctca tctccaacaa ggttatcatc gtctattgga aatgcttttt tttttttcag 8700

tttttaaagc ttttgaaaaa agtaacctat tttcttcatc ttgtgttgat tctagttcag 8760

gattgtatgt cgttcacatt gcagctgaga tggcacctgt agctaaggtg tgttcagcta 8820

aaccctcaaa aatatctctt gcttacatgt tagattttcc gaaatagaag cattgcatgg 8880

taaattttca actttatccc cattccaaat ttccttctgt aatttctgta ggtgggaggt 8940

ttgggagatg ttgtggcagg tcttggtaag gcattgcaaa gaaaaggtca tctggtggag 9000

attattcttc ccaaatatga ctgtatgcag tatgatcgcg tgcgtgactt aagggtaata 9060

ttgttgtcca atatgatgaa aacaaaatta taagcggact ttaactttta acgttttagt 9120

tggtttgatt tatttcactg caggctttgg atactgttgt ggagtcatat tttgatggga 9180

agttatataa aaacaaaatc tggattggca ctgttgaagg tgaggagact ttttcttttc 9240

tcgtgtttgt gtcttttggt tttcaaaatg atttgaagtt tctctctatt gacaggttta 9300

cctgtacatt tcattgaacc tcaacatcca agcaaattct tctggagagg acagttttat 9360

ggagagcaag atgatttcag acgcttctcg tattttagcc gagctgcact agagttgctt 9420

cttcagtccg gcaaaaaacc cgacatcata cattgtcatg actggcaaac agcttttgtt 9480

gtacggcttg atcttatttt gaagcagtaa cgtaattcat tttgtttagg atacatcttg 9540

aagctttctt tttttttttt ttttataaca ggcgccgctg tattgggatc tgtatgctcc 9600

aaagggatta gattctgcaa gaatatgctt tacatgtcat aattttgagt atcaaggtac 9660

cgcgtctgct tcagaattgg gatcttgcgg gcttgatgtt aaccagttaa atagaccaga 9720
cagaatgcag gatcactcgt ctggagatag agtcaatcct gttaaggtaa ataatgatca 9780
caaagccaat ataaaccgtg agagaaataa attcgacatt ttactcttgt tcatctctct 9840
ttctcctttt ggtccagggc gctataattt tctcaaacat tgtaacaacc gtgtccccta 9900
cttatgcaca agaagttcga acagctgagg tagaagaatt tcctttggta actttccaat 9960
gttaattaag taaagctttc ttactaaaat gtcttatgta tctacaggga ggaaaaggac 10020
tccattcaac actcaatttt cactccaaga aattcattgg aatcctcaac ggcattgaca 10080
cagattcatg gaatcctgcc accgacccct tcctcaaggc tcagttcaac gctaaagatc 10140
tacaagggaa agaagaaaac aaacacgccc ttagaaagca gcttggactt tcttcagcgg 10200
agtcaagacg gcctttggta acttaatgtt tccgcgaata ctgcgagagt cttgagcata 10260
cagattcagt taatttatta cacatttatt ttggtatagg ttggttgcat aacaagatta 10320
gtaccacaga aaggagttca tctaatcaga catgccatat acagaacatt agagttaggt 10380
ggacaatttg tacttcttgg ttctagcccg gttccacata ttcaggtaac atcccggtcc 10440
cttagatgct cttgatttct tctatttaat ctccagtttc gtggttttga gctttgaggc 10500
attttgtttt tttgtagagg gaatttgaag gtattgaaca acagtttaaa agccatgatc 10560
atgtccggtt gttactgaag tacgatgaag ctctgtctca tacgatttac gcagcctccg 10620
atttgttcat cattccgtca atttttgagc cttgtggact tacacaggta ccatacaaaa 10680
catgatcttt ttttgtgtgc aaaacatgat ctctagaagg aaatcgaacc agtggtgtga 10740
ctaaatgtct gctaatatca ttgtcggatt ctctctttgc acagatgatc gctatgagat 10800
atggctccat tccaattgcg cgaaaaactg gaggcttaaa tgacaggtaa aaccaaaaac 10860
ttaagaactg atctgatctt cagatgacat tggtttcaaa gatcatttct tttgtttcag 10920
tgtcttcgac attgatgatg atacaatacc aacacagttt caaaatggat ttacatttca 10980
aaccgcggac gaacaggtaa tttcacgatg ctctcatctt tatcaatctt tgctcatcat 11040
agctgaaaat agggatggaa atttatttag tttggttcag ttttacatgt ccgtctttag 11100
ctgaaaaagg aaatgtgaaa aaacagggtt tcaattatgc gttggagcga gcatttaatc 11160
actacaagaa agatgaagag aaatggatga gacttgtaga gaaagtgatg agtatagatt 11220
tcagctgggg atcatcagct acacaatacg aagaactcta cacgagatca gtgtccagag 11280
caagagctgt tcctaatcgc acgtgagtat cacatagctc ctagagattc tttcttgtaa 11340
cacattttta taattttact tgttcataat agaaaaaaat atttgggagt ttcaatcaca 11400
ttcaaatgat ttatactccc tttatttcat atgataataa atgttttgta cacattaaaa 11460
aaaaa 11465

<210>  7
<211>  1071
<212>  PRT
<213>  Arabidopsis thaliana

<400>  7

Met Thr Thr Lys Leu Ser Ser Phe Cys Phe Leu Thr His Gly Leu Ala
1               5                   10                  15

Gly Ile Ser Cys Glu Arg Glu His Gly Ser Ser Arg Arg Phe Phe Tyr
            20                  25                  30

Leu Pro Ser Arg Arg Leu Val Ser Thr Ser Cys Lys Met Arg Gln Gln
        35                  40                  45

Arg Gly Phe Asp Ser Ser Lys Arg Gln Glu Val Lys Lys Gly Ser Pro
    50                  55                  60

Lys Pro Ile Leu Ser Ile Asn Ser Gly Leu Gln Ser Asn Asn Asp Glu
65                  70                  75                  80

Glu Ser Asp Leu Glu Asn Gly Ser Ala Asp Ser Val Pro Ser Leu Lys
                85                  90                  95

Ser Asp Ala Glu Lys Gly Ser Ser Ile His Gly Ser Ile Asp Met Asn
            100                 105                 110

His Ala Asp Glu Asn Leu Glu Lys Lys Asp Asp Ile Gln Thr Thr Glu
        115                 120                 125

Val Thr Arg Arg Lys Ser Lys Thr Ala Lys Lys Lys Gly Glu Ser Ile
    130                 135                 140

His Ala Thr Ile Asp Ile Gly His Asp Asp Gly Lys Asn Leu Asp Asn
145                 150                 155                 160

Ile Thr Val Pro Glu Val Ala Lys Ala Leu Ser Leu Asn Lys Ser Glu
                165                 170                 175

Gly Glu Gln Ile Ser Asp Gly Gln Phe Gly Glu Leu Met Thr Met Ile
            180                 185                 190

Arg Ser Ala Glu Lys Asn Ile Leu Arg Leu Asp Glu Ala Arg Ala Thr
        195                 200                 205

Ala Leu Asp Asp Leu Asn Lys Ile Leu Ser Asp Lys Glu Ala Leu Gln
    210                 215                 220

Gly Glu Ile Asn Val Leu Glu Met Lys Leu Ser Glu Thr Asp Glu Arg
225                 230                 235                 240

Ile Lys Thr Ala Ala Gln Glu Lys Ala His Val Glu Leu Leu Glu Glu
                245                 250                 255

Gln Leu Glu Lys Leu Arg His Glu Met Ile Ser Pro Ile Glu Ser Asp
            260                 265                 270

Gly Tyr Val Leu Ala Leu Ser Lys Glu Leu Glu Thr Leu Lys Leu Glu
        275                 280                 285

Asn Leu Ser Leu Arg Asn Asp Ile Glu Met Leu Lys Ser Glu Leu Asp
    290                 295                 300

Ser Val Lys Asp Thr Gly Glu Arg Val Val Val Leu Glu Lys Glu Cys
305                 310                 315                 320

Ser Gly Leu Glu Ser Ser Val Lys Asp Leu Glu Ser Lys Leu Ser Val
                325                 330                 335

Ser Gln Glu Asp Val Ser Gln Leu Ser Thr Leu Lys Ile Glu Cys Thr
            340                 345                 350

Asp Leu Trp Ala Lys Val Glu Thr Leu Gln Leu Leu Leu Asp Arg Ala
        355                 360                 365

Thr Lys Gln Ala Glu Gln Ala Val Ile Val Leu Gln Gln Asn Gln Asp
    370                 375                 380

Leu Arg Asn Lys Val Asp Lys Ile Glu Glu Ser Leu Lys Glu Ala Asn
385                 390                 395                 400

Val Tyr Lys Glu Ser Ser Glu Lys Ile Gln Gln Tyr Asn Glu Leu Met
                405                 410                 415

Gln His Lys Val Thr Leu Leu Glu Glu Arg Leu Glu Lys Ser Asp Ala
            420                 425                 430

Glu Ile Phe Ser Tyr Val Gln Leu Tyr Gln Glu Ser Ile Lys Glu Phe
        435                 440                 445

Gln Glu Thr Leu Glu Ser Leu Lys Glu Glu Ser Lys Lys Lys Ser Arg

450                 455                 460

Asp Glu Pro Val Asp Asp Met Pro Trp Asp Tyr Trp Ser Arg Leu Leu
465                 470                 475                 480

Leu Thr Val Asp Gly Trp Leu Leu Glu Lys Lys Ile Ala Ser Asn Asp
                485                 490                 495

Ala Asp Leu Leu Arg Asp Met Val Trp Lys Lys Asp Arg Arg Ile His
            500                 505                 510

Asp Thr Tyr Ile Asp Val Lys Asp Lys Asn Glu Leu Phe Lys Ala Phe
        515                 520                 525

Glu Lys Ser Asn Leu Phe Ser Ser Ser Cys Val Asp Ser Ser Ser Gly
    530                 535                 540

Leu Tyr Val Val His Ile Ala Ala Glu Met Ala Pro Val Ala Lys Val
545                 550                 555                 560

Gly Gly Leu Gly Asp Val Val Ala Gly Leu Gly Lys Ala Leu Gln Arg
                565                 570                 575

Lys Gly His Leu Val Glu Ile Ile Leu Pro Lys Tyr Asp Cys Met Gln
            580                 585                 590

Tyr Asp Arg Val Arg Asp Leu Arg Ala Leu Asp Thr Val Val Glu Ser
        595                 600                 605

Tyr Phe Asp Gly Lys Leu Tyr Lys Asn Lys Ile Trp Ile Gly Thr Val
    610                 615                 620

Glu Gly Leu Pro Val His Phe Ile Glu Pro Gln His Pro Ser Lys Phe
625                 630                 635                 640

Phe Trp Arg Gly Gln Phe Tyr Gly Glu Gln Asp Asp Phe Arg Arg Phe
                645                 650                 655

Ser Tyr Phe Ser Arg Ala Ala Leu Glu Leu Leu Leu Gln Ser Gly Lys
            660                 665                 670

Lys Pro Asp Ile Ile His Cys His Asp Trp Gln Thr Ala Phe Val Ala
        675                 680                 685

Pro Leu Tyr Trp Asp Leu Tyr Ala Pro Lys Gly Leu Asp Ser Ala Arg
    690                 695                 700

Ile Cys Phe Thr Cys His Asn Phe Glu Tyr Gln Gly Thr Ala Ser Ala
705                 710                 715                 720

Ser Glu Leu Gly Ser Cys Gly Leu Asp Val Asn Gln Leu Asn Arg Pro
                725                 730                 735

Asp Arg Met Gln Asp His Ser Ser Gly Asp Arg Val Asn Pro Val Lys
            740                 745                 750

Gly Ala Ile Ile Phe Ser Asn Ile Val Thr Thr Val Ser Pro Thr Tyr
        755                 760                 765

Ala Gln Glu Val Arg Thr Ala Glu Gly Gly Lys Gly Leu His Ser Thr
    770                 775                 780

Leu Asn Phe His Ser Lys Lys Phe Ile Gly Ile Leu Asn Gly Ile Asp
785                 790                 795                 800

Thr Asp Ser Trp Asn Pro Ala Thr Asp Pro Phe Leu Lys Ala Gln Phe
                805                 810                 815

Asn Ala Lys Asp Leu Gln Gly Lys Glu Glu Asn Lys His Ala Leu Arg
            820                 825                 830

Lys Gln Leu Gly Leu Ser Ser Ala Glu Ser Arg Arg Pro Leu Val Gly
        835                 840                 845

Cys Ile Thr Arg Leu Val Pro Gln Lys Gly Val His Leu Ile Arg His
    850                 855                 860

Ala Ile Tyr Arg Thr Leu Glu Leu Gly Gly Gln Phe Val Leu Leu Gly
865                 870                 875                 880

Ser Ser Pro Val Pro His Ile Gln Arg Glu Phe Glu Gly Ile Glu Gln
                885                 890                 895

Gln Phe Lys Ser His Asp His Val Arg Leu Leu Leu Lys Tyr Asp Glu
            900                 905                 910

Ala Leu Ser His Thr Ile Tyr Ala Ala Ser Asp Leu Phe Ile Ile Pro
        915                 920                 925

Ser Ile Phe Glu Pro Cys Gly Leu Thr Gln Met Ile Ala Met Arg Tyr
    930                 935                 940

Gly Ser Ile Pro Ile Ala Arg Lys Thr Gly Gly Leu Asn Asp Ser Val
945                 950                 955                 960

Phe Asp Ile Asp Asp Asp Thr Ile Pro Thr Gln Phe Gln Asn Gly Phe
                965                 970                 975

Thr Phe Gln Thr Ala Asp Glu Gln Leu Lys Ile Gly Met Glu Ile Tyr
            980                 985                 990

Leu Val Trp Phe Ser Phe Thr Cys  Pro Ser Leu Ala Glu  Lys Gly Asn
        995                 1000                 1005

Val Lys  Lys Gln Gly Phe Asn  Tyr Ala Leu Glu Arg  Ala Phe Asn
    1010                 1015                 1020

His Tyr  Lys Lys Asp Glu Glu  Lys Trp Met Arg Leu  Val Glu Lys
    1025                 1030                 1035

Val Met  Ser Ile Asp Phe Ser  Trp Gly Ser Ser Ala  Thr Gln Tyr
    1040                 1045                 1050

Glu Glu  Leu Tyr Thr Arg Ser  Val Ser Arg Ala Arg  Ala Val Pro
    1055                 1060                 1065

Asn Arg  Thr
    1070

<210>  8
<211>  962
<212>  PRT
<213>  Oryza sativa

<400>  8

Met Ala Ala Cys Gly Ala Ala Ala Ala Glu Tyr Ser Ala Leu Leu Ser
1               5                   10                  15

Leu Ser Cys Gly Pro Ile Thr Arg Arg Arg Phe Ala Val Ser Cys Arg
            20                  25                  30

Ala Arg Pro Pro Gly Asn Leu Ser Ala Gln Gln Lys Lys Lys Arg Gly
        35                  40                  45

Lys Asn Ile Ala Pro Lys Gln Arg Ser Ser Asn Ala Lys Leu Leu Leu
    50                  55                  60

Thr Thr Glu Glu Asn Gly Gln Leu Pro Ser Thr Ser Leu Arg Thr Ser

65                  70                  75                  80

Met Glu Arg Pro Gln Lys Ser Thr Ser Ser Glu Asp Asp Thr Asn Gly
                85                  90                  95

Ala Ile Ser Gln Ile Asp Glu Lys Ile Ala Ala Ile Gly Asn Glu Gln
            100                 105                 110

Gln Glu Val Leu Pro Tyr Trp Gly Gly His Ile Leu Ile Ser Ala Tyr
        115                 120                 125

Gln Asp Ile Leu Leu Leu Asn Gln Ala Arg Leu Gln Ala Ile Glu Asp
    130                 135                 140

Val Asp Lys Ile Leu Thr Glu Lys Glu Ala Leu Gln Lys Lys Val Asp
145                 150                 155                 160

Thr Leu Glu Met Asn Leu Ser Lys Ala Leu Ala Thr Lys Gly Asn Ile
                165                 170                 175

Asn Thr Asp Ile Pro Gly Asp His Leu Glu Lys Phe Thr Lys Glu Ile
            180                 185                 190

Leu Ile Glu Ser Ala Leu Ser Gly Gly Asn Pro Ala His Leu Cys Glu
        195                 200                 205

Ser Pro Leu Phe Met Glu Leu Thr Val Leu Lys Glu Glu Asn Met Leu
    210                 215                 220

Leu Lys Ala Asp Ala Gln Phe Leu Lys Ala Lys Ile Val Glu Phe Ala
225                 230                 235                 240

Glu Thr Glu Glu Phe Leu Phe Lys Leu Glu Lys Glu Arg Ser Leu Leu
                245                 250                 255

Asp Ala Thr Val Arg Glu Leu Glu Ala Arg Phe Leu Val Ala Gln Thr
            260                 265                 270

Asp Ile Trp Lys Val Val Pro Leu Gln Tyr Asp Val Trp Met Glu Lys
        275                 280                 285

Val Glu Asn Leu Gln His Met Leu Gly Cys Leu Lys Asn His Val Glu
    290                 295                 300

Lys Tyr Ala Ala Leu Leu Asp Gln His Asp Asp Leu His Asp Lys Ile
305                 310                 315                 320

Asp Glu Leu Glu Ala Ser Leu Lys Glu Gly Lys Thr Ser Glu Phe Ser
                325                 330                 335

Pro Tyr Val Val Glu Leu Leu Gln Gln Lys Leu Lys Ala Ala Lys Ser
            340                 345                 350

His His Gln Ala Gly His Gln Glu Thr Asn Thr His Ile Gln Val Tyr
        355                 360                 365

Gln Gln Leu Thr Glu Glu Phe Gln Asp Asn Leu Gly Lys Leu Ile Glu
    370                 375                 380

Glu Ser Gly Arg Leu Glu His Ser Ala Asn Ser Met Pro Ser Glu Phe
385                 390                 395                 400

Trp Ser His Ile Leu Leu Met Ile Asp Gly Trp Phe Leu Glu Arg Lys
                405                 410                 415

Ile Pro Asn Thr Asp Ala Arg Met Leu Arg Glu Met Ala Trp Lys Arg
            420                 425                 430

Asp Asp Arg Ile Cys Glu Ala Tyr Phe Ala Cys Lys Gly Ala Lys Glu
        435                 440                 445

Ser Asp Val Met Glu Thr Phe Leu Lys Leu Ser Leu Ser Gly Asn Ser
    450                 455                 460

Ser Gly Leu His Ile Val His Ile Ala Ala Glu Met Ala Pro Val Ala
465                 470                 475                 480

Lys Val Gly Gly Leu Ala Asp Val Val Ala Gly Leu Gly Lys Ala Leu
                485                 490                 495

Gln Thr Lys Gly His Leu Val Glu Ile Val Leu Pro Lys Tyr Asp Cys
            500                 505                 510

Met Gln Leu Asp Gln Ile Thr Asn Leu Lys Val Leu Asp Val Val Ile
        515                 520                 525

Gln Ser Tyr Phe Asp Gly Asn Leu Phe Ser Asn Asn Val Trp Thr Gly
    530                 535                 540

Thr Val Glu Gly Leu Pro Val Tyr Phe Ile Glu Pro Gln His Pro Ser
545                 550                 555                 560

Lys Phe Phe Trp Arg Ala Gln Tyr Tyr Gly Glu His Asp Asp Phe Lys
                565                 570                 575

Arg Tyr Ser Tyr Phe Ser Arg Ala Ala Leu Glu Leu Leu Tyr Gln Ser
            580                 585                 590

Gly Lys Lys Ile Asp Ile Ile His Cys His Asp Trp Gln Thr Ala Phe
        595                 600                 605

Val Ala Pro Leu Tyr Trp Asp Ile Tyr Ala Thr Arg Gly Phe Ser Ser
    610                 615                 620

Ala Arg Ile Cys Phe Thr Cys His Asn Phe Glu Tyr Gln Gly Thr Ala
625                 630                 635                 640

Pro Ala Pro Asp Leu Ser Tyr Cys Gly Leu Asp Val Glu Gln Leu Asp
                645                 650                 655

Arg Pro Asp Arg Met Gln Asp Asn Ala His Gly Arg Ile Asn Val Ala
            660                 665                 670

Lys Gly Gly Ile Val Tyr Ser Asn Ile Val Thr Thr Val Ser Pro Thr
        675                 680                 685

Tyr Ala Leu Glu Val Arg Ser Glu Gly Gly Arg Gly Leu Gln Asp Thr
    690                 695                 700

Leu Lys Met His Ser Arg Lys Phe Val Gly Ile Leu Asn Gly Ile Asp
705                 710                 715                 720

Thr Gly Thr Trp Asn Pro Ser Thr Asp Arg Phe Leu Ala Val Gln Tyr
                725                 730                 735

Ser Ala Thr Asp Leu Gln Gly Lys Ala Ala Asn Lys Ala Phe Leu Arg
            740                 745                 750

Lys Gln Leu Gly Leu Tyr Ser Glu Asp Ala Ser Gln Pro Leu Val Ala
        755                 760                 765

Cys Ile Thr Arg Leu Val Pro Gln Lys Gly Leu His Leu Ile Arg His
    770                 775                 780

Ala Ile Tyr Lys Thr Ala Glu Leu Gly Gly Gln Phe Val Leu Leu Gly
785                 790                 795                 800

Ser Ser Pro Val Pro His Ile Gln Arg Glu Phe Glu Gly Val Ala Asp
                805                 810                 815

Gln Phe Gln Lys Asn Asn Asn Ile Arg Leu Ile Leu Lys Tyr Asp Glu
            820                 825                 830

Ala Leu Ser His Cys Ile Tyr Ala Ala Ser Asp Met Phe Ile Ile Pro
        835                 840                 845

Ser Met Phe Glu Pro Cys Gly Leu Thr Gln Met Ile Ala Met Arg Tyr
    850                 855                 860

Gly Ser Val Pro Ile Val Arg Gln Thr Gly Gly Leu Cys Asp Ser Val
865                 870                 875                 880

Phe Asp Phe Asp Asp Glu Thr Ile Pro Val Glu Leu Arg Asn Gly Phe
                885                 890                 895

Thr Phe Ala Arg Thr Asp Glu Gln Asp Leu Ser Ser Cys Leu Glu Arg
            900                 905                 910

Ala Phe Ser Tyr Tyr Ser Arg Lys Pro Met Val Trp Lys Gln Leu Val
        915                 920                 925

Gln Lys Asp Met Gln Ile Asp Phe Ser Trp Asp Ser Pro Ala Ser Gln
    930                 935                 940

Tyr Glu Asn Leu Tyr Gln Ser Ala Val Ala Gln Ala Arg Gly Ala Ala
945                 950                 955                 960

Gln Thr

<210>  9
<211>  919
<212>  PRT
<213>  Oryza sativa

<400>  9

Met Ala Cys Leu Ala Ala Gly Ala Glu Ala Ala Pro Leu Leu Phe Arg
1               5                   10                  15

Arg Arg Leu Ala Pro Ser Pro Val Ala Ala Arg Arg Arg Leu Leu Val
            20                  25                  30

Ser Cys Arg Ala Arg Arg Arg Gly Leu Arg Leu Val Ala Gln Ser Ala
        35                  40                  45

Gly Ser Arg Gly Cys Gly Val Val Gly Ala Pro Gly Cys Asp Tyr Trp
    50                  55                  60

Val Asn Met Gln Arg Asp Glu Ala Ser Val Ser Ser Asp Lys Glu Arg
65                  70                  75                  80

Gln Glu Lys Tyr Gly Asp Glu Asn Gly Ile Ser Asn Leu Gln Leu Glu
                85                  90                  95

Asp Leu Ile Gln Met Ile Gln Asn Thr Glu Lys Asn Ile Met Leu Leu
            100                 105                 110

Asn Gln Ala Arg Leu Gln Ala Leu Glu His Val Glu Thr Val Leu Lys
        115                 120                 125

Glu Lys Glu Asp Leu Gln Arg Lys Leu Lys Ile Leu Glu Thr Arg Leu
    130                 135                 140

Ser Glu Thr Asp Ala Arg Leu Lys Leu Ser Ala Glu Gly Gln Phe Gly
145                 150                 155                 160

Thr Glu Ile Asn Asp Ser Leu Pro Val Leu Glu Leu Asp Asp Ile Lys
                165                 170                 175

Glu Glu Asn Met Leu Leu Lys Asp Asp Ile Gln Phe Leu Lys Thr Met
            180                 185                 190

Leu Ile Glu Val Ala Glu Thr Glu Asn Ser Ile Phe Thr Leu Glu Lys
        195                 200                 205

Glu Arg Ala Leu Leu Asp Ala Ser Leu Arg Glu Leu Glu Ser Arg Phe
    210                 215                 220

Ile Asp Ala Gln Ala Asp Met Leu Lys Ser Asp Pro Arg Gln Tyr Asp
225                 230                 235                 240

Ala Trp Trp Glu Lys Val Glu Asn Leu Gly Asp Leu Leu Glu Thr Ala
                245                 250                 255

Thr Asn Lys Val Glu Asn Ala Ala Met Val Leu Gly Arg Asn His Asp
            260                 265                 270

Leu Glu Asp Lys Val Asp Lys Leu Glu Ala Ser Leu Ala Glu Ala Asn
        275                 280                 285

Ile Ser Lys Phe Ser Cys Tyr Phe Val Asp Leu Leu Gln Glu Lys Ile
    290                 295                 300

Lys Ser Val Glu Glu Arg Phe Gln Val Cys Asn His Glu Met His Ser
305                 310                 315                 320

Gln Ile Glu Leu Tyr Glu Asn Ser Ile Ala Glu Phe His Asp Ile Leu
                325                 330                 335

Ser Lys Leu Val Glu Glu Thr Glu Lys Arg Ser Leu Glu His Ser Ala
            340                 345                 350

Ser Ser Met Pro Ser Glu Leu Trp Ser Arg Ile Ser Leu Leu Ile Asp
        355                 360                 365

Gly Trp Leu Leu Glu Lys Arg Ile Ser Tyr Asn Asp Ala Asn Thr Leu
    370                 375                 380

Arg Glu Met Val Arg Lys Arg Asp Ser Cys Leu Arg Glu Ala Tyr Leu
385                 390                 395                 400

Ser Cys Arg Gly Met Lys Asp Arg Glu Ile Val Asp Asn Phe Leu Lys
                405                 410                 415

Ile Thr Leu Pro Gly Thr Ser Ser Gly Leu His Ile Ile His Ile Ala
            420                 425                 430

Ala Glu Met Ala Pro Val Ala Lys Val Gly Gly Leu Ala Asp Val Ile
        435                 440                 445

Ser Gly Leu Gly Lys Ala Leu Gln Lys Lys Gly His Leu Val Glu Ile
    450                 455                 460

Ile Leu Pro Lys Tyr Asp Cys Met Gln Asn Asp Gln Val Asn Asn Leu
465                 470                 475                 480

Lys Val Leu Asp Val Val Val Gln Ser Tyr Phe Glu Gly Asn Leu Phe
                485                 490                 495

Asn Asn Lys Ile Trp Thr Gly Thr Val Glu Gly Leu Pro Val Tyr Phe
            500                 505                 510

Ile Glu Pro Gln His Pro Ala Lys Phe Phe Trp Arg Ala Gln Tyr Tyr
        515                 520                 525

Gly Glu His Asp Asp Phe Lys Arg Phe Ala Tyr Phe Ser Arg Ala Ala
    530                 535                 540

Leu Glu Leu Leu Tyr Gln Ser Gln Lys Lys Ile Asp Ile Ile His Cys
545                 550                 555                 560

His Asp Trp Gln Thr Ala Phe Val Ala Pro Leu Tyr Trp Glu Ala Tyr
                565                 570                 575

Ala Asn Leu Gly Phe Asn Ser Ala Arg Ile Cys Phe Thr Cys His Asn
            580                 585                 590

Phe Glu Tyr Gln Gly Ala Ala Pro Ala Gln Asp Leu Ala Cys Cys Gly
        595                 600                 605

Leu Asp Val Gln Gln Leu Asp Arg Glu Asp Arg Met Arg Asp Asn Ser
    610                 615                 620

His Gly Arg Ile Asn Val Val Lys Gly Ala Ile Val Tyr Ser Asn Ile
625                 630                 635                 640

Val Thr Thr Val Ser Pro Thr Tyr Ala Leu Glu Val Arg Ser Glu Gly
                645                 650                 655

Gly Arg Gly Leu Gln Asp Ser Leu Lys Leu His Ser Arg Lys Phe Val
            660                 665                 670

Gly Ile Leu Asn Gly Ile Asp Thr Asp Thr Trp Asn Pro Ser Thr Asp
        675                 680                 685

Arg His Leu Lys Val Gln Tyr Asn Ala Asn Asp Leu Gln Gly Lys Ala
    690                 695                 700

Ala Asn Lys Ala Ala Leu Arg Lys Gln Leu Asn Leu Ser Ser Thr Asn
705                 710                 715                 720

Ala Ser Gln Pro Leu Val Gly Cys Ile Thr Arg Leu Val Pro Gln Lys
                725                 730                 735

Gly Val His Leu Ile Arg His Ala Ile Tyr Lys Thr Ala Glu Leu Gly
            740                 745                 750

Gly Gln Phe Val Leu Leu Gly Ser Ser Pro Val Pro His Ile Gln Arg
        755                 760                 765

Glu Phe Glu Gly Ile Ala Asp His Phe Gln Asn Asn Asn Asn Ile Arg

770                 775                 780

Leu Leu Leu Lys Tyr Asp Asp Ser Leu Ser His Trp Ile Tyr Ala Ala
785                 790                 795                 800

Ser Asp Met Phe Ile Val Pro Ser Met Phe Glu Pro Cys Gly Leu Thr
                805                 810                 815

Gln Met Ile Ala Met Arg Tyr Gly Ser Val Pro Ile Val Arg Lys Thr
            820                 825                 830

Gly Gly Leu Asn Asp Ser Val Phe Asp Phe Asp Asp Glu Thr Ile Pro
        835                 840                 845

Lys Glu Leu Arg Asn Gly Phe Thr Phe Val His Pro Asp Glu Lys Ala
    850                 855                 860

Leu Ser Gly Ala Met Glu Arg Ala Phe Asn Tyr Tyr Asn Arg Lys Pro
865                 870                 875                 880

Glu Val Trp Lys Gln Leu Val Gln Lys Asp Met Arg Ile Asp Phe Ser
                885                 890                 895

Trp Ala Ser Ser Ala Ser Gln Tyr Glu Asp Ile Tyr Gln Arg Ala Val
            900                 905                 910

Ala Arg Ala Arg Ala Ala Ala
        915

<210>  10
<211>  914
<212>  PRT
<213>  Triticum aestivum

<400>  10

Met Ala Cys Ser Ala Ala Ala Gly Val Glu Ala Thr Ala Leu Leu Ser
1               5                   10                  15

Pro Arg Cys Pro Ala Pro Ser Pro Pro Asp Gly Arg Ser Arg Arg Arg
            20                  25                  30

Leu Ala Leu Ala Ser Gly Thr Arg His Arg Ser Leu Arg Ala Ala Ala
        35                  40                  45

Gln Arg Pro His Lys Ser Ala Thr Gly Ala Asp Pro Leu Tyr Asn Asn
    50                  55                  60

Arg Ala Asn Val Arg Ser Asp Glu Ala Ser Val Ser Ala Glu Lys Glu
65                  70                  75                  80

Arg Gln Arg Lys Tyr Asn Asp Gly Asp Gly Ile Ser Asn Leu Lys Leu
                85                  90                  95

Glu Asp Leu Val Gly Met Ile Gln Asn Thr Glu Lys Asn Ile Leu Leu
            100                 105                 110

Leu Asn Gln Ala Arg Leu Gln Ala Met Glu His Ala Asp Lys Val Leu
        115                 120                 125

Lys Glu Lys Glu Ala Leu Gln Arg Lys Ile Asn Ile Leu Glu Thr Arg
    130                 135                 140

Leu Ser Glu Thr Asp Glu Gln His Lys Leu Ser Ser Glu Gly Asn Phe
145                 150                 155                 160

Ser Asp Ser Pro Leu Ala Leu Glu Leu Gly Ile Leu Lys Glu Glu Asn
                165                 170                 175

Ile Leu Leu Lys Glu Asp Ile Glu Phe Phe Lys Thr Lys Leu Ile Glu
            180                 185                 190

Val Ala Glu Ile Glu Glu Gly Ile Phe Lys Leu Glu Lys Glu Arg Ala
        195                 200                 205

Leu Leu Asp Ala Ser Leu Arg Glu Leu Glu Ser Arg Phe Ile Ala Ala
    210                 215                 220

Gln Ala Asp Thr Met Lys Leu Gly Pro Arg Asp Ala Trp Trp Glu Lys
225                 230                 235                 240

Val Glu Lys Leu Glu Asp Leu Leu Glu Thr Thr Ala Asn Gln Val Glu
                245                 250                 255

His Ala Ala Val Ile Leu Asp His Asn His Asp Leu Gln Asp Arg Leu
            260                 265                 270

Asp Asn Leu Glu Ala Ser Leu Gln Ala Ala Asn Ile Ser Lys Phe Ser
        275                 280                 285

Cys Ser Leu Val Asp Leu Leu Gln Gln Lys Val Lys Leu Val Glu Asp
    290                 295                 300

Arg Phe Gln Ala Cys Asn Ser Glu Met His Ser Gln Ile Glu Leu Tyr
305                 310                 315                 320

Glu His Ser Ile Val Glu Phe His Asp Thr Leu Ser Lys Leu Ile Glu
                325                 330                 335

Glu Ser Glu Lys Arg Ser Leu Glu Asn Phe Thr Gly Asn Met Pro Ser
            340                 345                 350

Glu Leu Trp Ser Lys Ile Ser Leu Leu Ile Asp Gly Trp Leu Leu Glu
        355                 360                 365

Lys Lys Ile Ala Tyr Asn Asp Ala Ser Met Leu Arg Glu Met Val Arg
    370                 375                 380

Lys Arg Asp Ser Arg Leu Arg Glu Ala Tyr Leu Ser Tyr Arg Gly Thr
385                 390                 395                 400

Glu Asn Arg Asp Val Met Asp Ser Phe Leu Lys Met Ala Leu Pro Gly
                405                 410                 415

Thr Ser Ser Gly Leu His Ile Ala His Ile Ala Ala Glu Met Ala Pro
            420                 425                 430

Val Ala Lys Val Gly Gly Leu Ala Asp Val Ile Ser Gly Leu Gly Lys
        435                 440                 445

Ala Leu Gln Lys Lys Gly His Leu Val Glu Ile Ile Leu Pro Lys Tyr
    450                 455                 460

Asp Cys Met Gln Val Asp Gln Val Ser Asn Leu Lys Val Leu Asp Val
465                 470                 475                 480

Leu Val Gln Ser Tyr Phe Glu Gly Asn Met Phe Asn Asn Lys Ile Trp
                485                 490                 495

Thr Gly Thr Val Glu Gly Leu Pro Val Tyr Phe Ile Glu Pro Gln His
            500                 505                 510

Pro Ala Met Phe Phe Ser Arg Ala Gln Tyr Tyr Gly Glu His Asp Asp
        515                 520                 525

Phe Lys Arg Phe Ser Tyr Phe Ser Arg Ala Ala Leu Glu Leu Leu Tyr
    530                 535                 540

Gln Ser Gly Lys Lys Val Asp Ile Ile His Cys His Asp Trp Gln Thr

545                 550                 555                 560

Ala Phe Val Ala Pro Leu Tyr Trp Asp Val Tyr Ala Asn Leu Gly Phe
                565                 570                 575

Asn Ser Ala Arg Ile Cys Phe Thr Cys His Asn Phe Glu Tyr Gln Gly
            580                 585                 590

Thr Ala Pro Ala Arg Asp Leu Ala Trp Cys Gly Leu Asp Val Glu His
        595                 600                 605

Leu Asp Arg Pro Asp Arg Met Arg Asp Asn Ser His Gly Arg Ile Asn
    610                 615                 620

Ala Val Lys Gly Ala Ile Val Tyr Ser Asn Ile Val Thr Thr Val Ser
625                 630                 635                 640

Pro Thr Tyr Ala Leu Glu Val Arg Ser Glu Gly Gly Arg Gly Leu Gln
                645                 650                 655

Asp Thr Leu Lys Val His Ser Arg Lys Phe Leu Gly Ile Leu Asn Gly
            660                 665                 670

Ile Asp Thr Asp Thr Trp Asn Pro Ser Thr Asp Arg Tyr Leu Lys Val
        675                 680                 685

Gln Tyr Asn Ala Lys Asp Leu Gln Gly Lys Ala Ala Asn Lys Ala Ala
    690                 695                 700

Leu Arg Glu Gln Leu Asn Leu Ala Ser Ala Tyr Pro Ser Gln Pro Leu
705                 710                 715                 720

Val Gly Cys Ile Thr Arg Leu Val Ala Gln Lys Gly Val His Leu Ile
                725                 730                 735

Arg Arg Ala Ile Tyr Lys Thr Ala Glu Leu Gly Gly Gln Phe Val Leu
            740                 745                 750

Leu Gly Ser Ser Pro Val Pro Glu Ile Gln Arg Glu Phe Glu Gly Ile
        755                 760                 765

Ala Asp His Phe Gln Asn Asn Asn Asn Ile Arg Leu Ile Leu Lys Tyr
    770                 775                 780

Asp Asp Ala Leu Ser His Cys Ile Tyr Ala Ala Ser Asp Met Phe Val
785                 790                 795                 800

Val Pro Ser Ile Phe Glu Pro Cys Gly Leu Thr Gln Met Ile Ala Met
                805                 810                 815

Arg Tyr Gly Ser Val Pro Ile Val Arg Lys Thr Gly Gly Leu Asn Asp
            820                 825                 830

Ser Val Phe Asp Phe Asp Asp Glu Thr Ile Pro Met Glu Val Arg Asn
        835                 840                 845

Gly Phe Thr Phe Val Lys Ala Asp Glu Gln Gly Leu Ser Ser Ala Met
    850                 855                 860

Glu Arg Ala Phe Asn Cys Tyr Thr Arg Lys Pro Glu Val Trp Lys Gln
865                 870                 875                 880

Leu Val Gln Lys Asp Met Thr Ile Asp Phe Ser Trp Asp Thr Ser Ala
                885                 890                 895

Ser Gln Tyr Glu Asp Ile Tyr Gln Lys Ala Val Ala Arg Ala Arg Ala
            900                 905                 910

Val Ala

<210>  11
<211>  874
<212>  PRT
<213>  Vigna unguiculata

<400>  11

Met Ile Lys Asn Ala Glu Lys Asn Ile Leu Leu Leu Asn Gln Ala Arg
1               5                   10                  15

Val His Ala Leu Glu Asp Leu Glu Lys Ile Leu Ala Glu Lys Glu Ala
            20                  25                  30

Leu Gln Gly Glu Ile Asn Val Leu Ala Met Arg Leu Ala Glu Ser Asp
        35                  40                  45

Val Arg Ile Glu Val Ala Ala Gln Glu Lys Thr Arg Val Glu Leu Leu
    50                  55                  60

Glu Gly Glu Leu Glu Lys Leu Arg Ser Glu Leu Ala Gln Lys Gly Ser
65                  70                  75                  80

Ile Glu Gly Arg Asp Ala Glu Leu His Glu Leu Gln Asn Gly Val Phe
                85                  90                  95

Ser Asp Ala Ile Thr Asn Asn Leu Ser His Asn Asp Lys Ile His Ser
            100                 105                 110

Leu Thr Glu Glu Leu Asn Ser Ile Arg Glu Glu Asn Ala Thr Leu Lys
        115                 120                 125

Asn Ala Ile Glu Ser Phe Lys Ala Gln Leu Asn Asp Val Ala Asn Asn
    130                 135                 140

Asp Glu Arg Leu Ala Val Leu Glu Lys Glu Arg Leu Ser Leu Arg Ser
145                 150                 155                 160

Ala Leu Lys Asp Met Glu Ser Lys Leu Ser Ile Phe Pro Glu Asp Val
                165                 170                 175

Ser Glu Leu Ser Thr Leu Arg Val Glu Cys Lys Asp Leu Ser Asp Lys
            180                 185                 190

Val Glu Asn Leu Gln Leu Leu Leu Asp Lys Ala Thr Lys Gln Asp Ser
        195                 200                 205

Gln Ala Val Thr Val Leu Gln Gln Asn Gln Asp Leu Gln Arg Lys Val
    210                 215                 220

Asp Lys Leu Glu Ala Ser Leu Glu Glu Ala Asn Ile Tyr Lys Leu Ser
225                 230                 235                 240

Ser Asp Lys Leu Gln Lys Ser Asn Glu Leu Met Gln Gln Lys Ile Lys
                245                 250                 255

Leu Leu Glu Ser Gln Leu Gln Lys Ser Asp Glu Asp Ile Asn Ser Tyr
            260                 265                 270

Val Gln Leu Tyr Gln Gln Ser Val Lys Glu Phe Gln Asp Thr Leu Asp
        275                 280                 285

Leu Leu Lys Lys Glu Ser Lys Arg Arg Ala Pro Asp Glu Pro Val Glu
    290                 295                 300

Asp Met Pro Trp Glu Phe Trp Ser Arg Leu Leu Leu Leu Ile Asp Gly
305                 310                 315                 320

Trp Ala Leu Glu Lys Lys Ile Ser Val Asp Asp Ala Lys Leu Leu Arg

                325                 330                 335

Glu Lys Val Trp Lys Arg Asp Lys Ser Val Ser Asp Val Tyr Met Ala
            340                 345                 350

Tyr Lys Glu Lys Thr Glu His Glu Ala Ile Ser Ala Phe Leu Gly Leu
        355                 360                 365

Thr Ser Ser Ala Thr Ser Pro Gly Leu Tyr Val Ile His Ile Ala Ala
    370                 375                 380

Glu Met Ala Pro Val Ala Lys Val Gly Gly Leu Gly Asp Val Ile Ser
385                 390                 395                 400

Gly Leu Ser Lys Ala Leu Gln Lys Lys Gly His Leu Val Glu Ile Ile
                405                 410                 415

Leu Pro Lys Tyr Asp Cys Met Gln Tyr Asp Arg Ile Gly Asp Leu Arg
            420                 425                 430

Ala Leu Asp Val Val Ile Glu Ser Tyr Phe Asp Gly Gln Leu Phe Lys
        435                 440                 445

Asn Lys Ile Trp Val Gly Thr Val Glu Gly Leu Pro Val Tyr Phe Ile
    450                 455                 460

Glu Pro His His Pro Gly Lys Phe Phe Trp Arg Gly Asp Tyr Tyr Gly
465                 470                 475                 480

Ala His Asp Asp Phe Arg Arg Phe Ser Tyr Phe Ser Arg Ala Ala Leu
                485                 490                 495

Glu Phe Leu Leu Gln Ala Gly Lys Lys Pro Asp Ile Ile His Cys His
            500                 505                 510

Asp Trp Gln Thr Ala Phe Ile Ala Pro Leu Tyr Trp Asp Val Tyr Ala
        515                 520                 525

Pro Lys Gly Leu Asn Ser Ala Arg Ile Cys Phe Thr Cys His Asn Phe
    530                 535                 540

Glu Tyr Gln Gly Thr Ala Gly Ala Ser Glu Leu Glu Ala Cys Gly Leu
545                 550                 555                 560

Asp Ser His Gln Leu Asn Arg Pro Asp Arg Met Gln Asp Asn Ser Ala
                565                 570                 575

His Asn Arg Val Asn Ser Val Lys Gly Ala Val Val Tyr Ser Asn Ile
            580                 585                 590

Val Thr Thr Val Ser Pro Thr Tyr Ala Gln Glu Val Arg Thr Ala Glu
        595                 600                 605

Gly Gly Lys Gly Leu His Ser Thr Leu Ser Thr His Ser Lys Lys Phe
    610                 615                 620

Ile Gly Ile Leu Asn Gly Ile Asp Thr Asp Ile Trp Asn Pro Ala Thr
625                 630                 635                 640

Asp Pro Phe Leu Gln Val Gln Tyr Asn Ala Asn Asp Leu Gln Gly Lys
                645                 650                 655

Ser Glu Asn Lys Glu Ala Leu Arg Arg Asn Leu Gly Leu Ser Ser Ala
            660                 665                 670

Asp Val Arg Arg Pro Leu Val Gly Cys Ile Thr Arg Leu Val Pro Gln
        675                 680                 685

Lys Gly Val His Leu Ile Arg His Ala Ile Tyr Leu Thr Leu Glu Leu
    690                 695                 700

Gly Gly Gln Phe Val Leu Leu Gly Ser Ser Pro Val Pro His Ile Gln
705                 710                 715                 720

Arg Glu Phe Glu Gly Ile Ala Asn His Phe Gln Asn His Asp His Ile
                725                 730                 735

Arg Leu Ile Leu Lys Tyr Asp Glu Ser Leu Ser His Ala Ile Tyr Ala
            740                 745                 750

Ala Ser Asp Met Phe Ile Ile Pro Ser Ile Phe Glu Pro Cys Gly Leu
        755                 760                 765

Thr Gln Met Ile Ser Met Arg Tyr Gly Ala Ile Pro Ile Ala Arg Lys
    770                 775                 780

Thr Gly Gly Leu Asn Asp Ser Val Phe Asp Val Asp Asp Asp Thr Ile
785                 790                 795                 800

Pro Ser Gln Phe Arg Asn Gly Phe Thr Phe Leu Asn Ala Asp Glu Lys
                805                 810                 815

Gly Ile Asn Asp Ala Leu Val Arg Ala Ile Asn Leu Phe Thr Asn Asp
            820                 825                 830

Pro Lys Ser Trp Lys Gln Leu Val Gln Lys Asp Met Asn Ile Asp Phe
        835                 840                 845

Ser Trp Asp Ser Ser Ala Ala Gln Tyr Glu Glu Leu Tyr Ser Lys Ser
    850                 855                 860

Val Thr Arg Gly Arg Ala Thr Lys Arg Ala
865                 870

<210>  12
<211>  24
<212>  DNA
<213>  artificial sequence

<220>
<223>  primer

<400>  12
catatggaga ctgatgaaag gatt                                              24

<210>  13
<211>  24
<212>  DNA
<213>  artificial sequence

<220>
<223>  primer

<400>  13
ctcgagttct ttataaacgt tggc                                              24

<210>  14
<211>  25
<212>  DNA
<213>  artificial sequence

<220>
<223>  primer

<400>  14
tgatgagaag aggaatgacc cgaaa                                             25

<210>  15
<211>  21
<212>  DNA
<213>  artificial sequence

<220>
<223>  primer

<400>  15
ccatagattt tcgatagccg a                                                21

<210>  16
<211>  25
<212>  DNA
<213>  artificial sequence

<220>
<223>  primer

<400>  16
ggaaccattc cggtggtcca tgccg                                            25

<210>  17
<211>  28
<212>  DNA
<213>  artificial sequence

<220>
<223>  primer

<400>  17
ctcaccaatg atacttagca gcaacaag                                         28

<210>  18
<211>  22
<212>  DNA
<213>  artificial sequence

<220>
<223>  primer

<400>  18
gtgcaagacg gtgatggagc aa                                               22

<210>  19
<211>  25
<212>  DNA
<213>  artificial sequence

<220>
<223>  primer

<400>  19
cacgtttttt atattgcttt gggaa                                            25

<210>  20
<211>  20
<212>  DNA
<213>  artificial sequence

<220>
<223>  primer

<400>  20

cgtgacttaa gggctttgga                                                    20

<210>  21
<211>  20
<212>  DNA
<213>  artificial sequence

<220>
<223>  primer

<400>  21
gcagctcggc taaaatacga                                                    20

<210>  22
<211>  21
<212>  DNA
<213>  artificial sequence

<220>
<223>  primer

<400>  22
tggaaggaaa cgaaggcttt g                                                  21

<210>  23
<211>  21
<212>  DNA
<213>  artificial sequence

<220>
<223>  primer

<400>  23
tgtctttggc gtattcgtgg a                                                  21

<210>  24
<211>  22
<212>  DNA
<213>  artificial sequence

<220>
<223>  primer

<400>  24
acaggttttg gacgtggtga tt                                                 22

<210>  25
<211>  23
<212>  DNA
<213>  artificial sequence

<220>
<223>  primer

<400>  25
acaggacaag cctcaatgtt cca                                                23

<210>  26
<211>  31
<212>  DNA
<213>  artificial sequence

<220>
<223>  primer

<400>  26
gatctttgcc ggaaaacaat tggaggatgg t                                    31


<210>  27
<211>  31
<212>  DNA
<213>  artificial sequence

<220>
<223>  primer

<400>  27
cgacttgtca ttagaaagaa agagataaca g                                    31

39

39

39

## Description

### FIELD OF THE INVENTION

This invention relates to the field of genetically modified plants having improved features of industrial interest, including improved characteristics of starch synthesis.

### BACKGROUND OF THE INVENTION

Starch is an important constituent of the grains of various plants, including cereal grains, accounting in the latter for about 65-67% of the weight of the grain at maturity. It is generally admitted that the starch content accounts for from 45% to 85% of the dry weight for cereals, from 30% to 70% of the dry weight for legumes, and from 65% to 85% of the dry weight for tubers.

Plant starches are comprised of two classes of glucose homopolymers, respectively amylose and amylopectin. Amylose is a lightly branched linear molecule with a degree of polymerization of 1000 to 6000 Glucose units. Amylopectin, which has a much larger polymer unit (with a degree of polymerization of 10⁵-10⁶ Glucose units), contains frequent α-1,6 branch linkages (4-5%).

Starch is mainly produced in the amyloplast of the grain endosperm by the concerted action of a number of enzymes, including ADP-Glucose pyrophosphorylase (EC 2.7.7.27), starch synthases (EC 2.4.1.21), branching enzymes (EC 2.4.1.18) and debranching enzymes (EC 3.2.1.41 and EC 3.2.1.68).

Starch synthases (SSs) catalyze the transfer of the glucosyl moiety of ADP-glucose (the activated glucosyl donor) to a preexisting α-1,4 glucan primer. In a study, it was shown that, despite its tiny genome, *Ostreococcus tauri,* a prasinophyte alga, thought to have diverged at the earliest stage within the green linage, displays the same number and family types of starch synthases as those documented in the rice or Arabidopsis genomes (Ral et al., 2004, Plant Physiol. 136, 3333-3340.). This high degree of conservation of the pathway suggests that these enzymes play a conserved and specific function in the building of starch. Five distinct starch synthase families have been reported in plants. Four (SSI to SSIV) of these are predominantly found as soluble enzymes while the fifth enzyme is granule-bound (GBSSI). Mutants for all the starch synthases have been obtained and analyzed in distinct plant and algal systems with the noticeable exception of SSIV. Predicted SSIV proteins show a C-terminal region highly similar to other SSs, which comprise the catalytic and starch-binding domains (Cao et al., 1999, Plant Physiol. 120, 205-216.). On the contrary, the N-terminal half of SSIV protein differs significantly from other SS isoforms.

However, to date, genetic evidence for a role of SSIV in starch biosynthesis is still lacking

The picture emerging from the studies performed with all other starch synthases (SSI to III and GBSSI) in Chlamydomonas, pea, Arabidopsis and cereals is that each enzyme is responsible for the synthesis of specific size classes of glucans within the amylopectin structure (Fontaine et al., 1992, J. Biol.Chem. 268, 16223-16230; Craig et al., 1998, Plant Cell 10, 413-426; James et al., 2003, . Curr. Opin. Plant Biol. 6, 215-222; Morell et al., 2003, Plant J. 34, 173-185.; Delvallé et al., 2005, Plant J. 43, 398-412.). In addition, GBSSI seems to be the only elongation enzyme involved in the biosynthesis of amylose, the second poorly branched and dispensable polysaccharide fraction found within starch (Klösgen et al., 1986, Mol. Gen. Genet. 203, 237-244, Delrue et al., 1992, J. Bacteriol. 174, 3612-3620.). Despite these specialized functions, some starch synthases display some degree of functional overlap while others do not.

Because starch, and mainly plant starch, is of a wide industrial interest for various purposes in food industry and non-food industry, there is a need in the art for the provision of suitable starches, including through methods leading to improved production conditions.

There is thus a need in the art for producing starch with improved production conditions, notably with the view of providing to the public various final products at a lower cost.

### SUMMARY OF THE INVENTION

This invention firstly relates to plants that are rendered defective for Soluble Starch Synthase IV (SSIV), the said plants containing starch granules having an increased granule size, as compared to the same plant not being defective for Soluble Starch Synthase IV (SSIV) activity.

This invention also pertains to plant cells as well as to seeds that originate from such modified plants that are defective for Soluble Starch Synthase IV (SSIV).

In preferred embodiments, the plants according to the invention consists of transformed plants, which may also be termed genetically modified plants, that are rendered defective for Soluble Starch Synthase IV (SSIV) by genetic engineering, that is, in most embodiments, by introducing a DNA construct that will effect the inhibition or the blocking of the production of a biologically active SSIV protein.

The present invention also deals with methods for obtaining transformed plants defective for Soluble Starch Synthase IV (SSIV), the said methods comprising a step of introducing in one or more plant cells DNA constructs that render the said cell(s) defective for SSIV.

As used herein, plants that are rendered defective for Soluble Starch Synthase IV (SSIV) encompass plants that are also defective for one or more other proteins, especially enzymes, that are involved in starch synthesis, including plants that are also defective for starch phosphorylase.

The invention also concerns transformed plants that are obtained according to the methods above, as well as to plant cells and seeds originating therefrom.

This invention also relates to methods for producing starch comprising a step of extracting starch from a transformed plant defective for Soluble Starch Synthase IV (SSIV).

### DESCRIPTION OF THE FIGURES

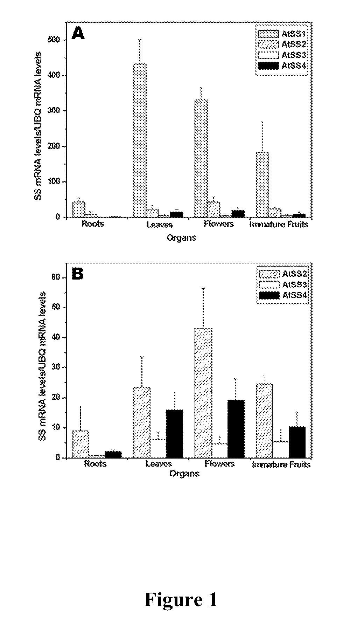

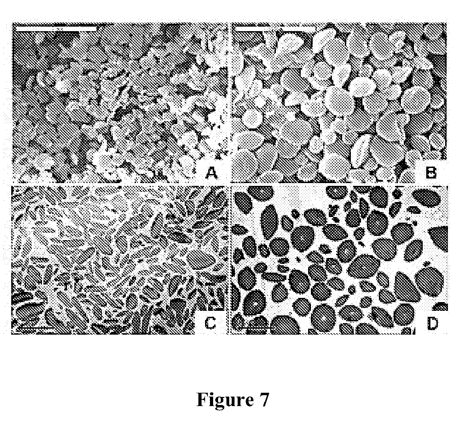

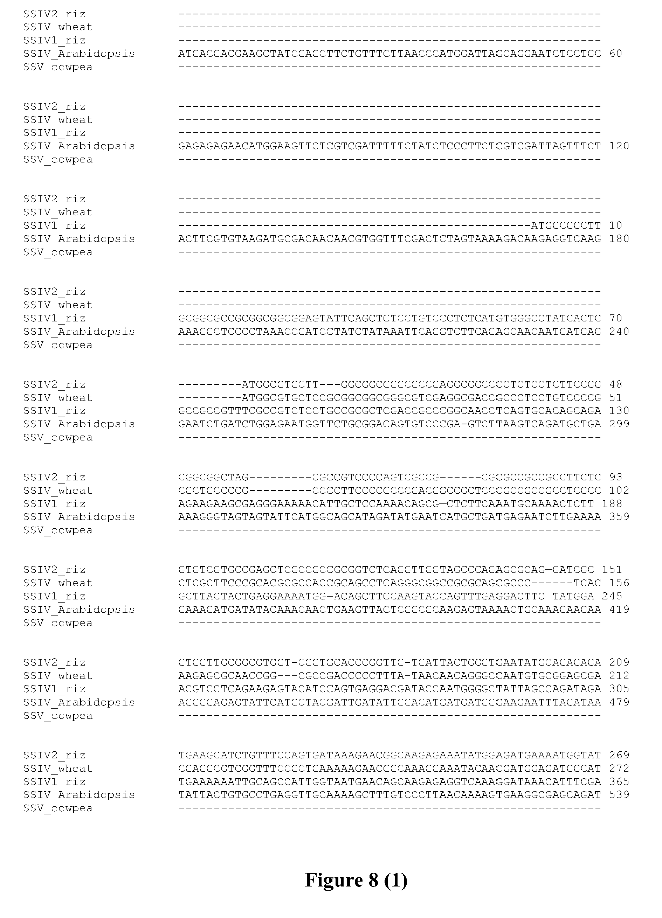

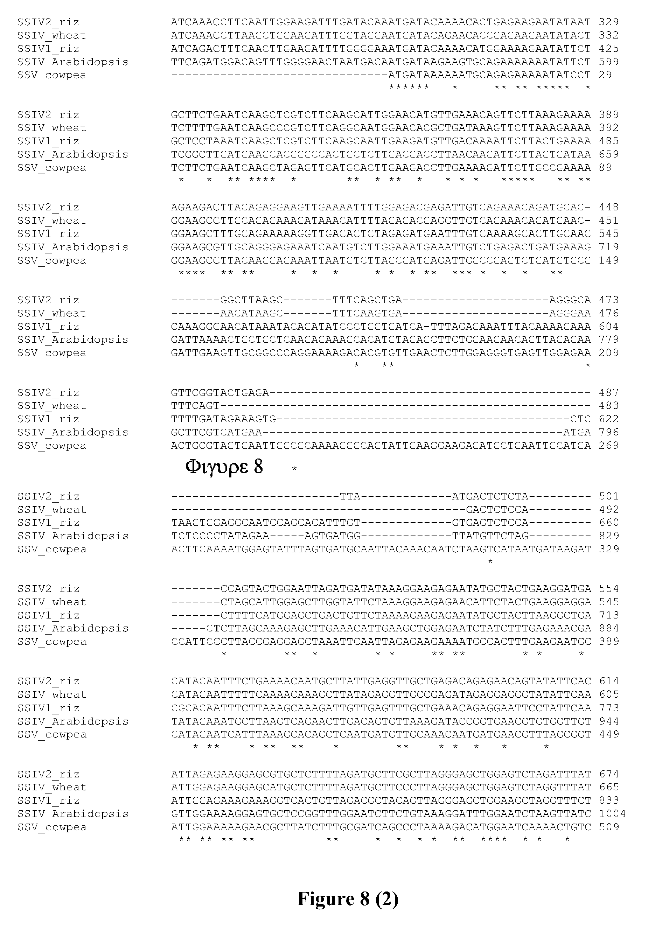

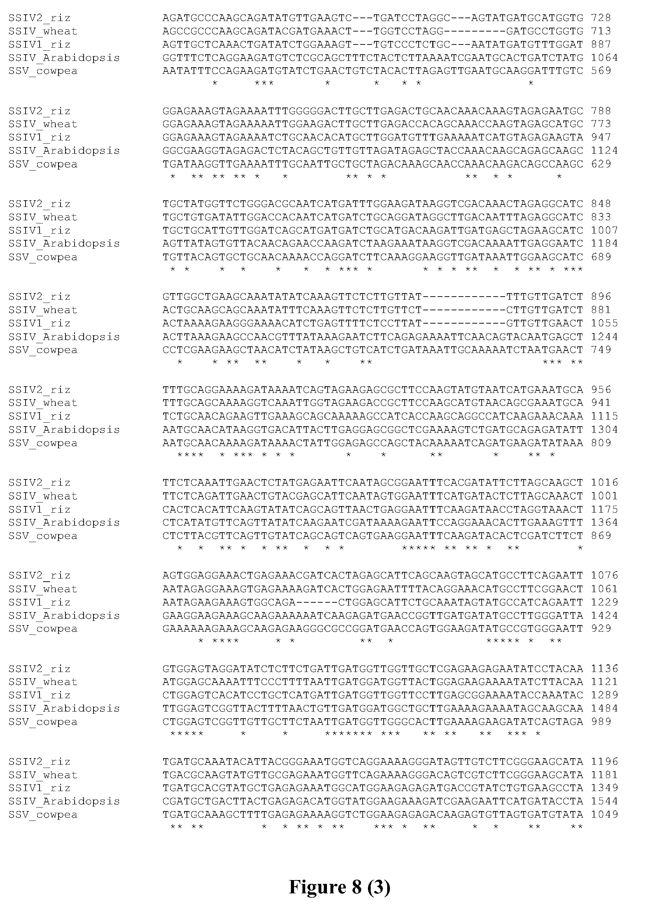

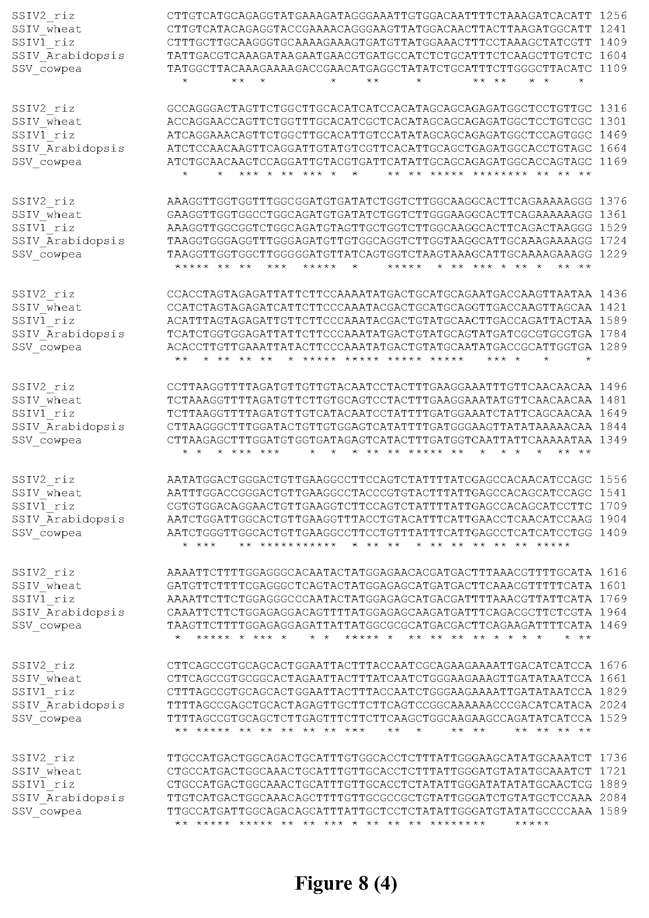

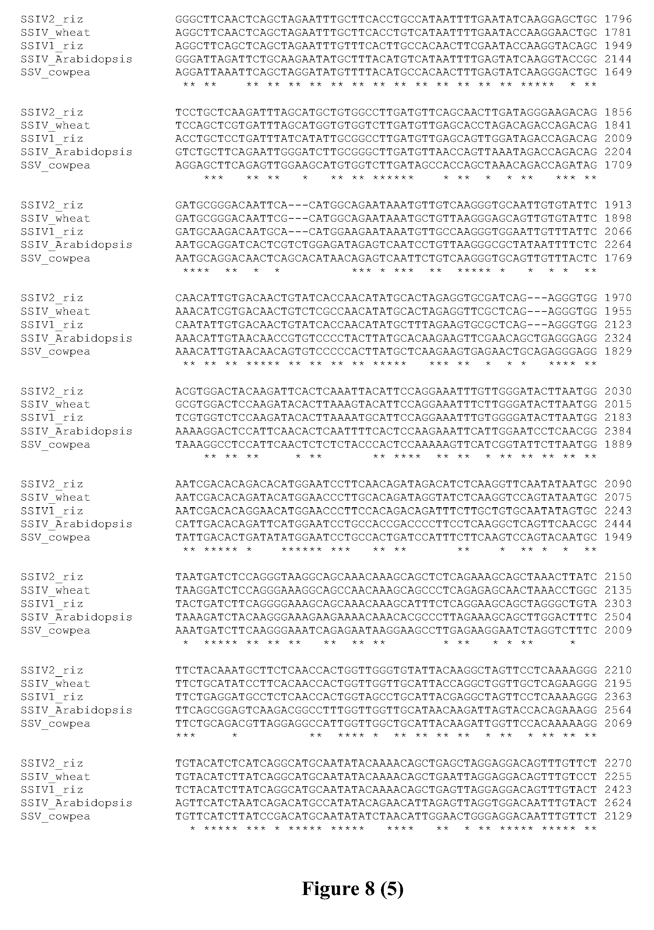

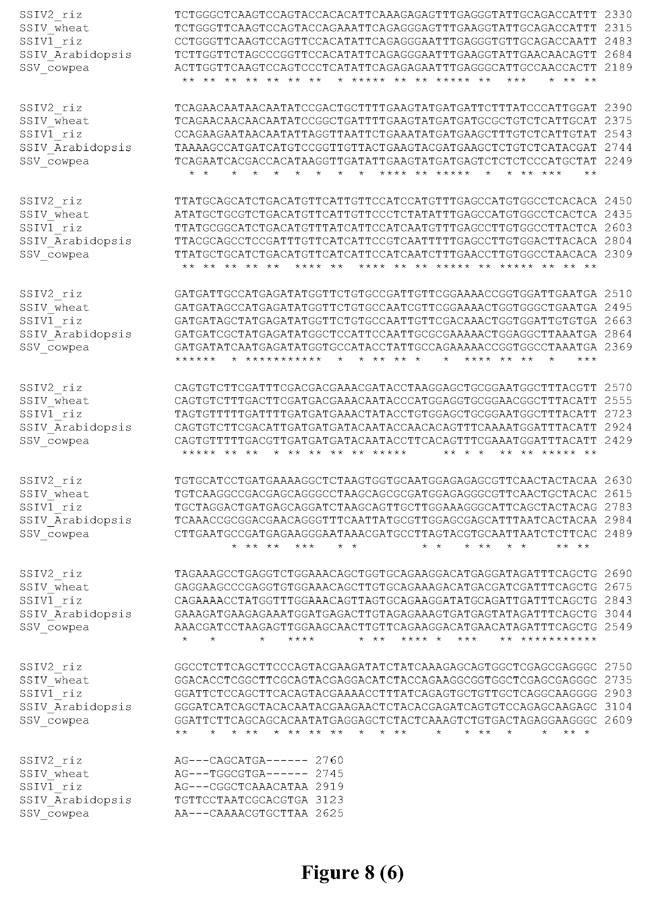

**Figure 1. Expression profile of *Arabidopsis* SS genes.**
   The absolute mRNA levels of all the *Arabidopsis* soluble starch synthase encoding genes (*AtSS1*: At5g24300; *AtSS2*: At3g01180; *AtSS3*: At1g11720; *AtSS4*: At4g18240) were determined by real-time quantitative RT-PCR as described in Experimental Procedures. Data shown in Fig. 1-B are the same than in Fig. 1-A but *AtSS1* gene expression was omitted to make a clearer comparison between the other *AtSS* genes. Values are the average of three determinations of at least two cDNA preparations from different experiments.
**Figure 2. Analysis of mutant lines in locus *AtSS4.***
   Fig. 2-A) : Genomic structure of *AtSS4* locus. Exons and introns are indicated as thick and thin black bars respectively. Insertion sites of T-DNA in mutant lines *Atss4-1* and *Atss4-2* at introns 11 and 2 respectively are indicated by triangles.
   Fig. 2-B) : Western blot analysis of leaves crude extracts of *Atss4-1* and *Atss4-2* mutant alleles and their respective wild type ecotypes. Proteins (25 µg) were separated by SDS-PAGE electrophoresis, transferred to nitrocellulose filters and immunolabelled with rabbit antiserum raised against a 178 amino acids fragment of the N-terminal region of SSIV protein (see Experimental Procedures). Molecular markers (kDa) positions are indicated. Bands of approximately 112 kDa matching the predicted SSIV mass in Col-O and WS wild type ecotypes are indicated by an arrow.
**Figure 3. Growth of *Atss4* mutant alleles and their respective wild** type ecotypes.
   Seeds of mutant and wild type plants were incubated in water at 4°C for 3 days before sowing in soil. Plants were cultured in growth cabinet under a photoregime of 16 h light/8 h dark.
   Fig. 3-A and Fig. 3-B: determination of above ground organs fresh weight (FW) in mg per plant (Y-axis) during time course experiment (X-axis in Days after germination of seeds). Vertical bars represent the SE of mean values determined on three independent samples cultivated at the same time. Black arrows are wild type plants (Col-O and WS in Fig. 3-A and
   Fig. 3-B respectively). Squares are mutant plants (*Atss4-1* and *Atss4-2* in Fig. 3-A and Fig. 3-B respectively).
**Figure 4. Starch accumulation in leaves of *Atss4-1* and Col-O plants during a day/night cycle.**
   Plants were cultured under a 16h light/8 h dark photoperiod during 21 days and then one leaf from three plants for each line were collected at indicated time. Starch content in leaves was determined by enzymatic assay as described in Experimental Procedure. Values are the average of three independent experiments. Vertical bars are Standard Error calculated from 3 independent samples.
**Figure 5.**

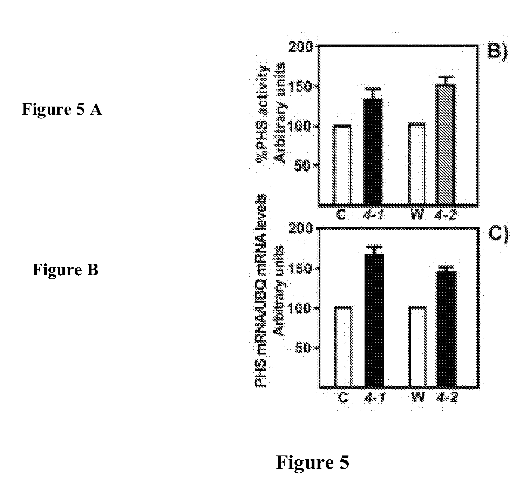

   Fig. 5-A : *In vitro* starch phosphorylase activities. Enzymatic assays were performed in leaves crude extracts using amylopectin as substrate as described in Experimental Procedures. Activities in mutant lines are expressed as percentage of values obtained for their respective wild type ecotypes, which are considered to be 100%. Values are the average of three different experiments (Vertical bars = SE).
   Fig. 5-B : Expression of *AtPHS1* (At3g29320) and *AtPHS2* (At3g46970) genes in leaves of *Atss4-1* and Col-O plants. Levels of *PHS1* and *PHS2* mRNAs were determined using real-time quantitative RT-PCR as described in Experimental Procedure. Data are normalized to values obtained for Col-O plants, which are considered to be 100. Black columns: mRNA levels *in Atss4-1* plants. White columns: mRNA levels in Col-O plants (Vertical bars = SE).
**Figure 6.**
   Amylopectin Chain Length (CL) distribution profiles for mutant alleles (*Atss4-1* and *Atss4-2*) and their respective wild type ecotypes (Col-O and WS). Amylopectin was purified using CL-2B column and subsequently debranched with a mix of isoamylase and pullulanase. The resulting linear glucans were analyzed by FACE after coupling with a fluorescent molecule (APTS) to their non-reducing ends. The relative proportion for each glucan in the total population is expressed as a percentage of the total number of chains. X-axes represent the degree of polymerization (DP) of the chains. Y-axes represent Molar %. In the two bottom diagrams, the normalized value for each wild type was subtracted from that of their respective mutant allele and in that case Yaxes represent Molar % difference. Values are the average of three different experiments. The standard deviation was less than □15% of the average values.
**Figure 7.**
   Scanning (Fig. 7-A and Fig. 7-B) and Transmission (Fig. 7-C and Fig. 7-D) electron microscopy analysis of starch from mutant and wild type plants. Starch granules were isolated by Percoll gradient as described in Experimental Procedures from leaves collected after 8 h of illumination (midday). Fig. 7-A) Col-O. Fig. 7-B) *Atss4-1.* Fig. 7-C) WS. Fig. 7-D) *Atss4-2.*
**Figure 8**
   Alignment of the nucleic acid sequences encoding the following SSIV proteins :
   - line 1 (upper line) : SSIV-2 from *Oryza sativa*;
   - line 2 : SSIV from *Triticum aestivum*;
   - line 3 : SSIV-1 from *Oryza sativa*;
   - line 4 : SSIV from *Arabidopsis thaliana*;
   - line 5 (bottom line) : SSIV from *Vigna unguiculata.*

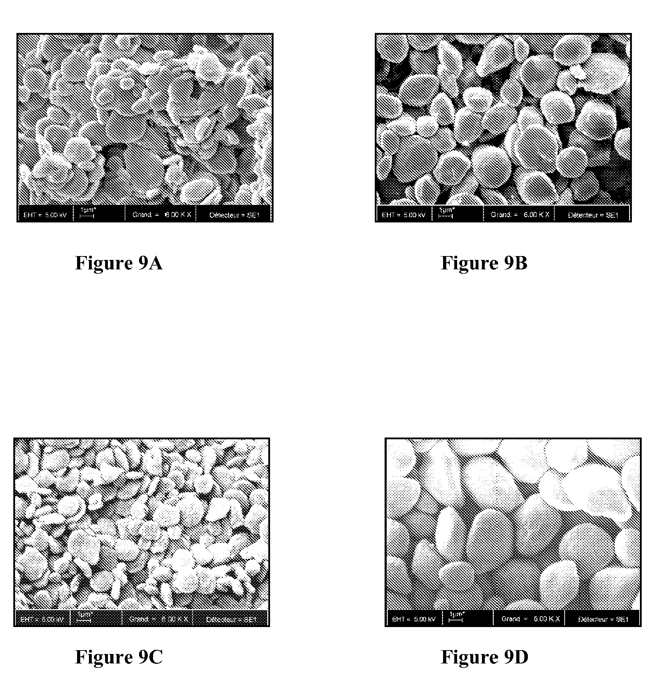

**Figure 9 : Microphotographs of starch grains originating from Wild-type and mutated *Arabidopsis thaliana* plants.**
   Figure 9 illustrates microphotographs from scanning electronic microscopy, at the same enlargement scale, of starch grains originating from, respectively :
   - Figure 9-A : Wild-type *Arabidopsis thaliana*;
   - Figure 9-B : Modified *Arabidopsis thaliana* that has been rendered defective for SSIV;
   - Figure 9-C : Modified *Arabidopsis thaliana* that has been rendered defective for starch phosphorylase, and
   - Figure 9-D : Modified *Arabidopsis thaliana* that has been rendered defective for both SSIV and starch phosphorylase (double mutant).

### DETAILED DESCRIPTION OF THE INVENTION

Surprisingly; it has been found according to the invention that plants that have been rendered defective for Soluble Starch Synthase IV (SSIV) produce less starch granules in their cells than the same plants that are not defective for SSIV, but that these starch granules at a lower number are higher in size, and thus contain a greater amount of starch, as compared to the starch granules produced by the same plants that are not defective for SSIV.

It has also been found that this increase in the starch granules size that is induced in SSIV-defective plants may be further amplified in plants that have been rendered defective for both SSIV and starch phosphorylase.

It indeed may be taken benefit from these features of the plants rendered defective for Soluble Starch Synthase IV (SSIV), and optionally also for starch phosphorylase, for performing improved methods of extracting starch, notably methods allowing better production yields. As it will be described further in the present specification, the size of the starch granules consists of a key parameter in view of performing optimal qualitative and/or quantitative industrial starch extraction processes.

More precisely, it has been found according to the invention that plants that have been rendered defective for Soluble Starch Synthase IV (SSIV) by mutation of the SSIV-encoding gene produce starch granules of a far higher size than the starch granules that are produced by the same but non-defective plants, despite a modest but significant decrease of the overall starch production. Notably, it has been found that plants that have been rendered defective for Soluble Starch Synthase IV (SSIV) produce starch granules having a size of at least 1.5 times higher than starch granules originating from the same but non SSIV-defective plants.

Further, the structure of the starch granules is unaffected in such plants that have been rendered defective for SSIV.

Still further, it has been found that plants that have been rendered defective for both (i) Soluble Starch Synthase IV (SSIV) and (ii) starch phosphorylase produce starch granules having a size of at least 4 times higher than starch granules originating from the same but non SSIV-defective and non starch phosphorylase-defective plants.

Yet further, it has been found that plants that have been rendered defective for both (i) Soluble Starch Synthase IV (SSIV) and (ii) starch phosphorylase possess a starch content which is far higher than the same but non SSIV-defective and non starch phosphorylase-defective plants. In these specific SSIV-defective plants, the starch content is at least 1.5 times higher than in the same but wild-type plants, and even at least 2, 2.5, 3 or 3.5 times higher than in the same but wild-type plants. In *Arabidopsis thaliana* plants that have been rendered defective for both (i) Soluble Starch Synthase IV (SSIV) and (ii) starch phosphorylase, a starch content of almost 4 times the starch content that is found in the same but wild-type plants has been determined, as it is shown in the examples herein.

It should be understood that, throughout the present specification, plants that have been rendered defective for SSIV encompass both (1) plants that have been rendered defective exclusively for SSIV and (2) plants that have been rendered defective for both (i) SSIV and (ii) starch phosphorylase.

It has also been surprisingly found that plants that have been rendered defective for SSIV produce starch molecules, the chemical composition of which is at least highly similar to, if not identical to, the starch molecules which are produced by the same but non-defective plants. Notably, the same amylose/amylopectin ratio is found in both SSIV-defective and SSIV-non-defective plants. Also, plants that are rendered defective for SSIV produce starch having a Chain Length (CL) distribution profile that is highly similar to the same but non-defective plants, with only eventual minor changes on the structure of amylopectin, as illustrated by an eventual slight reduction on the amount of chains of DP=7-10.

Thus, it has been shown for the first time according to the invention that the structurally well known plant Soluble Starch Synthase IV (SSIV) possess a specific function in the control of the starch granule number.

It has further been shown herein that an alteration of the plant cell production of a biologically active SSIV does not detectably affect the synthesis of the other enzymes involved in the production of starch, including the synthesis of the other SSI, SSII, SSIII and GBSSI starch synthases. Sole an increase of the synthesis of starch phosphorylase may be induced in plants rendered defective for SSIV.

An object of the present invention consists of a plant which is rendered defective for Soluble Starch Synthase IV (SSIV).

The said SSIV-defective plant comprises plant cells containing starch granules having an increased granule size, as compared to the same plant that are not defective for production of biologically active Soluble Starch Synthase IV (SSIV).

As intended herein, a plant, a plant part or a plant cell that is "defective" for Soluble Starch Synthase IV (SSIV) does not synthesize or produce SSIV in an amount sufficient for metabolizing starch like in wild type plant cells that synthesize or produce normal amounts of biologically active SSIV. Notably, pants, plant parts and plant cells that are "defective" for SSIV have an altered starch synthesis pathway that leads to the production of a mean number of starch granules per cell that is significantly lower than the number of starch granules per cell that is found in the same plants that are not defective for SSIV. Illustratively, *Arabidopsis thaliana* plants defective for SSIV produce 1, or exceptionally 2, starch granules per chloroplast, whereas the same *Arabidopsis thaliana* plants that are not defective for SSIV produce about 4-5 starch granules per chloroplast, as it is shown in the examples herein.

As used herein, the "size" of a starch granule encompasses the absolute dimensional value of the said starch granule, as well as the relative value of the mean size of two populations of starch granules that are compared. In certain embodiments, the "size" of a starch granule according to the absolute dimensional value may be performed by image analysis and laser diffraction method, for example by using the technique disclosed by Wilson et al. (2005, Cereal chemistry, Vol. 83 : 259-268). In certain other embodiments, the "size" of a starch granule may determined according to a method of Gravitational Field-Flow Fractionation (GFFF), such as disclosed by Reschiglian et al. (2002, Ann Chim, Vol. 92(4) : 457-467). In certain further embodiments, simpler methods for starch granule size determination are used. These simpler methods for starch granule size determination encompass (i) performing at least one microphotograph of a population of starch granules to be sized, (ii) measuring the largest diameter of at least 10, advantageously at least 30, more preferably at least 50, and most preferably at least 100 starch granules to be sized and (iii) calculating the mean largest diameter of the starch granules of the studied population of starch granules, the said mean largest diameter value consisting of the "size" of the said starch granules, that may thus be compared to the mean largest diameter (""size") of another population of starch granules. These simpler methods for starch granule size determination also encompass (i) performing at least one microphotograph of a population of starch granules to be sized, (ii) measuring the visible surface area of at least 10, advantageously at least 30, more preferably at least 50, and most preferably at least 100 starch granules to be sized and (iii) calculating the mean largest visible surface area of the starch granules of the studied population of starch granules, the said mean visible surface area value consisting of the "size" of the said starch granules, that may thus be compared to the mean visible surface area ("size") of another population of starch granules.

Generally, in the present specification, a size difference is qualified, such as a size increase or a size decrease, following the comparison of two distinct populations of starch granules. Using methods for determining the "size" of starch granules by first performing microphotographs of populations of starch granules, an increase or a decrease of at least 1.5 times of the absolute size values, between the two starch granules populations that are compared, is accurately measurable, so as to qualify the measured size difference as a statistically significant increase or decrease of the starch granule size.

A plant defective for SSIV according to the invention possesses a starch granule size that is at least 1.5 times greater than the size of the starch granules found in the same but non SSIV-defective plant, it being understood that the two population of starch granules that are compared, respectively (i) the SSIV-defective plant and (ii) the non SSIV-defective plant, preferably originate from the same plant part or from the same plant tissue, e.g. stems, leaves etc. In most embodiments, a plant defective for SSIV according to the invention possesses a starch granule size that is at least twice greater than the size of the starch granules found in the same but non SSIV-defective plant,

Among the SSIV-defective plants according to the invention, a plant defective for both (i) SSIV and (ii) starch phosphorylase possesses a starch granule size that is at least 3 times greater than the size of the starch granules found in the same but (i) non SSIV-defective and (ii) non starch phosphorylase-defective plant, it being understood that the two population of starch granules that are compared, respectively (i) the defective plant and (ii) the non defective plant, preferably originate from the same plant part or from the same plant tissue, e.g. stems, leaves etc. In most embodiments, a plant defective for both (i) SSIV and (ii) starch phosphorylase according to the invention possesses a starch granule size that is at least four times greater than the size of the starch granules found in the same but non defective plant,

In a plant, a plant part or a plant cell that is defective for SSIV, the defect consisting of a lack of a sufficient amount biologically active SSIV for metabolizing starch, in contrast to the same plant, plat part or plant cell that is not defective for SSIV may be artificially induced by using various methods that are well known form the one skilled in the art, since numerous SSIV nucleic acid sequences and/or SSIV amino acid sequences are already known in the art. Methods for obtaining plants that are rendered defective for SSIV include the following techniques :
(i) techniques causing an alteration of the genomic nucleic acids encoding SSIV, so that (i) no or a very low amount of the corresponding mRNA is produced or (ii) an altered mRNA is produced that encodes for a non biologically active SSIV;
(ii) techniques causing an inhibition or a blocking of the synthesis of the SSIV-encoding mRNAs;
(iii) techniques causing an inhibition or a blocking of the production of an SSIV protein from the SSIV-encoding mRNA; and
(iv) techniques causing an inhibition or a blocking of the biological activity of a produced SSIV protein.

Techniques (i) to (iii) above are preferred. Techniques (i) to (iii) above involve a step of genetic modification of the plant, plant parts or plant cells, which step of genetic modification generally consists of introducing in one or more plant cells, eventually in one or more plant parts or in one or more plants, at least a DNA construct comprising a DNA sequence selected from the group consisting of (i) DNA sequences causing an alteration of the genomic nucleic acid encoding a SSIV protein, e.g. a T-DNA originating from *Agrobacterium tumefaciens*, (ii) DNA sequences causing an inhibition or a blocking of the synthesis of the SSIV-encoding mRNAs, e.g. a SSIV-specific sense nucleic acid, (iii) DNA sequences causing an inhibition or a blocking of the production of an SSIV protein from the SSIV-encoding mRNA, e.g. a SSIV-specific antisense nucleic acid or a SSIV-specific ribozyme.

Assays for determining if a plant, a plant part or a plant cell is defective for SSIV may be conducted according to various techniques well known from the one skilled in the art.

In certain embodiments, techniques for determining a SSIV defective phenotype consist of assays for detecting the presence of SSIV biological activity in the plant, plant part or plant cell to be tested. Illustratively, a protein extract may be prepared from the corresponding plant sample prior to being brought into contact with an immunoaffinity chromatography substrate having anti-SSIV antibodies immobilized thereon. Production of anti-SSIV antibodies is disclosed in the examples herein. Then, the SSIV protein molecules that are eventually bound to the chromatography substrate are eluted and the resulting elution sample is assayed for the presence of starch synthase biological activity, optionally after *in vitro* addition of one or more exogenous other starch synthases among SSI, SSII, SSIIII and GBSSI, for example according to the technique disclosed by Delvallé et al. (2005, Plant J. 43, 398-412). Using these techniques, a SSIV defective phenotype is determined for the tested plant, plant part or plant cell exhibiting a SSIV biological activity of 50% or less than in the same wild type plant that is not defective for SSIV.

In certain other embodiments, techniques for determining a SSIV defective phenotype consist of assays for detecting the presence of SSIV protein molecules in the plant, plant part or plant cell to be tested. Illustratively, a protein extract is prepared from the corresponding plant sample prior to be subjected to a gel electrophoresis, so as to separate in defined protein bands the various proteins initially contained in the said protein extract. Then, after staining, a determination for the presence of a protein band at the expected location of SSIV is performed. Eventually, starch synthase activity may be assayed on the separated protein bands, according to well known techniques. Further illustratively, a protein extract is prepared from the corresponding plant sample prior to being subjected to an immunoblot analysis using anti-SSIV antibodies, for example anti-SSIV antisera. Such SSIV assays using gel electrophoresis or immunoblotting are for example disclosed by Dian et al. (2005, J Exp Botany, 56(n°412) : 623-632), as well as in the examples herein. Using these techniques, a SSIV defective phenotype is determined for the tested plant, plant part or plant cell exhibiting an amount of SSIV protein of 50% or less than in the same wild type plant that is not defective for SSIV.

In certain further embodiments, techniques for determining a SSIV defective phenotype consist of assays for detecting and/or quantifying SSIV-encoding mRNAs in the plant, plant part or plant cell to be tested. Illustratively, total RNA is extracted from the corresponding plant sample prior to perform PCR amplification using one or more suitable primer sets and then separating the resulting PCR products before detection and/or quantification, for example by staining. Such a PCR SSIV assay is disclosed for example by Dian et al. (2005, J Exp Botany, 56(n°412) : 623-632), as well as in the examples herein. Illustratively, primer sets that may be used for PCR amplification of SSIV-specific *A*. *thaliana* mRNAs include SEQ ID N°12-13 and SEQ ID N° 20-21 that are disclosed herein. Using these techniques, a SSIV defective phenotype is determined for the tested plant, plant part or plant cell exhibiting an amount of SSIV mRNA of 50% or less than in the same wild type plant that is not defective for SSIV.

In still further embodiments, techniques for determining a SSIV defective phenotype consist of assays for detecting alterations in the SSIV-encoding gene comprised in the chromosomal DNA contained in the plant, plant part or plant cell to be tested. Illustratively, total DNA from the corresponding plant sample is extracted prior to generate PCR amplification products using suitable primer sets and, after separation of the said PCR amplification products, characterize eventual genomic alterations in the SSIV-encoding gene, for example by single gel electrophoresis of the PCR amplification products or by sequencing the same. Also, alterations in the SSIV-encoding gene may be detected by detection of alterations in the corresponding mRNAs, using techniques well known from the one skilled in the art, including those disclosed in the examples herein. Using these techniques, a SSIV defective phenotype is determined for the tested plant, plant part or plant cell if genomic alterations include mutations in the promoter sequence, or in the intron(s) or exon(s) of the coding region, which include mutations in the regions of junction between exons and introns, as well as mutations in exons leading to an alteration of the amino acid sequence of the SSIV protein.

Techniques for assaying for starch phosphorylase activity are well known from the one skilled in the art. Such techniques are described for example in the US Patent n° US 5,998,701 or in the PCT Application n° WO 2005/097999, that herein incorporated in their entirety by reference.

As already mentioned above, the metabolizing pathway of plant starch is highly conserved throughout plant phylogeny, since the same system of starch synthesis is found from prasinophyte algae to the highest multicellular plant organisms, including cereals like wheat or rice. This high degree of conservation suggests that the enzymes involved in starch synthesis like the starch synthases, including SSIV, play a conserved and specific function in the building of starch. This is also true for starch phosphorylases, that may also be termed alpha-1,4 glucan phosphorylases, that are internationally classified as EC 2.4.1.1. enzymes, and that are generally well known by the one skilled in the art. Illustratively, well known starch phosphorylases encompass those originating from *Arabidopsis thaliana* (PCT Application n° WO 2005/097999), broad bean (Swissprot database, Access N° P53536), potato (Swissprot database, Access N° P04045 or N° P53535), beet, spinach, maize (PCT Application n° WO 98/40503), green pea, rice (EMBL database, Access n° D23280 or Q9AUV8) as well as from wheat (EMBL database, Access n° AAQ73181). Indeed, other starch phosphorylases are full available to the one skilled in the art, for example by using computer programs for amino acid sequence or nucleic acid sequence retrieving, such as the Blast program or the FastDB programs, using the default parameters, for example by using any one of the starch phosphorylase sequence cited above as the reference sequence. Other starch phosphorylases are also available to the one skilled in the art through hybridization assays, using any one of the starch phosphorylase nucleic acid cited above as the reference hybridization material, and performing a high stringency hybridization assay according to the method disclosed by Sambrook et al. (Molecular Cloning. A Laboratory Manual; Cold Spring Harbor Press, 1989, especially paragraphs 11.1 to 11.61).

Thus, without wishing to be bound by any particular theory, the applicant believes that a defect in SSIV activity will induce in all plants the same phenotype of a higher starch granule size that is found in *Arabidopsis thaliana.* This belief is highly reinforced by the fact that most, if not all, known SSIV protein amino acid sequences originating from a wide variety of plants share a strong amino acid identity. Illustratively, the amino acid sequence of the SSIV protein from *Arabidopsis thaliana* possesses 68% identity with SSIV from *Vigna unguiculata* (cowpea), 56% identity with SSIV from *Triticum aestivum* (wheat), 54% identity with SSIV-1 from *Oryza sativa* (rice) and 56% identity with SSIV-2 from *Oryza sativa* (rice). Still illustratively, at the cDNA level, the nucleic acid sequence encoding the SSIV protein from *Arabidopsis thaliana* possesses 73% identity with SSIV from *Vigna unguiculata* on a nucleic acid length of 2323 nucleotides, 67% identity with SSIV from *Triticum aestivum* on a nucleotide length of 2051 nucleotides, and 67% identity with SSIV-1 and SSIV-2 from *Oryza sativa* on a nucleotide length of 1865 nucleotides and 2053 nucleotides, respectively. Importantly, the highest amino acid identity, as well as the highest nucleic acid identity, between all SSIV is found in two protein regions located respectively at the N-terminal portion and at the C-terminal portion of all SSIV proteins. The most SSIV-specific protein region of high identity between all SSIV proteins is located at the N-terminal portion of the said proteins, in a protein region encompassing the amino acid residue at position 200 to the amino acid residue located at position 420 of each SSIV protein. Thus, in view of rendering any plant defective for SSIV, the one skilled in the art will use anyone of the techniques disclosed in the present specification, including techniques requiring DNA constructs comprising DNA sequences specifically targeted against SSIV-encoding nucleic acids, encompassing nucleic acid sequences targeted against SSIV-specific DNA or RNA regions encoding the said common N-terminal region of SSIV proteins.

It is shown in the examples herein that a plant may be rendered defective for SSIV by mutation of the SSIV-encoding gene, and more precisely by insertion of an heterologous nucleic acid within the sequence of the SSIV-encoding gene and that the resulting transformed or recombinant plants defective for SSIV produce less starch granules per cell, but of a greater size, than the corresponding wild type plant. These transformed or recombinant plants defective for SSIV are of a high industrial interest, mainly because these transformed or recombinant plants allow a far easier and less expensive industrial starch extraction, as compared with the same plants that are not defective for SSIV.

It is also shown in the examples herein that a plant may be rendered defective for both (i) SSIV and (ii) starch phosphorylase by mutation of both the SSIV-encoding gene and tha starch phosphorylase-encoding gene. More precisely, it is shown herein that these mutations may be generated by the insertion of an heterologous nucleic acid within (i) the sequence of the SSIV-encoding gene and (ii) the sequence of the starch phosphorylase-encoding gene, and that the resulting transformed or recombinant plants defective for SSIV produce less starch granules per cell, but of a greater size than both (i) the corresponding SSIV-defective / starch phosphorylase-non defective plant and (ii) SSIV-non defective./ starch phosphorylase-non defective plant These transformed or recombinant plants defective for both SSIV and starch phosphorylase are of a high industrial interest, mainly because these transformed or recombinant plants allow a far easier and less expensive industrial starch extraction, as compared with the same plants that are not defective for SSIV and starch phosphorylase.

According to certain preferred embodiments of a plant that is defective for SSIV, SSIV activity is totally absent or blocked, or alternatively only residual SSIV activity is present. In these preferred embodiments, SSIV residual activity does not exceed 20% the SSIV activity found in the same plant that is not defective for SSIV. Most preferably, SSIV residual activity does not exceed 19.5%, 19%, 18.5%, 18%, 17.5%, 17%, 16.5%, 16%, 15.5%, 15%, 14.5%, 14%, 13.5%, 13%, 12.5%, 12%, 11.5%, 11%, 10.5%, 10%, 9.5%, 9%, 8.5%, 8%, 7.5%, 7%, 6.5%, 6%, 5.5%, 5%, 4.5%, 4%, 3.5%, 3%, 2.5%, 2%, 1.5%, 1%, 0.5% or even 0.1% the SSIV activity found in the same plant that is not defective for SSIV.

In the preferred embodiments illustrated in the examples herein, SSIV activity is totally absent since all the exemplified transformed or recombinant plants are homozygous for a mutation within the SSIV-encoding gene that cause the synthesis of an altered messenger RNA.

Thus, in certain embodiments of a plant that is rendered defective for SSIV according to the invention, the said plant is genetically modified so as to produce a mutated inactive SSIV. Techniques that are well known in the art for genetically modifying plants and which are herein adapted so as to cause production of a mutated inactive SSIV protein will be described in detail further in the present specification.

In certain other embodiments of a plant that is rendered defective for SSIV according to the invention, the said plant is genetically modified so as to exhibit a reduced synthesis or no synthesis of SSIV. As intended herein, a reduced SSIV synthesis means that an amount of active SSIV protein of less than 50% the amount found in the same plant that is not defective for SSIV is determined, advantageously less than 10% and preferably less than 9%, 8.5%, 8%, 7.5%, 7%, 6.5%, 6%, 5.5%, 5%, 4.5%, 4%, 3.5%, 3%, 2.5%, 2%, 1.5%, 1%, 0.5% or even 0.1% the amount found in the same plant that is not defective for SSIV is determined. Techniques that are well known in the art for genetically modifying plants and which are herein adapted so as to cause a reduced synthesis or no synthesis of an SSIV protein will be described in detail further in the present specification. Those techniques include methods for inhibiting or blocking transcription of the SSIV-encoding gene, as well as methods for inhibiting or blocking translation of the SSIV-encoding mRNAs.

In further embodiments of a plant that is rendered defective for SSIV according to the invention, the said plant is genetically modified so as to exhibit a reduced synthesis or no synthesis of the mRNA encoding the said Soluble Starch Synthase IV (SSIV). As intended herein, a reduced synthesis of a mRNA encoding SSIV means that an amount of SSIV-specific mRNA of less than 50% the amount found in the same plant that is not defective for SSIV is determined, advantageously less than 10% and preferably less than exceed 9%, 8.5%, 8%, 7.5%, 7%, 6.5%, 6%, 5.5%, 5%, 4.5%, 4%, 3.5%, 3%, 2.5%, 2%, 1.5%, 1 %, 0.5% or even 0.1 % the amount found in the same plant that is not defective for SSIV is determined. Techniques that are well known in the art for genetically modifying plants and which are herein adapted so as to cause a reduced synthesis or no synthesis of a mRNA encoding SSIV will be described in detail further in the present specification.

In yet further embodiments of a plant that is rendered defective for SSIV according to the invention, the said plant is genetically modified so as to exhibit a reduced synthesis or no synthesis of the SSIV protein that is caused by an inhibition or a blocking of the translation of the SSIV-encoding mRNAs. As intended herein, a reduced translation of a mRNA encoding SSIV means that an amount of SSIV protein of less than 50% the amount found in the same plant that is not defective for SSIV is determined, advantageously less than 10% and preferably less than 9%, 8.5%, 8%, 7.5%, 7%, 6.5%, 6%, 5.5%, 5%, 4.5%, 4%, 3.5%, 3%, 2.5%, 2%, 1.5%, 1%, 0.5% or even 0.1% the amount found in the same plant that is not defective for SSIV is determined. Techniques that are well known in the art for genetically modifying plants and which are herein adapted so as to cause a reduced translation of a mRNA encoding SSIV will be described in detail further in the present specification.

In still further embodiments of a plant that is rendered defective for SSIV according to the invention, the said plant is genetically modified so as to introduce mutation(s) within the nucleic acid sequence of the gene encoding SSIV, which include introducing substitution(s), addition(s) and deletion(s) of one or more nucleotide in the genomic sequence encoding the corresponding SSIV protein. Mutation(s) in the nucleic acid sequence of a SSIV-encoding gene may be introduced in the promoter sequence and/or in the coding region. When introduced in the coding region of a SSIV-encoding gene, mutation(s) may be located in the exon sequences and/or in the intron sequences, including in the sequences consisting of the junctions between an exon sequence and an intron sequence. Notably, plants that are defective for SSIV according to the invention encompass plants that have been genetically modified by introduction or insertion of one or more nucleotides in the nucleic acid sequence of at least one intron sequence and/or one exon sequence of the SSIV-encoding gene. Techniques that are well known in the art .for genetically modifying plants and which are herein adapted so as to cause mutation(s) in a SSIV-encoding gene will be described in detail further in the present specification.

In respect to the modified plants according to the invention that have been rendered defective for both SSIV and starch phosphorylase, the same techniques as those used for inducing a defect in SSIV activity may readily be used also for inducing a defect in starch phosphorylase activity. Especially, it is provided in the examples herein modified plants that have been rendered defective for both SSIV and starch phosphorylase by mutation of both (i) the SSIV-encoding gene and (ii) the starch phosphorylase-encoding gene. More precisely, it is provided in the examples herein modified plants that have been rendered defective for both SSIV and starch phosphorylase by introduction or insertion of one or more nucleotides in the nucleic acid sequence of at least one intron sequence and/or one exon sequence of both (i) the SSIV-encoding gene and (ii) the starch phosphorylase-encoding gene. Techniques that are well known in the art .for genetically modifying plants and which are herein adapted so as to cause mutation(s) in a SSIV-encoding gene and/or in a starch phosphorylase-encoding gene will be described in detail further in the present specification.

Generally, plants that are rendered defective for SSIV by genetic engineering in accordance with the present invention are selected from the group consisting of monocots (monocotyledonous) and dicots (dicotyledonous). Illustrative examples of dicots include plants from *Brassicacae,* Solonacae, legumes, *Arabidopsis* species, including *Arabidopsis thaliana.* Illustrative examples of monocots include cereals like wheat, corn and rice.

Preferably, the plants that are rendered defective for SSIV by genetic engineering in accordance with the present invention are useful plants cultivated by man for nutrition or for technical, in particular industrial, purposes. They are preferably starch-storing plants, encompassing cereal species (rye, barley, oat, wheat, millet, sago etc.), rice, pea, marrow pea, cassava and potato; tomato, rape, soybean, hemp, flax, sunflower, cow pea or arrowroot, fiber-forming plants (e.g. flax, hemp, cotton), oil-storing plants (e.g. rape, sunflower, soybean) and protein-storing plants (e.g. legumes, cereals, soybeans). These plants also encompass fruit trees and palms. Starch-storing plants are preferred. Sugar cane and sugar beet, maize, rice, wheat, cowpea and tomato plants are particularly preferred, and potato plants most preferred.

Thus, the present invention is applicable to all plants which produce or store starch. Examples of such plants include cereals such as maize, wheat, rice, sorghum, barley; fruit producing species such as banana, apple, tomato or pear; root crops such as cassava, potato, yam, beet or turnip; oilseed crops such as rapeseed, canola, sunflower, oil palm, coconut, linseed or groundnut; meal crops such as soya, bean or pea; and any other suitable plant species.

In certain preferred embodiments, plants that are rendered defective for SSIV by genetic engineering in accordance with the present invention belong to a species selected from the group consisting of *Brassicacae,* corn (*Zea mays*)*,* potato (*Solanum tuberosum*)*,* broad bean (*Vicia Faba L*)*,* rice (*Oryza sativa*)*,* beet (*Beta vulgaris*)*,* spinach (*Spinacia oleracea*)*,* green pea (*Pisum sativum*) and wheat (*Triticum*)*.*

In certain preferred embodiments, plants that are rendered defective for SSIV by genetic engineering consist of *Arabidopsis thaliana* wherein the wild type SSIV-encoding gene comprises the nucleic acid sequence of SEQ ID N°1.

In certain other preferred embodiments, plants that are rendered defective for SSIV by genetic engineering consist of *Oryza sativa* (rice) wherein the wild type SSIV-encoding gene comprises the nucleic acid sequence of SEQ ID N° 2 (SSIV-1 isoform) or the nucleic acid sequence of SEQ ID N° 3 (SSIV-1 isoform).

In certain other preferred embodiments, plants that are rendered defective for SSIV by genetic engineering consist of *Triticum aestivum* (wheat) wherein the wild type SSIV-encoding gene comprises the nucleic acid sequence of SEQ ID N° 4.

In certain other preferred embodiments, plants that are rendered defective for SSIV by genetic engineering consist of *Vigna unguiculata* (cowpea) wherein the wild type SSIV-encoding gene comprises the nucleic acid sequence of SEQ ID N° 5.

In one embodiment, plants that are rendered defective for SSIV by genetic engineering consist of *Arabidopsis thaliana* wherein the SSIV-encoding gene has been mutated by insertion of an heterologous T-DNA within the nucleic sequence of an intron. These plants encompass *Arabidopsis thaliana* wherein a T-DNA is inserted within intron 11 and wherein the mutated SSIV-encoding gene comprises the sequence of SEQ ID N° 7, the said transformed plants being termed "*Atss4-1*" herein. These plants also encompass *Arabidopsis thaliana* wherein a T-DNA is inserted within intron 2 of SEQ ID N° 1, the said transformed plants being termed "*Atss4-2*" herein.

Another object of the invention consists of a plant which is rendered defective for Soluble Starch Synthase IV (SSIV), which plant consists of a transformed *Arabidopsis thaliana* comprising a mutated Soluble Starch Synthase IV (SSIV) consisting of SEQ ID N° 7.

Another object of the invention consists of a SSIV-defective plant as described above which has further been rendered defective for starch phosphorylase.

Thus, another object of the present invention consists of a plant defective for SSIV, wherein the said SSIV-defective plant is rendered defective for both (i) SSIV and (ii) starch phosphorylase, including by genetic engineering.

In one embodiment, plants that are rendered defective for SSIV and starch phosphorylase by genetic engineering consist of *Arabidopsis thaliana* wherein (i) the SSIV-encoding gene and (ii) the starch phosphorylase-encoding gene have been mutated by insertion of an heterologous T-DNA within the nucleic sequence of an intron or an exon. These plants encompass *Arabidopsis thaliana* plants that are termed herein "phs1-/ss4-", as shown in the examples.

This invention also relates to a plant part, including any plant tissue or organ, cells, protoplasts, stems, flowers and leaves originating from a plant which is rendered defective for Soluble Starch Synthase IV (SSIV) as defined in the present specification.

This invention also relates to a plant cell originating from a plant which is rendered defective for Soluble Starch Synthase IV (SSIV) as defined in the present specification.

This invention also deals with a plant seed originating from a plant which is rendered defective for Soluble Starch Synthase IV (SSIV) as defined in the present specification.

Various techniques that are generally known in the art and which may be adapted for obtaining plants that are rendered defective for Soluble Starch Synthase IV (SSIV) are described below.

### Methods for obtaining plants defective for SSIV

The methods disclosed hereunder are all aimed at inhibiting or blocking expression or function of a SSIV-encoding gene, or alternatively at inhibiting or blocking the enzyme activity of the SSIV protein, so as to obtain modified plants with a lower number of starch granules per cell, as compared to the same -but non-modified- plants wherein expression or function of the said SSIV-encoding gene is not inhibited or blocked.

Any of the mechanisms known in the art to effect inhibition of gene expression or function can be used to optimally interfere with the final production of a biologically active SSIV protein.

The techniques described below are usable for obtaining any one of the plants defective for SSIV that are disclosed in the present specification, including the plants that are rendered defective for both (i) SSIV and (ii) starch phosphorylase. Thus, although the techniques below relate to the use of SSIV proteins or SSIV-encoding nucleic acids as the targets, these techniques are also readily usable using also starch phosphorylase proteins or starch phosphorylase-encoding nucleic acids as the targets.

### Methods for inhibiting or blocking SSIV enzyme activity

In certain embodiments, plants that are defective for SSIV may be obtained by bringing plants expressing an active SSIV protein into contact with one or more organic or inorganic compounds that inhibit or block SSIV protein enzyme activity.

Such SSIV inhibitor compounds may be selected through any method known in the art, including through a screening method comprising the following steps :
a) providing plants expressing an active SSIV protein;
b) bringing the plants of step a) with a candidate inhibitor compound to be tested;
c) measuring the SSIV enzyme activity in the plant cells originating from the plants obtained at the end of step b);
d) selecting positively the said candidate compound, if the SSIV enzyme activity that is measured at step c) is lower than the SSIV enzyme activity that is measured when step b) is omitted.

Most preferably, according to the screening method above, the said candidate compound is positively selected at step d) if the SSIV enzyme activity that is measured at step c) is less than 10% and preferably less than 9%, 8.5%, 8%, 7.5%, 7%, 6.5%, 6%, 5.5%, 5%, 4.5%, 4%, 3.5%, 3%, 2.5%, 2%, 1.5%, 1%, 0.5% or even 0.1 % the SSIV enzyme activity that is measured at step c), when step b) is omitted.

Candidate compounds may consist of any organic or inorganic compound. The candidate compounds preferably consist of low molecular weight compounds, most preferably candidate compounds having a molecular weight of less than 10 000 Da, even less than 5 000 Da, so that these candidate compounds may easily penetrate within the plant cells.

According to a first aspect of the screening method above, the candidate compounds, more particularly the candidate inhibitor compounds, may be selected from a library of compounds previously synthesised.

According to a second aspect of the screening method above, the candidate compounds, more particularly the candidate inhibitor compounds, are selected from compounds, the chemical structure of which is defined in a database, for example an electronic database.

According to a third embodiment of the screening method above, the candidate compounds, more particularly the candidate inhibitor compounds, are conceived *de novo.*

The candidate compounds may be selected from the group consisting of (a) proteins or peptides, (b) nucleic acids, and (c) organic or mineral chemical compounds.

Illustratively, libraries of pre-selected candidate nucleic acids may be obtained by the one skilled in the art by performing the SELEX method, using the SSIV protein of interest as the target molecule. For performing the SELEX method, the one skilled in the art may refer to the content of the US patents N° US 5,475,096 and N° US 5,270,163, the content of these two documents being herein incorporated by reference.

Further illustratively, candidate compounds may consist of antibodies that are specifically directed against the SSIV protein of interest.

### Methods for inhibiting the expression of a SSIV-encoding gene

As used herein, methods for inhibiting the expression of a gene encoding a SSIV protein encompass methods causing an inhibition or a blocking of the corresponding messenger RNA synthesis, as well as methods causing an inhibition or a blocking of the translation of the corresponding messenger RNA into an active SSIV protein.

Methods for inhibiting gene expression are well known in the art and include, but are not limited to, homology-dependent gene silencing, antisense technology, RNA interference (RNAi), and the like. The general term homology-dependent gene silencing encompasses the phenomenon of cis-inactivation, trans-inactivation, and cosuppression (disclosed in Finnegan et al. (1994) Biotech. 12: 883-888; and Matzke et al. (1995) Plant Physiol. 107: 679-685; both incorporated herein in their entirety by reference). These mechanisms represent cases of gene silencing that involve transgene/transgene or transgene/endogenous gene interactions that lead to reduced expression of protein in plants. A "transgene" is a recombinant DNA construct that has been introduced into the genome by a transformation procedure. As one alternative, incorporation of SSIV-specific antisense RNA into plants can be used to inhibit the expression of endogenous SSIV-encoding genes and produce a functional mutation within the genome. The effect is achieved by introducing into the cell(s) DNA that encodes RNA that is complementary to the sequence of a SSIV-encoding mRNA (disclosed in e.g. Bird et al. (1991) Biotech and Gen. Eng. Rev. 9: 207-226; incorporated herein in its entirety by reference). Manipulation of plant gene expression by homology-dependent gene silencing, antisense RNA, RNAi, or other inhibitory mechanism can be used to optimally interfere with starch biosynthesis through the inhibition or the blocking of the final production of an active SSIV protein, thereby resulting in the production of starch granules of increased size within the plant, or a desired plant part thereof, including plant cells and plant seeds thereof.

Any nucleic acid that may be used for inhibiting the expression of a SSIV-encoding gene is preferably targeted against the SSIV-encoding portion of the corresponding DNA or RNA, including mRNA, that is found in every SSIV-encoding sequence, namely the nucleic acid portion encoding the N-terminal portion of a SSIV protein, more precisely the N-terminal portion encompassing the amino acid sequence located from the amino acid residue at position 200 to the amino acid residue at position 420 of any of the SSIV amino acid sequence

The above embodiment is preferred when selecting the appropriate nucleic acids encoding a sense or an antisense nucleic acid, as well as a RNAi or also a ribozyme.

### Tilling

For obtaining plants that are rendered defective for SSIV according to the invention, another technique that is termed "tilling" (for "Targeting Induced Local Lesions IN Genomes"), and which is well known from the one skilled in the art, may also be used. The method of tilling is for example disclosed by Mc Callum et al. (2000, Plant Physiology, Vol. 123 : 439-442).

The identification and the characterization of mutants in SSIV gene take place in four steps, using a TILLING system, according to the method below which comprises the steps of :
a) generating of a collection of plant mutants by chemical mutagenesis. The mutations are obtained by chemical mutagenesis with ethylmethyl sulfonate (EMS) (Koornneef et al., 1982): mutagenesis on 30 000 seeds, sowing of the mutants and production of the M2 generation from 5000 M1 plants.
b) extracting DNA from 20 plants per M2 family and formation of DNA pools in 3 dimensions from a population of 100 000 M2 plants (5000 families).
c) performing a PCR amplification of the targeted genes and search for mutations by denaturating HPLC. The SSIV gene is sequenced The PCR products are subsequently denatured and then paired so as to allow the formation of heteroduplexes. The mutations are then detected either by denaturing HPLC (McCallum et al., 2000) or by means of an enzyme which allows detection of "mismatches" in the heteroduplex (CEL1 enzyme, Oleykowski et al., 1998), and
d) characterizing the mutants in order to evaluate their behaviour with starch granules. Step d) preferably consists of determining the mean size of the starch granules found in the mutant plants, or, alternatively, comparing the mean size of the starch granules found in the mutant plants with the mean size of the starch granules found in SSIV non-defective plants, including plants from a wild-type ecotype.

### Antisense and sense nucleic acids

### 1. Antisense nucleic acids

Use of antisense and sense nucleotide sequences for the silencing of plant genes is well known in the art. Antisense suppression of gene expression is disclosed notably in the following documents : U.S. Pat. No. 5,190,931 and 5,272,065; U.S. Pat. No. 5,478,369; U.S. Pat. No. 5,453,566; Weintrab et al. (1985) Trends Gen. 1:22-25; and Bourque and Folk (1992) Plant Mol. Biol. 19:641-647. Antisense nucleotide sequences are particularly effective in manipulating metabolic pathways to alter the phenotype of an organism. Reduction in gene expression can be mediated at the DNA level and at transcriptional, post-transcriptional, or translational levels. For example, it is thought that dsRNA suppresses gene expression by both a post-transcriptional process and by DNA methylation. (Sharp & Zamore (2000) Science 287:2431-2433). Antisense polynucleotides, when introduced into a plant cell, are thought to specifically bind to their target polynucleotide and inhibit gene expression by interfering with transcription, splicing, transport, translation and/or stability. Antisense polynucleotides can be targeted to chromosomal DNA, to a primary RNA transcript or to a processed mRNA. Preferred target regions include splice sites and translation initiation and termination codons, and other sequences within the open reading frame.

It is understood that the antisense polynucleotides of the invention need not be completely complementary to the target SSIV-encoding gene or to the target SSIV-encoding RNA, nor that they hybridize to each other along their entire length to modulate expression or to form specific hybrids. Furthermore, the antisense polynucleotides of the invention need not be full length with respect to the target SSIV-encoding gene or to the target SSIV-encoding RNA. In general, greater homology can compensate for shorter polynucleotide length. Typically antisense molecules will comprise an RNA having 60-100% sequence identity with at least 8, 10, 12, 14, 15, 16, 17, 18, 19, 20, 25, 30, 35, 40, 45, 50, 75, 100, 200, 500, or at least 1000 consecutive nucleotides of the target gene. Preferably, the sequence identity will be at least 70%, more preferably at least 75%, 80%, 85%, 90%, 95%, 98% and most preferably at least 99%. Target genes include SSIV-encoding genes comprising a nucleic acid sequence selected form the group consisting of SEQ ID N° 1 to SEQ ID N°5.

Antisense polynucleotides may be designed to bind to exons, introns, exon-intron boundaries, the promoter and other control regions, such as the transcription and translational initiation sites. Methods for inhibiting plant gene expression using antisense RNA corresponding to entire and partial cDNA, 3' non-coding regions, as well as relatively short fragments of coding regions are known in the art. (U.S. Pat. Nos. 5,107,065 and 5,254,800, the contents of which are incorporated by reference; Sheehy et al. (1988) Proc. Natl. Acad. Sci. USA 85:8805-8809; Cannon et al. (1990) Plant Mol. Biol. 15:39-47; and Chang et al. (1989) Proc. Nat'l. Acad. Sci. USA 86:10006-10010). Furthermore, Van der Krol et al. (1988, Biotechniques 6:958-976), describe the use of antisense RNA to inhibit plant genes in a tissue-specific manner.

Gene specific inhibition of expression in plants by an introduced sense polynucleotide is termed "co-suppression." Methods for co-suppression are known in the art. Partial and full-length cDNAs have been used for the co-suppression of endogenous plant genes. (U.S. Pat. Nos. 4,801,340; 5,034,323; 5,231,020; and 5,283,184, the contents of each are herein incorporated by reference; Van der Kroll et al. (1990) The Plant Cell 2:291-299, Smith et al. (1990) Mol. Gen. Genetics 224:477-481; and Napoli et al. (1990) The Plant Cell 2:279-289).

### 2. sense nucleic acids

For sense suppression, it is believed that introduction of a sense polynucleotide blocks transcription of the corresponding target gene. In the methods of the present invention, the sense polynucleotide will have at least 80%, 90%, 95% or more sequence identity with the target plant SSIV-encoding gene or to the target SSIV-encoding RNA. The introduced sense polynucleotide need not be full length relative to the target gene or transcript. Preferably, the sense polynucleotide will have at least 95%, 96%, 97%, 98%, 99% or 100% sequence identity with at least 100 consecutive nucleotides of a SSIV-encoding gene or of a SSIV-encoding RNA, including the nucleotide sequences listed in SEQ ID NOs:1-5. The regions of identity comprise introns and and/or exons and untranslated regions. The introduced sense polynucleotide is stably integrated into a plant chromosome or extrachromosomal replicon.

In the methods of the invention, the sense polynucleotides will encode the amino acid sequence of the target plant SSIV protein or an amino acid sequence that is at least 90%, 95%, 98%, 99% or more identical to the target plant SSIV protein. Preferably, the sense polynucleotides, including the polynucleotide sequences listed in SEQ ID NOs:1-5 will have 5 or fewer alterations in amino acid residues that are not highly conserved between species. The introduced sense polynucleotide is stably integrated into a plant chromosome or extrachromosomal replicon.

### Double-stranded RNA (also termed RNA interference or RNAi)

In another aspect, the invention provides a double-stranded RNA (dsRNA) for the post-transcriptional inhibition of a target plant SSIV-encoding gene. In the methods of the present invention, the dsRNA is specific for a target SSIV-encoding gene or for a target SSIV-encoding RNA. Preferably, the dsRNA will be at least 25, 30, 35, 40, 45, 50, 55, 60, 65, 70, 75, 80, 85, 90, 95, or 100 base pairs in length (Hamilton & Baulcombe (1999) Science 286:950). Typically, the hybridizing RNAs of will be of identical length with no over hanging 5' or 3' ends and no gaps. However, dsRNAs having 5' or 3' overhangs of up to 100 nucleotides may be used in the methods of the present invention.

Thus, in one embodiment, the invention provides a dsRNA, comprising: a first ribonucleic acid having at least 95% complementary with at least 25, 30, 35, 40, 45, 50, 55, 60, 65, 70, 75, 80, 85, 90, 95, or 100 consecutive nucleotides of a SSIV-encoding gene or of a SSIV-encoding RNA, including nucleotide sequences listed in SEQ ID N0s:1-5; and a second ribonucleic acid that is substantially complementary to the first ribonucleic acid.

The dsRNA may comprise ribonucleotides or ribonucleotide analogs, such as 2'-O-methyl ribosyl residues or combinations thereof. (U.S. Pat. Nos. 4,130,641 and 4,024,222). A dsRNA polyriboinosinic acid:polyribocytidylic acid is described in U.S. Pat. No. 4,283,393. Methods for making and using dsRNA are known in the art. One method comprises the simultaneous transcription of two complementary DNA strands, either in vivo, or in a single in vitro reaction mixture. (U.S. Pat. No. 5,795,715, the content of which is incorporated herein by reference). In the methods of the present invention, the dsRNA is expressed in a plant cell through the transcription of two complementary RNAs.

### Methods for disrupting a SSIV-encoding gene

Various methods known in the art may be used for disrupting a SSIV-encoding gene, including for introducing mutations in a SSIV-encoding gene.

Mutant plants defective for SSIV may be generated in many ways including chemical mutagenesis, ionizing radiation-induced mutagenesis X-rays-induced mutagenesis, gamma rays-induced mutagenesis, U.V.-induced mutagenesis, site directed mutagenesis and also by random mutagenesis including transposon-induced mutagenesis.

As set forth above, the manipulation of the level of gene expression or protein activity of plant SSIV protein of the present invention may also be carried out by causing a disruption in a SSIV-encoding gene in a plant cell. As defined above, the term "causing a disruption in a gene" is used herein to refer to a means of altering the expression of a gene. Suitable techniques and methods also include gene disruption techniques such as, for example, the use of ribozymes, site-directed and random (chemical or radiation-induced) mutagenesis, T-DNA or transposon insertions, and alteration of expression of target gene accessory proteins.

Thus, one embodiment of the invention is a method for obtaining a plant defective for SSIV, which defective plant produces a lower number of starch granules per cell as compared with the same plant that is not defective for SSIV, the said method comprising the steps of :
a) causing a disruption in a SSIV-encoding gene in a plant cell; and
b) regenerating a plant from the plant cell, wherein the plant has a disruption in the endogenous SSIV-encoding gene and the plant exhibits an altered starch metabolism, particularly a lower number of starch granules per cell as compared with the same plant cells wherein the said SSIV-encoding gene is not disrupted.

In preferred embodiments of the method above, step a) comprises the following steps :
a1) exposing plant cells to an agent able to cause a disruption in a SSIV-encoding gene;
a2) screening, in the plant cells obtained at the end of step a1), for the occurrence of a disruption of the SSIV-encoding gene;
a3) selecting positively the plant cells possessing a disrupted SSIV-encoding gene.

In these preferred embodiments, step b) is performed exclusively with cells that have previously been positively selected at step a3).

Generally, step a2) of screening is performed by a PCR amplification of the SSIV-encoding nucleic acid or part thereof, and detecting a disruption in the amplified product, e.g. by sequencing the amplification product or by detecting mutations with appropriate oligonucleotide probes.

In specific embodiments, step a1) consists of bringing into contact cultured plant cells of interest with a random mutagenesis agent, such as a chemical compound or a mixture of chemical compounds, ionizing radiation, X-rays, Gamma rays, Ultraviolet (UV) light and the like.

### 1. Disruption by ribozymes

One such technology is the use of ribozymes. In the methods of the invention ribozymes are used to reduce the expression of a target SSIV-encoding gene or of a target SSIV-encoding RNA that is AGB1, GPA1 or an ortholog thereof.

Methods for making and using ribozymes are known to those skilled in the art. (U.S. Pat. Nos. 6,025,167; 5,773,260; 5,695,992; 5,545,729; 4,987,071; and 5,496,698, the contents of which are incorporated herein by reference; Haseloff & Gerlach (1988) Nature 334:586-591; Van Tol et al. (1991) Virology 180:23; Hisamatsu et al. (1993) Nucleic Acids Symp. Ser. 29:173; Berzal-Herranz et al. (1993) EMBO J. 12:2567 (describing essential nucleotides in the hairpin ribozyme); Hampel & Tritz, (1989) Biochemistry 28:4929; Haseloff et al. (1988) Nature 334:585-591; Haseloff & Gerlach (1989) Gene 82:43 (describing sequences required for self-cleavage reactions); and Feldstein et al. (1989) Gene 82:53). The one skilled in the art may find details of various ribozyme motifs, and hairpin ribozyme in particular, in the following documents : Ahsen & Schroeder (1993) Bioassays 15:299; Cech (1992) Curr. Opi. Struc. Bio. 2:605; and Hampel et al. (1993) Methods: A Companion to Methods in Enzymology 5:37.

The portion of the ribozyme that hybridizes to the target SSIV-encoding gene or to the target SSIV-encoding RNA transcript is typically at least 7 nucleotides in length. Preferably, this portion is at least 8, 9, 10, 12, 14, 16, 18 or 20 or more nucleotides in length. The portion of the ribozyme that hybridizes to the target SSIV-encoding gene or to the target SSIV-encoding RNA need not be completely complementary to the said target, as long as the hybridization is specific for the said target. In a preferred embodiment, the ribozyme will contain a portion having at least 7 or 8 nucleotides that have 100% complementarity to a portion of the target SSIV-encoding RNA. In one embodiment, the target RNA transcript corresponds to a SSIV-encoding gene comprising the nucleotide sequences listed in SEQ ID NOs:1-5.

### 2. Disruption by T-DNA insertion or transposon insertion

Similarly, methods for the disruption of target plant SSIV-encoding genes include T-DNA or transposon insertion methodologies. As part of the disease process, bacteria of the genus Agrobacterium transfer a segment of DNA to the nucleus of the host plant cell. This transferred DNA (T-DNA) integrates at random locations in the host genome. Transgenic plants with T-DNA integrations within the open reading frame or the promoter region of the target gene are identified using a polymerase chain reaction screening procedure that is well known by those skilled in the art. (Krysan et al. (1996) Proc. Nat'l. Acad. Sci. USA 93:8145-50).

Target SSIV-encoding gene inactivation may also be performed via transposon insertion in the promoter or coding region of the said SSIV-encoding gene. In the methods of the present invention, the transposon used to inactivate the gene is native to the species in which the mutagenesis is being conducted (e.g., Blauth et al. (2002) Plant Mol. Biol. 48:287-97) or derived from a heterologous species (e.g., Kohli et al. (2001) Mol. Genet. Genomics 266:1-11). In either case, a polymerase chain reaction method analogous to that described above is utilized to identify plant lines with the desired gene disruption. Insertional mutagenesis technologies are reviewed by Parinov & Sundaresan (2000) Curr. Opin. Biotechnol. 11:157-61; and Krysan, Young & Sussman (1999) Plant Cell 11:2283-90.

### 3. Disruption by introducing other mutations in a SSIV-encoding gene.

Other well-known gene disruption technologies for inhibition of a target plant SSIV-encoding gene can be used in the methods of the invention. One such method relates to directed or random mutagenesis of a target SSIV-encoding gene. Thus, in another embodiment of the invention, directed alteration of a target SSIV protein activity is performed through genetic manipulation of the cloned SSIV-encoding cDNA coding region. The directed genetic manipulation of the cloned cDNA generates a mutation in a highly conserved region of the SSIV-encoding target, resulting in a non-conservative amino acid substitution which inactivates or alters (i.e. decreases) the activity of the target SSIV protein in a genetically dominant manner. Alternatively, directed genetic manipulation of cloned SSIV-encoding cDNA is used to produce a deletion (so-called truncation), or addition of one or more amino acids within the amino acid sequence of the corresponding SSIV protein, including a deletion or addition of one or more amino acids to the amino-terminal and/or carboxy-terminal end of the corresponding SSIV protein.

Methods for such directed genetic manipulations are generally known in the art. For example, plants producing an inactive SSIV protein can be prepared by mutations in the cloned DNA sequence encoding the native SSIV protein of interest. Methods for mutagenesis and nucleotide sequence alterations are well known in the art. (Walker & Gaastra, eds. (1983) Techniques in Molecular Biology (MacMillan Publishing Company, New York); Kunkel (1985) Proc. Natl. Acad. Sci. 82:488-492; Kunkel et al. (1987) Methods Enzymol. 154:367-382; Sambrook et al. (1989) Molecular Cloning: A Laboratory Manual (2d ed., Cold Spring Harbor Laboratory Press, Plainview, N.Y.; U.S. Pat. No. 4,873,192; and the references cited therein; all of which are herein incorporated by reference).

Thus, one embodiment of the invention includes methods for obtaining plants defective for SSIV by introducing mutations within a SSIV-encoding gene, wherein said method comprises the steps of:
a) introducing into a plant cell an expression cassette comprising a sense nucleotide sequence that is a mutated SSIV-encoding sequence and that contains a dominant site-directed mutation; and
b) regenerating a plant that has a stably integrated the expression cassette introduced at step a) in the plant cell, wherein the plant produces a lower number of starch granules per cell as compared to the same plant that is not defective for SSIV.

### 4. Disruption by random mutagenesis

The methods of the invention include methods for disrupting a target SSIV-encoding gene in a plant using random mutagenesis. For the random mutagenesis of a target gene, the mutagenesis is performed using chemicals, irradiation.

It is to be noted that T-DNA, or transposon insertion which have already been disclosed above are also encompassed by random mutagenesis techniques.

Thus, in another embodiment of the invention, mutagenesis of a SSIV-encoding gene may be performed randomly, using either a chemical mutagenic compound or through irradiation of the DNA. Inactivation of the target SSIV-encoding gene is accomplished by generating a mutation resulting in a non-conservative amino acid substitution, preferably in a highly conserved region of the target SSIV-encoding gene. Alternatively, target SSIV-encoding gene inactivation is obtained through alteration of any of the codons in the coding region of the target gene that result in the truncation of the resulting SSIV protein. Such techniques for the generation of random mutations in target genes are well known in the art. (Koncz, Chua & Schell, eds., (1993) Methods in Arabidopsis Research (World Scientific Publishing, River Edge, N.J.)).

### Transgenic plants defective for SSIV

The present invention encompasses transgenic plants having stably integrated into their genome an expression cassette comprising a nucleotide sequence that is antisense, sense, dsRNA, a ribozyme, or an inverted repeat to a plant nucleotide sequence consisting of a SSIV-encoding sequence. Further encompassed by the present invention are transgenic plants having a disruption in a gene consiting of a SSIV-encoding gene endogenous to the plant. The transgenic plants of the invention include dicots as well as monocots, as described elsewhere in the present specification.

This invention encompasses transgenic plants defective for SSIV originating from a plant species selected from the group consisting of *Brassicacae,* corn (*Zea mays*), potato (*Solanum tuberosum*), broad bean (*Vicia Faba L*), rice (*Oryza sativa*), beet (*Beta vulgaris*), spinach (*Spinacia oleracea*), green pea (*Pisum sativum*) and wheat (*Triticum aestivum*)*.*

### Methods for obtaining transgenic plants defective for SSIV

Transgenic plants of the present invention are made according to methods set forth herein and other methods known in the art.

The polynucleotides of the invention may be introduced into any plant or plant cell. By plants is meant monocotyledons and dicotyledons, and the cells, organs and tissues thereof. Methods for the introduction of polynucleotides into plants and for generating transgenic plants are known to those skilled in the art. (Weissbach & Weissbach (1988) Methods for Plant Molecular Biology, Academic Press, N.Y.; Grierson & Corey (1988) Plant Molecular Biology, 2d., Blackie, London; Miki et al. (1993) Procedures for Introducing Foreign DNA into Plants, CRC Press, Inc. pp.67-80).

Vectors containing the expression cassettes of the invention are used in the methods of the invention. By "vector" it is intended to mean a polynucleotide sequence that is able to replicate in a host cell. Preferably, the vector contains genes that serve as markers useful in the identification and/or selection of transformed cells. Such markers include, but are not limited to, barnase (bar), G418, hygromycin, kanamycin, bleomycin, gentamicin, and the like. The vector can comprise DNA or RNA and can be single or double stranded, and linear or circular. Various plant expression vectors and reporter genes are described in Gruber et al. in Methods in Plant Molecular Biology and Biotechnology, Glick et al., eds, CRC Press, pp.89-119, 1993; and Rogers et al. (1987) Meth Enzymol 153:253-277. In a preferred embodiment, the vector is an E. coli/A. tumefaciens binary vector. In another preferred embodiment of the invention the expression cassette is inserted between the right and left T-DNA borders of an Agrobacterium Ti plasmid.

A recombinant "expression cassette" of the invention contains 5' and 3' regulatory sequences necessary for transcription and termination of the polynucleotide of interest. Expression cassettes generally comprise at least one promoter and a transcriptional terminator. Promoters of the present invention are described more fully herein. In certain embodiments of the invention, other functional sequences are included in the expression cassettes. Such functional sequences include, but are not limited to, introns, enhancers, and translational initiation and termination sites and polyadenylation sites. The control sequences function in at least one plant, plant cell, or plant tissue. These sequences may be derived from one or more genes, or can be created using recombinant technology. Polyadenylation signals include, but are not limited to, the Agrobacterium octopine synthase signal (Gielen et al (1984) EMBO J. 3:835-846) and the nopaline synthase signal (Depicker et al. (1982) Mol. and Appl. Genet. 1:561-573). Transcriptional termination regions include, but are not limited to, the terminators of the A. tumefaciens Ti plasmid octopine synthase and nopaline synthase genes. (Ballas et al. (1989) Nuc. Acid Res. 17:7891-7903; Guerineau et al. (1991) Mol. Gen. Genet. 262:14144; Joshi et al. (1987) Nuc. Acid Res. 15:9627-9639; Mogen et al. (1990) Plant Cell 2:1261272; Munroe et al. (1990) Gene 91:15158; Proudfoot (1991) Cell 64:671-674; and Sanfacon et al. (1991) Genes Devel. 5:14149).

The expression cassettes of the invention may be covalently liked to a polynucleotide encoding a selectable or screenable marker. Examples of such markers include genes encoding drug or herbicide resistance, such as hygromycin resistance (hygromycin phosphotransferase (HPT)), spectinomycin (encoded by the aadA gene), kanamycin and gentamycin resistance (neomycin phosphotransferase (nptll)), streptomycin resistance (streptomycin phosphotransferase gene (SPT)), phosphinothricin or basta resistance (barnase (bar)), chlorosulfuron reistance (acetolactase synthase (ALS)), chloramphenicol resistance (chloramphenicol acetyl transferase (CAT)), G418 resistance, lincomycin resistance, methotrexate resistance, glyphosate resistance, and the like. In addition, the expression cassettes of the invention may be covalently linked to genes encoding enzymes that are easily assayed, for example, luciferase, alkaline phosphatase, beta-galactosidase (beta-gal), beta-glucuronidase (GUS), and the like.

Methods include, but are not limited to, electroporation (Fromm et al. (1985) Proc Natl Acad Sci 82:5824; Riggs et al. (1986) Proc. Nat'l. Acad. Sci. USA 83:5602-5606); particle bombardment (U.S. Pat. Nos. 4,945,050 and 5,204,253, the contents of which are herein incorporated by reference; Klein et al. (1987) Nature 327:70-73; McCabe et al. (1988) Biotechnology 6:923-926); microinjection (Crossway (1985) Mol Gen. Genet. 202:179-185; Crossway et al. (1986) Biotechniques 4:320-334); silicon carbide-mediated DNA uptake (Kaeppler et al. (1990) Plant Cell Reporter 9:415-418); direct gene transfer (Paszkowski et al. EMBO J. 3:2717-2722); protoplast fusion (Fraley et al. (1982) Proc. Nat'l. Acad. Sci. USA 79:1859-1863); polyethylene glycol precipitation (Paszowski et al.(1984) EMBO J. 3:2717-2722; Krens et al (1982) Nature 296:72-74); silicon fiber delivery; agroinfection (U.S. Pat. No. 5,188,958, incorporated herein by reference; Freeman et al. (1984) Plant Cell Physiol. 25:1353 (liposome-mediated DNA uptake); Hinchee et al. (1988) Biotechnology 6:915-921; Horsch et al. (1984) Science 233:496-498; Fraley et al. (1983) Proc. Nat'l. Acad. Sci. USA 80:4803; Hernalsteen et al. (1984) EMBO J. 3:3039-3041; Hooykass-Van Sloteren et al. (1984) Nature 311:763-764; Grimsley et al. (1987) Nature 325:1677-1679; Gould et al. (1991) Plant Physiol. 95:426-434; Kindle (1990) Proc. Nat'l. Acad. Sci. USA 87:1228 (vortexing method); Bechtold et al. (1995) In Gene Transfer to Plants, Potrykus et al., eds., Springer-Verlag, NewYork, N.Y. pp19-23 (vacuum infiltration); Schell (1987) Science 237:1176-1183; and Plant Molecular Biology Manual, Gelvin & Schilperoort, eds., Kluwer, Dordrecht, 1994).

Preferably, the polynucleotides of the invention are introduced into a plant cell by agroinfection. In this method, a DNA construct comprising a polynucleotide of the invention is inserted between the right and left T-DNA borders in an Agrobacterium tumefaciens vector. The virulence proteins of the A. tumefaciens host cell will mediate the transfer of the inserted DNA into a plant cell infected with the bacterium. As an alternative to the A. tumefaciens/Ti plasmid system, Agrobacterium rhizogenes-mediated transformation may be used. (Lichtenstein & Fuller in: Genetic Engineering, Volume 6, Ribgy, ed., Academic Press, London, 1987; Lichtenstein & Draper, in DNA Cloning, Volume 2, Glover, ed., IRI Press, Oxford, 1985).

If one or more plant gametes are transformed, transgenic seeds and plants can be produced directly. For example, a method of producing transgenic seeds and plants involves agroinfection of the flowers and collection of the transgenic seeds produced from the agroinfected flowers. Alternatively, transformed plant cells can be regenerated into plants by methods known to those skilled in the art. (Evans et al, Handbook of Plant Cell Cultures, Vol I, MacMollan Publishing Co. New York, 1983; and Vasil, Cell Culture and Somatic Cell Genetics of Plants, Acad. Press, Orlando, Vol 11, 1986).

Once a transgenic plant has been obtained, it may be used as a parent to produce progeny plants and plant lines. Conventional plant breeding methods can be used, including, but not limited to, crossing and backcrossing, self-pollination, and vegetative propagation. Techniques for breeding plants are known to those skilled in the art. The progeny of a transgenic plant are included within the scope of the invention, provided that the progeny contain all or part of the transgenic construct. Progeny may be generated by both asexual and sexual methods. Progeny of a plant include transgenic seeds, subsequent generations of the transgenic plant, and the seeds thereof.

Thus, one embodiment of the invention comprises using conventional breeding methods and/or successive iterations of genetic transformation to produce SSIV-defective plant lines with genotypes including disruption of a SSIV-encoding gene; and phenotypes including an altered starch metabolism, which encompasses phenotypes including a lower number of starch granules per cell as compared with the same plants that are not defective for SSIV.

### Methods

Thus, the present invention is also related to a method for obtaining a transformed plant defective for Soluble Starch Synthase IV (SSIV) comprising the steps of :
a) providing a plant cell;
b) introducing into the plant cell provided at step a) a DNA construct which renders the said plant cell defective for Soluble Starch Synthase IV (SSIV) activity, whereby a transformed plant cell defective for Soluble Starch Synthase IV (SSIV) activity is obtained;
c) regenerating a whole plant from the transformed plant cell obtained at the end of step b).

The "DNA construct" used in the method above encompasses any "expression cassette" that is disclosed in the present specification. Consequently, the said DNA construct encompasses expression cassettes comprising a nucleotide sequence that is antisense, sense, dsRNA, a ribozyme, or an inverted repeat to a plant nucleotide sequence consisting of a SSIV-encoding sequence that are described herein.

In certain embodiments of the method above, it may be used at step b) a DNA construct which renders the said plant cell also defective for starch phosphorylase activity, whereby a transformed plant cell defective for Soluble Starch Synthase IV (SSIV) activity is obtained at step c), that is also defective for starch phosphorylase activity.

According to a first aspect of these embodiments, for rendering a plant defective for both (i) SSIV and (ii) starch phosphorylase, a unique DNA construct may be used at step b), that may cause the inactivation of both the SSIV-encoding gene and of the starch phosphorylase-encoding gene. Illustratively, such a DNA construct may consist of a T-DNA, or any vector bearing a T-DNA, that is not target-specific.

According to a second aspect of these embodiments, for rendering a plant defective for both (i) SSIV and (ii) starch phosphorylase, at least two distinct DNA constructs are used at step b), respectively a first DNA construct that is SSIV-specific and a second DNA construct that is starch phosphorylase-specific. Illustratively, such DNA constructs may comoprise, or consist of, DNA constructs encoding target-specific sense or antisense nucleic acids, respectively for SSIV and starch phosphorylase.

This invention also deals with transformed plants, also termed transgenic plants, that are defective for SSIV and which have been obtained by the method above.

The said transformed plants, or transgenic plants, may be selected from the group consisting of monocots and dicots.

Preferably, the said transformed plants, or transgenic plants, belong to a plant species selected from the group consisting of *Brassicacae,* corn (*Zea mays*)*,* potato (*Solanum tuberosum*)*,* broad bean (*Vicia Faba L*)*,* rice (*Oryza sativa*)*,* beet (*Beta vulgaris*)*,* spinach (*Spinacia oleracea*)*,* green pea (*Pisum sativum*) and wheat (*Triticum aestivum*)*.*

A further object of the present invention consists of a transformed plant, or a transgenic plant, defective for SSIV and wherein the SSIV-encoding gene has been inactivated by mutation in a SSIV sequence selected from the group consisting of the nucleic acid sequences of SEQ ID N° 1 (*Arabidopsis thaliana*)*,* SEQ ID N°2 and 3 *(Oryza sativa),* SEQ ID N° 4 (*Triticum aestivum*) and SEQ ID N° 5 (*Vignaunguiculata*)*.*

Notably, this invention pertains to transformed plans, or to transgenic plants, comprising a mutated Soluble Starch Synthase IV (SSIV) gene consisting of SEQ ID N° 7.

This invention also concerns plant parts, including plant cells and plant seeds originating from a transformed, or a transgenic, plant as defined above, which contain a lower number of starch granules per cell as compared with the same material from the same plants that are not defective for SSIV.

### Methods for producing starch and uses thereof.

As already disclosed in many places earlier in the present specification, a plant according to the invention that has been rendered defective for SSIV possesses in their cells a lower number of starch granules per cell, but of a greater size, than the same plant that is not defective for SSIV.

As already mentioned, plants that have been rendered defective for SSIV produce starch granules having a size of at least 1.5 times higher starch granules originating from the same but non SSIV-defective plants, whereas the structure of the starch granules is unaffected in such plants that have been rendered defective for SSIV.

Further, it has been found that plants that have been rendered defective for both (i) SSIV and (ii) starch phosphorylase produce starch granules having a size of at least 4 times higher than starch granules originating from the same but non SSIV-defective and non starch phosphorylase-defective plants.

Still further, it has been shown that the plants producing a functional SSIV protein (wild-type plants) produce rather flat-shaped starch granules, whereas SSIV-defective plants produce rather round-shaped starch granules.

Firstly, the higher size of the starch granules originating from the SSIV-defective plants allow better yields in industrial starch production, at least simply because large size starch granules better and faster sediment than small size starch granules. Thus, at least when performing steps of washing the starch granules following their extraction from the plant tissue, the loss of starch granules is lowered when using starch granules originating from SSIV-defective plants.

Secondly, while the applicant does not wish to be bound by any particular theory, it is believed that high size and round-shaped starch granules originating from SSIV-defective plants are far less subject to enzyme hydrolysis during the industrial process of starch production, thus further increasing yields in the final product.

This essential feature of a plant defective for SSIV according to the invention allows an easier and less expensive extraction of starch from the pertinent plants parts, including grains or seeds, as compared with plants that are not defective for SSIV.

Thus, the present invention further extends to the production and uses of starch originating from a plant according to the invention that is rendered defective for SSIV.

The present invention also relates to a process for the production of starch comprising the step of introducing a transgenic plant cell, plant and/or part of a plant according to the invention into a process for the production/extraction of starch.

The present invention further relates to a process for the production of modified starch comprising the step of introducing a starch according to the invention into a process of chemical and/or physical modification/treatment of starch.

This invention also pertains to a method for producing starch comprising a step of extracting starch from a plant defective for Soluble Starch Synthase IV (SSIV) which is disclosed in the present specification.

Processes for starch extraction from plants, plant cells, or parts thereof are well known in the art. Such processes are described, for example, in Eckhoff et al. (Cereal Chem. 73 (1996), 54-57). Extraction of maize starch is achieved by, e.g., "wet-milling". Other methods for starch extraction from various plants are described, e.g., in Starch: Chemistry and Technology (eds.: Whistler, BeMiller and Paschall (1994) 2nd Edition, Academic Press Inc. London LTD; ISBN 0-12-746270-8; Chapter XII, page 417-468: Corn and Sorghum Starches: Production; by Watson, S. A.; Chapter XIII, page 469-479: Tapioca, Arrowroot and Sago Starches: Production by Corbishley and Miller; Chapter XIV, page 479-490: Potato Starch: Production and Uses; by Mitch; Chapter XV, page 491-506: Wheat starch: Production, Modification and Uses; by Knight and Olson; and Chapter XVI, page 507-528: Rice starch: Production and Uses; by Rohwer and Klem). Means usually used in methods for the extraction of starches from plant materials are separators, decanters, hydrocyclones and different kinds of machines for drying the starch, e.g., spray drier or jet drier.

The starch product that may be obtained by easier extraction and/or increased yield extraction from a plant that is rendered defective for SSIV according to the invention may be conventionally used, both in foodstuff products and in non-foodstuff products.

The present invention is further illustrated, without in any way being limited to, the examples hereunder.

### EXAMPLES

### A. MATERIAL AND METHODS OF THE EXAMPLES

### A.1. Arabidopsis lines, growth conditions and media

Mutants lines of *Arabidopsis thaliana* were obtained from the T-DNA mutant Collections generated at URGV, INRA, Versailles (Bechtold et al., 1993, C. R. Acad. Sci. Paris Life Sci. 316, 1194-1199; Bouchez et al., 1993, C.R. Acad. Sci. Paris Life Sci. 316, 1188-1193) and the GABI-KAT mutant Collection (Rosso et al., 2003, Plant Mol Biol. 53,247-259.). Wild type ecotypes (Wassilewskija, WS and Columbia, Col-O) and mutant lines were grown in growth cabinets under 16 h light/8 h dark photoregime at 23 °C (day)/20 °C (night), 70% humidity and a light intensity at the plant levels of 120 µE m⁻² s⁻¹ supplied by white fluorescent lamps. Seeds were sown in soil and irrigated with 0.5X MS medium (Murashige and Skoog, 1962, Plant Physiol. 15, 473-497).

### A.2. RNA extraction and Reverse Transcription

Total RNA was isolated according to (Prescott and Martin 1987, Plant Mol. Biol. Rep.4, 219-224.). Prior to cDNA synthesis and, in order to remove contaminating genomic DNA, the RNA preparations were incubated with 10 Units of DNAse I FPLC Pure for 10 min at 37 °C, extracted with phenol and chloroform, precipitated and dissolved in nuclease-free MiIIiQ-water. First strand complementary DNA (cDNA) was synthesized from 10 µg total RNA using MMLV-RT and oligo(dT)12-18 primer, according to manufacturer's instructions. The reaction was incubated at 37 °C during 2 h and stopped by adding 1 ml of nuclease-free MilliQ-water. All the reagents were from Amersham Biosciences (Uppsala, Sweden).

### A.3. Production of polyclonal antibody against SSIV

Leaf total RNA was used to obtain cDNA as described above. Oligonucleotides SA215 (5'-CATATGGAGACTGATGAAAGGATT-3' **SEQ ID N° 12**) and SA216 (5'-CTCGAGTTCTTTATAAACGTTGGC-3' **SEQ ID N° 13**) were used to amplify a 521 bp fragment of SSIV cDNA encoding from glutamate 236 to glutamate 414 of the SSIV amino acids sequence. Those oligonucleotides introduced restriction sites for Ndel and *Xho*l at the 5' and 3' end of the cDNA fragment respectively, which were used to clone the cDNA fragment in the expression vector pGEX-4T (Amersham Biosciences, Uppsala, Sweden) fused in frame to the 3'-end of glutathione-S-transferase gene. Construct was confirmed by DNA sequencing and transformed into *Escherichia coli* BL21 (DE3) strain. Protein expression, purification of GST-SSIV fragment fusion protein with glutathione agarose and purification of SSIV fragment by cleavage of the matrix-bound GST fusion protein with thrombin were performed following the procedure of Ausubel et al. (1987, Current Protocols in Molecular Biology. (New York: Greene Publishing Associates and Willey-Interscience). Rabbit polyclonal antiserum was raised against the purified SSIV fragment. Finally, immunoglobulin G fraction of antiserum was purified by FPLC using a Protein A Sepharose column (Amersham Biosciences, Uppsala, Sweden) following manufacturer's instructions

### A.4. Real-time RT- PCR analysis

Real-time quantitative reverse transcriptase-PCR (real-time RT-PCR) assays were achieved using an iCycler instrument (Bio-Rad, CA, USA). The PCR reaction mixture contained in a total volume of 25 µl, 5 µl of cDNA, 0.2 mM of dNTPs, 2.5 mM of MgCl₂, a 1:100000 dilution of SYBR® Green I nucleic gel stain (Molecular Probes, Eugene, OR, USA) / Fluorescein calibration dye (BioRad, CA, USA), 0.3 Units of Taq polymerase, 2.5 µl 10x Taq polymerase buffer, and 0.2 µM of each primer. Specific oligonucleotides used were: SA198 (5'-TGATGAGAAGAGGAATGACCCGAAA-3' **SEQ ID N° 14**) and SA199 (5'-CCATAGATTTTCGATAGCCGA-3' **SEQ ID N° 15**) for *AtSS1*; SA126 (5'-GGAACCATTCCGGTGGTCCATGCCG-3' **SEQ ID N° 16**) and SA127 (5'- CTCACCAATGATACTTAGCAGCAACAAG-3' **SEQ ID N° 17**) for *AtSS2;* SA200 (5'- GTGCAAGACGGTGATGGAGCAA-3' **SEQ ID N° 18**) and SA201 (5'- CACGTTTTTTATATTGCTTTGGGAA- 3' **SEQ ID N° 19**) for *AtSS3;* SA419 (5'- CGTGACTTAAGGGCTTTGGA -3' **SEQ ID N° 20**) and SA420 (5'- GCAGCTCGGCTAAAATACGA -3' **SEQ ID N° 21**) for *AtSS4;* SA546 (5'-TGGAAGGAAACGAAGGCTTTG-3' **SEQ ID N° 22**) and SA547 (5'-TGTCTTTGGCGTATTCGTGGA-3' **SEQ ID N° 23**) for *AtPHS1*; SA548 (5'-ACAGGTTTTGGACGTGGTGATT-3' **SEQ ID N° 24**) and SA549 (5'- ACAGGACAAGCCTCAATGTTCCA-3' **SEQ ID N° 25**) for *AtPHS2*; UBQF (5'-GATCTTTGCCGGAAAACAATTGGAGGATGGT-3' **SEQ ID N° 26**) and UBQR (5'-CGACTTGTCATTAGAAAGAAAGAGATAACAG-3' **SEQ ID N° 27**) for *UBQ10.* Thermal cycling consisted of 94 °C for 3 min; followed by 40 cycles of 10 sec at 94 °C, 15 sec at 61 °C and 15 sec at 72 °C. After that, a melting curve was generated to check the specificity of the amplified fragment. The efficiency of all the primers at the above conditions was between 75-110% in all the tested samples and product identity was confirmed by sequence analysis. *Arabidopsis Ubiquitin 10* (Sun and Callis, 1997, Plant J. 11, 1017-1027) was used as a house-keeping gene in the expression analysis. Absolute Quantification (Ginzinger, 2002, Exper. Hematol. 30, 503-512.) was performed by cloning the amplified products in pGEM-T vector (Promega, Madison, USA), and using them as external calibration standards.

### A.5. Extraction and purification of starch

For the analysis of the structure and composition of starch, *Arabidopsis* leaves were harvested at the end of the light period. Approximately 10 g of fresh material was homogenized using a Tissue Tearor (Biospec Products, Inc., Bartlesville, OK, USA) in 30 ml of the following buffer: 100 mM 3-(N-morpholino) propanesulphonic acid (MOPS), pH 7.2, 5 mM EDTA, 10% (v/v) ethanediol. The homogenate was filtered through two layers of Miracloth and centrifuged for 15 min at 4 C and 4000 g. The pellet was resuspended in 30 ml Percoll 90% (v/v) and centrifuged for 40 min at 4 °C and 10 000 g. The starch pellet was washed six times with distilled sterile water (10 min at 4 °C and 10 000 g between each wash). Starch was finally stored at 4 °C in 20% ethanol. For the analysis of starch content in leaves along the diurnal cycle the method was scaled down and 3 leaves (approximately 300 mg) from three different plants were used in each point. Material was frozen with liquid nitrogen, homogenized with mortar and pestle and resuspended in 1 ml of buffer. Starch isolation was performed using Percoll gradient centrifugation as described above.

### A.6. Determination of starch and WSP contents and spectral properties of the iodine-starch complex

Starch content in leaves was quantified enzymatically as described previously by Lin et al. (1988). A full account of λmax (maximal absorbance wavelength of the iodine-polysaccharide complex) measures can be found in Delrue et al. (1992, J. Bacteriol. 174, 3612-3620.). Water-soluble glucan contents in leaves were determined as described in Zeeman et al. (1998, Plant Physiol. 135, 849-858).

### A.7. Separation of starch polysaccharides by size exclusion chromatography

Starch (1.5-2.0 mg) was dissolved in 500 µl of 10 mM NaOH and subsequently applied to a Sepharose CL-2B column (0.5 cm i.d. x 65 cm), which was equilibrated and eluted with 10 mM NaOH. Fractions of 300 µl were collected at a rate of one fraction per 1.5 min. Glucans in the fractions were detected by their reaction with iodine and levels of amylopectin and amylose were determined by amyloglucosidase assays.

### A.8. Chain length distribution of amylopectin

FACE of debranched amylopectin: After purification on a Sepharose CL-2B column, 500 mg of amylopectin were dialyzed against distilled water and subsequently lyophilized. The amylopectin pellet was resuspended in 1 ml of 55 mM sodium acetate, pH 3.5 buffer, and incubated overnight at 42 °C with 20 units of isoamylase isolated from *Pseudomonas amyloderamosa* (Hayashibara Biochemical Laboratories, Okayama, Japan) and 1 unit of pullulanase from *Klebsiella pneumoniae* (Sigma, St. Louis, USA). Salts were subsequently removed by passage through an extract-clean carbograph column (Alltech, Deerfield, IL, USA). Derivatization procedure: Glucans were derivatized with 8- amino-1,3,6-pyrenetrisulfonic acid (APTS) according to the manufacturer's recommendations (Beckman Coulter, Fullerton, CA, USA). Briefly, 2 ml APTS in15% acetic acid solution and 2 ml of 1 M of NaBH3CN in tetrahydrofolate were added and the coupling reaction was allowed overnight at 37 °C in the dark. Capillary electrophoresis analysis: Separation and quantification of APTS-coupled glucans were carried out on a P/ACE System 5000 (Beckman Coulter, Fullerton, CA, USA) equipped with a laser-induced fluorescence system using a 4-mW argon ion laser. The excitation wavelength was 488 nm and the fluorescence emitted at 520 nm was recorded on the Beckman P/ACE station software system (version 1.0). Uncoated fused-silica capillaries of 57-cm length x 75-µm i.d. were used. Running buffers were from Beckman Coulter. Samples were loaded into the capillaries by electroinjection at 10 kV for 10 s and a voltage of 30 kV was applied for 20 min at a constant temperature of 25 °C.

### A.9 Zymograms techniques

A complete description of these techniques can be found in Delvallé et al. (2005, Plant J. 43, 398-412.).

### A.10. In vitro assays of starch synthesis enzymes

ADP-glucose pyrophosphorylase was assayed in the synthesis direction according to the procedure described in Crevillén et al. (2003, J Biol Chem 278, 28508- 28515.). Starch synthase activity was assayed as described in (Delvallé et al., 2005) using amylopectin or glycogen as primers. Branching enzymes, starch phosphorylase and α-1,4 glucanotransferase activities were performed according to procedures described by Zeeman et al. (1998, Plant Physiol. 135, 849-858).

### A.11. Western blot analysis

Proteins were transferred from an SDS-polyacrylamide gel to nitrocellulose membrane by electroblotting in a Trans-Blot SD transfer cell (Bio-Rad, USA), according to the manufacturer's instructions. Blots were probed with rabbit anti- SSIV followed by horseradish peroxidase-conjugated goat-anti-rabbit serum and detected using ECL Plus Advanced Western Blotting Reagent (Amersham Biosciences, Uppsala, Sweden).

### A.12. Microscopy analysis

Full-expanded leaves from plants cultured under a 16 h light/8 h dark photoperiod were collected at indicated times. Small pieces (2 mm²) of leaves were cut with a razor blade and immediately fixed in 1% paraformaldehyde and 0.5% glutaraldehyde in 0.05M Na-cacodylate buffer, pH 7.4, containing 25 mg of sucrose per ml (3.5 h at 4°C, under vacuum). After fixing and rinsing with the same buffer, tissues were dehydrated in an ethanol series and progressively embedded in LR White resin (London Resin Co., Reading, UK). Resin was polymerized with UV light at -20°C (Fedorova et al., 1999, Planta 209, 25-32). Alternatively, some samples were fixed in 3% glutaraldehyde in the above buffer and embedded in Araldite Durcupan ACM as described by Lucas et al., (1998, Protoplasma 204, 61-70). Semithin (1 µm) and ultrathin (60 nm) sections were cut with a Leika Ultracut microtome (Vienna, Austria) fitted with a diamond knife. Semithin sections for light microscopy were stained with 1% (w/v) toluidine blue in aqueous 1% sodium borate for direct observation with a Zeiss Axiophot photomicroscope (Oberkochen, Germany). Ultrathin sections for transmission electron microscopy were contrasted with 2% aqueous uranyl acetate and lead citrate (Reynolds, 1963, J. Cell Biol. 17, 208-213). Observations were performed with a STEM LEO 910 electron microscope (Oberkochen, Germany) at 80 kV, equipped with a Gatan Bioscan 792 digital camera (Pleasanton, CA, USA). Different sections from at least three different leaves samples were analyzed.

For scanning electron microscopy analysis samples were sputter-coated with gold and viewed with a JEOL JSM-5400 (Tokio, Japan) microscopy.
Transmission microscopy analysis was performed as described in Delvallé et al. (2005, Plant J. 43, 398-412)

### Example 1 : Levels of SSIV mRNA in different organs.

As a first step in the characterization of the function of the SSIV isoform, the spatial pattern of expression of *AtSS4* gene (locus At4g 18240) was established. Using quantitative real-time RT-PCR, this pattern was compared to that of the other classes of SSs (SSI, SSII and SSIII encoded by *AtSS1, AtSS2* and *AtSS3* loci respectively). The four genes were expressed in all organs studied (leaves, roots, flowers and immature fruits). In all cases the steady-state level of *AtSS1* mRNA was one order of magnitude higher than that of the other *AtSS* genes (Figure 1A). On the other hand, *AtSS4* mRNA accumulated at similar levels in all organs analyzed, with values equivalent to those obtained for the *AtSS3* gene (Figure 1 B).

### Example 2 : Isolation of mutant lines defective in SSIV

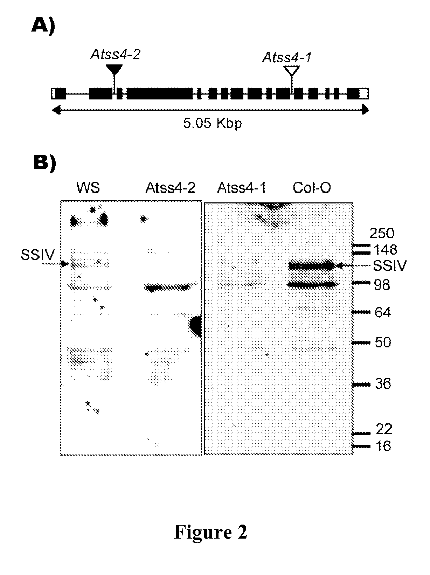

The *AtSS4* (At4g18240) gene is located in chromosome 4 and is composed of 16 exons and 15 introns. It encodes a 1040 amino acid protein with a predicted mass of 117747 Da. This protein shows a high level of similarity with the previously annotated SSIV proteins found in other species such as *Vigna unguiculata* (71% identity. Accession number AJ006752), wheat (58.2% identity. Accession number AY044844), or rice (56.8% and 58.3% identity with SSIVa (accession number AY373257) and SSIVb (accession number AY373258) respectively). Bioinformatic analysis predicted the presence of a chloroplast-targeting signal comprising the first 42 amino acids, rendering a mature protein of 112997 Da (ChloroP at http://www.cbs.dtu.dk/services/ChloroP/, (Emanuelsson et al., 1999). Two independent mutant alleles designed *Atss4-1* (Col-O ecotype) and *Atss4-2* (WS ecotype) corresponding to T-DNA insertions in the *AtSS4* gene were found in the GABI-KAT (http://www.gabi-kat.de/, (Rosso et al., 2003) and the Genoplante-INRA (http://flagdb-genoplante-info.infobiogen.fr/projects/fst/, (Balzergue et al., 2001) mutant collections respectively. The T-DNA insertions are located in intron 11 and 2 (position +3763 bp and + 227 with respect to the start codon for *Atss4-1* and *Atss4-2* respectively) (Figure 2).

Homozygous mutant plants were selected and expression of the *AtSS4* mutant alleles was analyzed by RT-PCR using specific oligonucleotides for different regions of the gene. This analysis indicated that a modified messenger corresponding to *AtSS4* was still present in both alleles (data not shown). Western blot analysis was then performed to check for the absence of SSIV protein in both mutant lines. The rabbit antiserum used was raised against a 178 amino acids polypeptide fragment of SSIV protein corresponding to a region that displays no similarity with all other SS isoforms (from Glutamate236 to Glutamate414) (see Experimental Procedures). Western blots showed the presence of two close bands with a mass of approximately 112 kDa in both wild type ecotypes (Figure 2B). These bands match the size of the expected mature SSIV protein and are both absent in the two mutant alleles. Truncated versions of the SSIV protein were not detected in the mutant lines either (smaller, unspecific bands, were found in both mutant and WT lines) (Figure 2B). The presence of these 2 bands in both wild-type ecotypes is not understood yet but could be due to posttransiational modification of the protein.

### Example 3 : Phenotypic characterization of Atss4 mutant alleles

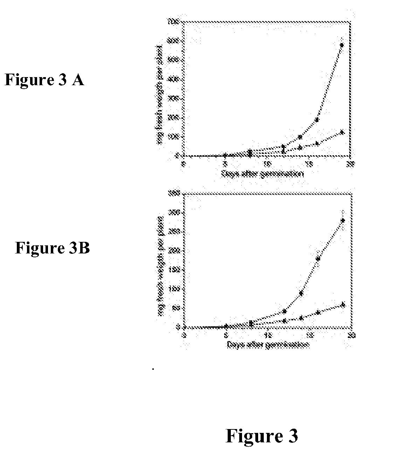

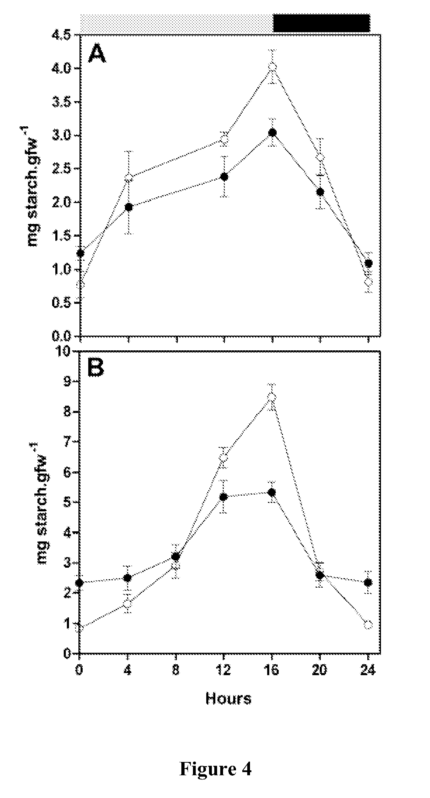

The absence of the SSIV protein has deleterious effect on plant growth. Both mutant lines showed lower growth rates under a 16 h day/8 h night photoregime when compared their respective wild type ecotypes (Figure 3-A and Figure 3-B). Rosette leaves of mutant alleles were smaller than those of WT (not shown). In addition we recorded a delay in flowering time: 31+/-3 days for *Atss4-1* versus 25 days for Col-O, and 21 days for *Atss4-2* versus 18+/-1 for WS ecotype. However, the number of rosette leaves at bolting was the same in mutant and WT plants, indicating that the delay in flowering time comes as a consequence of the reduced growth rate in the mutant lines.
Fruit size, number of seed per silique and germination ratios were not altered in the mutant lines. The amount of leaf starch was also determined in the mutant lines. The starch content at the end of the illuminated period was reduced in both cases: 35% decrease for *Atss4-1* and 50% for *Atss4-2* line with respect to their WT genetic backgrounds. A more detailed analysis of starch accumulation along the day/night cycle was carried out on the *Atss4-1* line. As shown in Figure 4, starch turnover along a diurnal cycle was lower in mutant than in WT plants, with a clear reduction of both synthesis and degradation rates. Starch amounts at the beginning of the light period was higher in the *Atss4-1* than in the WT plants. However the reduced rate of starch synthesis in the mutant led to lower starch level in the mutant at the end of the illuminated period.

### Example 4 : Enzymological characterization of Atss4 mutant lines

The levels of enzymatic activities involved in the synthesis and the degradation of starch were determined in mutant lines using both zymograms and *in vitro* assays (Experimental procedures). No decrease in total soluble starch synthase activity was observed *in vitro* in both mutants using rabbit liver glycogen or amylopectin as primers (Table 1). Other starch metabolizing enzymes were unaffected (Table 1), except for starch phosphorylase whose total activity was increased by 1.4 to 2 fold depending on the substrate used for the assay (Table 1). Zymograms analysis showed that this induction was caused by an increase in the activity of both the cytosolic (PHS2) and plastidial (PHS1) isoforms of starch phosphorylase (Not shown). In order to test whether this increase could be attributable to an interaction between SSIV and starch phosphorylase proteins that was missed in the mutant lines, or to another indirect effect, the mRNA level of both *PHS* genes in *Atss4-1* was determined by real-time RT-PCR. Figure 5-B shows that the expression of both genes was increased in the mutant line with respect to the WT ecotype. The extent of *PHS* mRNAs induction was comparable to that found for PHS activity (Figure 5-A and 5-B), suggesting that the absence of SSIV induced starch phosphorylase activity trough a metabolic alteration that triggers the induction of both plastidial and cytosolic *PHS* genes expression.

### Example 5 : Effect of eliminating SSIV on the structure and composition of starch

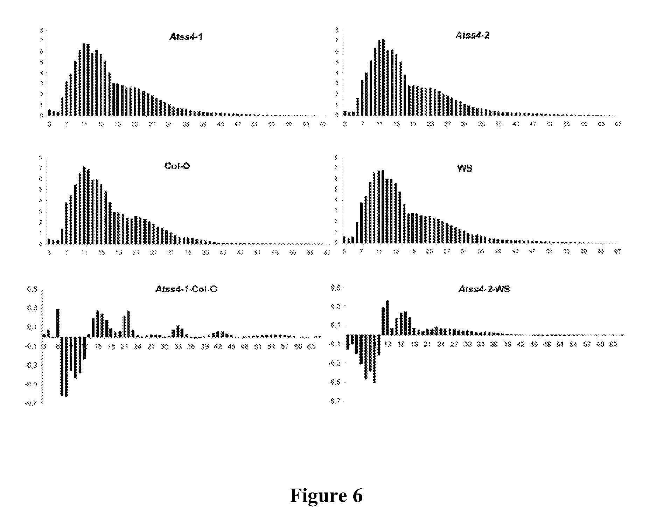

Interruption of *AtSS4* locus did not affect the total soluble starch synthase activity *in vitro* (Table 1). However starch synthesis was clearly reduced in the mutants. In order to determine if *Atss4* mutations were altering the structure and composition of starch, the amylose/amylopectin ratio and chain length distribution of amylopectin were analyzed in both mutant alleles.
Amylose and amylopectin polymers were separated using size exclusion chromatography performed on sepharose CL-2B columns, and subsequently quantified by the amyloglucosidase assay. This analysis showed that the amylose/amylopectin ratio was not affected in *Atss4* mutants.
Purified amylopectin was then subjected to complete enzymatic debranching and the chain length (CL) distribution was determined by fluorophore-assisted capillary electrophoresis (FACE) after coupling the resulting linear glucans with APTS. Comparison of CL distribution profiles of *Atss4* mutant alleles and their respective WT ecotypes indicated that the *Atss4* mutation had minor effects on the structure of amylopectin (Figure 6). Indeed, only a slight reduction on the amount of chains of DP= 7-10 could be observed (note the scale of the difference plots in Figure 6).
Morphology and size of the starch granules were analyzed using both scanning (SEM) and transmission (TEM) electron microscopy. Starch granules were isolated from *Atss4* mutant and wild type leaves collected at midday and processed as described in Experimental Procedures. Starch granule morphology did not seem to be affected in mutant plants (Figure 7). However a dramatic enlargement of granules size was detected in both mutant alleles (Figure 7-B and 7-D). A more detailed study was carried out by transmission electron microscopy (Not shown) and light microscopy (Not shown) analysis on sections of leaves collected at 4 h and 12 h within the light phase. Two relevant results arose from those analyses: the greatest difference in size between Col-O and *Atss4-1* starch granules was observed at the beginning of the day (4 h of light, Not shown). This result is in line with the lower rate of starch degradation observed in mutant plant (Figure 4), which is expected to yield larger starch granules in the latter after the dark period. Secondly, most of the chloroplasts in *Atss4-1* mutant plants contain a single starch granule with only few exceptions (two granules per chloroplast). This observation comes in stark contrast with that obtained in WT chloroplasts, where 4-5 starch granules per chloroplasts could be observed. Different sections from different leaf samples were analyzed yielding the same results in all cases (data not shown). We therefore conclude that a loss of function mutation at the AtSS4 locus affects the number and size of starch granules synthesized in the chloroplast.

### Additional discussion of the results shown in Examples 1 to 5

In this study, the phenotypic alterations produced by the specific loss of starch synthase class IV (SSIV) were analyzed. The analysis of two independent T-DNA insertion mutants obtained in two distinct genetic backgrounds, such as Col-O and WS ecotypes, indicates that the shared phenotypic alterations found in the two mutants are specifically caused by the loss of the SSIV protein. The T-DNA insertions were located in intron 2 and 11 of *AtSS4* genomic locus (At4g18240) in the two mutant alleles studied in the examples herein (Figure 2). These insertions determined in both cases the synthesis of a modified SSIV mRNA. However, Western blot analysis indicated that these messengers failed to produce a wild type SSIV protein (Figure 2). The antibody used in this analysis was raised against a 178 amino acids fragment of the amino-terminal region of the protein, upstream of the T-DNA insertion site, so that the presence of truncated versions of SSIV protein in the mutant alleles should be also ruled out as no small, mutants specific, polypeptide was detected by Western blot (Figure 2).
At variance with what is found for mutants for any one of the three other starch synthases, *Atss4* mutants showed amylopectin CL profiles similar to those found in the respective wild type ecotypes. Only a weak, although reproducible, reduction of the amount of short chains (DP 7 to 10) could be observed (Figure 6). Amylose/amylopectin ratio and levels of water soluble polysaccharides remained unchanged in both mutant alleles with respect to values in their respective WT (data not shown).
Zymogram analysis did not reflect any change of the activity levels of either SSI or SSIII isoforms (Not shown), thereby ruling out the existence of compensation mechanisms leading to the selective increase in the activity of other starch synthases making up for the loss of SSIV. These results taken together suggest that the major function of SSIV isoform might be different from the elongation of amylopectin chains during the process of starch biogenesis.
The main alterations in starch metabolism in the *Atss4* mutants consisted in the reduction of the starch synthesis and degradation rates (Figure 4) that correlated with an enlargement of starch granule size and a decrease in granule numbers per chloroplast (Figure 7). The reduced rate of starch turnover along the diurnal cycle cannot be explained by a reduction in activity of the starch metabolizing enzymes as no significant changes in such activities could be detected by both *in vitro* and zymograms analysis (Table 1).
The reduced number of starch granules in *Atss4* mutants would also be responsible for the size enlargement observed in these granules (Figure 7). In this case, all the ADP-glucose pool must be channelled to one or two granules, leading to considerably bigger starch granules in the mutants by comparison to wild type plants. The data reported here indicate that SSIV is required to determine the correct number of starch granules per chloroplasts and that the amount of polysaccharide surface is an important determinant of the rates of starch synthesis and degradation.

### Example 6 : Further SSIV-defective plants

It is provided herein further SSIV-defective plants, wherein beyond alterations to the SSIV-encoding gene, also alterations to the starch phosphorylase-encoding gene have been introduced.

Thus, it is provided in example 6 *Arabidopsis thaliana* plants that have been rendered defective both for (i) SSIV and (ii) starch phosphorylase.

The starch content of the plant leaves of (i) wild-type (WS) *Arabidopsis thaliana* plants, (ii) starch phosphorylase-defective (phs1-) *Arabidopsis thaliana* plants and of (iii) two SSIV-defective (ss4-; phs1-/ss4-) *Arabidopsis thaliana* plants according to the invention has been determined. The results are shown in Table 2 below.

**Table 2**

| ***A. thaliana* plant line** | **mean starch content in leaves*** | **standard deviation** |
|---|---|---|
| WS (wild-type) | 5,1 | 0,2 |
| phs1- | 5,7 | 0,4 |
| ss4- | 4,3 | 0,2 |
| phs1-/ss4- | 19,3 | 2,5 |

| | | |
|---|---|---|
| * mg of starch per g of leave tissue | | |

As shown in Table 2 above, the overall starch content of plants that have been rendered defective exclusively for SSIV (ss4-) does not significantly differ from wild-type plants. However, as it is shown in Figure 9, the starch granules originating from the (ss4-) plants have a significantly greater size than those from the wild-type plants (WS). From the microphotographs of Figure 9, it has been determined that the size of the starch granules originating from the (ss4-) plants are about two to three times larger than those originating from wild-type plants (WS).

As shown in Table 2 above, the starch content of plants that have been rendered defective for both (i) SSIV and (ii) starch phosphorylase (phs1-/ss4-) is increased by almost four times, as compared with wild-type plants (WS). Further, as shown in Figure 9, the starch granules originating from the (phs1-/ss4-) plants have a far greater size than those from the wild-type plants (WS). From the microphotographs of Figure 9, it has been determined that the size of the starch granules originating from the (phs1-/ss4-) plants are about four to five times larger than those originating from wild-type plants (WS).

Further, as shown in Figure 9, the starch granules originating from the wild-type plants (WS) are rather flat-shaped, whereas the starch granules originating from both SSIV-defective plants, namely (ss4-) and (phs1-/ss4-) are rather round-shaped.

**Table 1. In vitro assays of several starch metabolizing enzym es performed with leaf crude extracts of AtSS4 mutant alleles (Atss4-1 and Atss4-2) and their wild type ecotypes (Col-0 and WS respectively). Activities are expre ssed in nmol.min-1.mg-1 of proteins □standard error (in each case n = 3). 1 Starchphosphorylase activity was a ssayed in the sense of glucan degradation.**

| **Activity** | **Col-O** | ***Atss4-1*** | **WS** | ***Atss4-2*** |
|---|---|---|---|---|
| | | | | |
| **Soluble Starch Synthase** | | | | |
| ***with rabbit liver glycogen*** | 7.86+/-0.38 | 8.50+/-0.47 | 6.35+/-0.15 | 6.36+/-0.10 |
| ***with Amylopectin*** | 6.31 +/-0.25 | 6.42+/-0.13 | 5.54+/-0.23 | 5.39+/-0.18 |
| | | | | |
| **AGPase (biosynthetic assay)** | 21.07+/-3.5 | 21.77+/-4.8 | 28.30+/-2.1 | 27.48+/-3.0 |
| | | | | |
| **Starch Branching Enzyme** | 617+/-32 | 582+/-26 | 378+/-47 | 447+/-38 |
| | | | | |
| **α-1,4-glucanotransferase** | 0.028+/-0.002 | 0.029+/-0.001 | 0.12+/-0.002 | 0.11+/-0.003 |
| | | | | |
| **Starch Phosphorylase1** | | | | |
| ***with Amylopectin*** | 16.0+/-2 21. | 2+/-1.7 7 | 14.7+/-1.1 1 | 22+/-1.2 |
| ***with DP7*** | 32.2+/-2.1 1 | 62.5+/-3.1 | 36.8+/-3.1 | 64.2+/-2.8 |

**Table 3 : Sequences**

| **SEQ ID N°** | **Type** | **Designation** |
|---|---|---|
| 1 | Nucleic acid | SSIV from *Arabidopsis thaliana* |
| 2 | Nucleic acid | SSIV-1 from *Oryza sativa* |
| 3 | Nucleic acid | SSIV-2 from *Oryza sativa* |
| 4 | Nucleic acid | SSIV from *Triticum aestivum* |
| 5 | Nucleic acid | SSIV from *Vigna unguiculata* |
| 6 | Nucleic acid | SSIV from *Arabidopsis thaliana* disrupted by T-DNA from *Agrobacterium tumefaciens* |
| 7 | Protein | SSIV from *Arabidopsis thaliana* |
| 8 | Protein | SSIV-1 from *Oryza sativa* |
| 9 | Protein | SSIV-2 from *Oryza sativa* |
| 10 | Protein | SSIV from *Triticum aestivum* |
| 11 | Protein | SSIV from *Vigna unguiculata* |
| 12-27 | Nucleic acid | Primers |

## Claims

1. A plant which is modified so as to be rendered defective for Soluble Starch Synthase IV (SSIV), having plant cells which contain starch granules having an increased granule size, as compared to the same plant that is not defective for Soluble Starch Synthase IV (SSIV).

2. The plant according to any claim 1, producing a mutated inactive Soluble Starch Synthase IV (SSIV).

3. The plant according to claim 1, having a reduced synthesis or no synthesis of Soluble Starch Synthase IV (SSIV).

4. The plant according to claim 1, having a reduced synthesis or no synthesis of the mRNA encoding the said Soluble Starch Synthase IV (SSIV).

5. The plant according to claim 1, wherein the gene encoding Soluble Starch Synthase IV (SSIV) has been mutated by one or more insertions, deletions or substitutions of a nucleotide.

6. The plant according to claim 5, wherein the gene encoding Soluble Starch Synthase IV (SSIV) has been mutated in at least one gene sequence portion selected from the group consisting of the promoter sequence, an intron sequence and an exon sequence.

7. The plant according to claim 5, wherein the gene encoding Soluble Starch Synthase IV (SSIV) has been mutated by at least one nucleotide insertion within at least one intron or one exon.

8. The plant according to any one of claims 1 to 7, which has further been rendered defective for starch phosphorylase.

9. The plant according to any one of claims 1 to 8, which is selected from the group consisting of a monocot or a dicot.

10. The plant according to any one of claims 1 to 8 belonging to a species selected from the group consisting of *Brassicacae,* corn (*Zea mays),* potato *(Solanum tuberosum),* broad bean *(Vicia Faba L),* rice (*Oryza sativa*), beet (*Beta vulgaris*), spinach (*Spinacia oleracea*), green pea (*Pisum sativum*) and wheat (*Triticum aestivum*).

11. The plant according to claim 10, consisting of a transformed *Arabidopsis thaliana* comprising a mutated Soluble Starch Synthase IV (SSIV) gene consisting of SEQ ID N° 7.

12. A plant cell originating from a plant according to any one of claims 1 to 11.

13. A seed from a plant according to any one of claims 1 to 11.

14. A method for obtaining a transformed plant defective for Soluble Starch Synthase IV (SSIV) comprising the steps of :
a) providing a plant cell;
b) introducing into the plant cell provided at step a) a DNA construct which renders the said plant cell defective for Soluble Starch Synthase IV (SSIV) activity, whereby a transformed plant cell defective for Soluble Starch Synthase IV (SSIV) activity is obtained;
c) regenerating a whole plant from the transformed plant cell obtained at the end of step b).

15. The method of claim 14, wherein step b) further comprises introducing into the same plant a DNA construct which renders the said plant cell defective for starch phosphorylase activity, whereby a transformed plant cell defective for Soluble Starch Synthase IV (SSIV) activity is obtained, that is also defective for starch phosphorylase activity.

16. The method according to any one of claims 14 or 15, wherein the said DNA construct introduced at step b) consists of an expression cassette comprising a nucleotide sequence that is selected form the group consisting of antisense, sense, dsRNA, a ribozyme, or an inverted repeat to a plant nucleotide sequence consisting of a SSIV-encoding sequence.

17. A transformed plant defective for Soluble Starch Synthase IV (SSIV) activity, which has been obtained by the method according to any one of claims 14 to 16.

18. The transformed plant according to claim 17, which is selected from the group consisting of a monocot or a dicot.

19. The transformed plant according to claim 18, belonging to a species selected from the group consisting of *Brassicacae,* corn (*Zea mays*)*,* potato *(Solanum tuberosum),* broad bean *(Vicia Faba L),* rice (*Oryza sativa*)*,* beet (*Beta vulgaris*)*,* spinach (*Spinacia oleracea*)*,* green pea (*Pisum sativum*) and wheat (*Triticum aestivum*)*.*

20. The transformed plant according to claim 18, consisting of a transformed *Arabidopsis thaliana* comprising a mutated Soluble Starch Synthase IV (SSIV) gene consisting of SEQ ID N° 7..

21. A transformed plant cell defective for Soluble Starch Synthase IV (SSIV) activity originating from a transformed plant cell according to any one of claims 17 to 20.

22. A method for producing starch comprising a step of extracting starch from a plant defective for Soluble Starch Synthase IV (SSIV) according to any one of claims 1 to 11 and 17 to 20 or from a plant cell according to any one of claims 12 and 13.
